# EUROPEAN PATENT APPLICATION

(11) **EP 4 556 494 A1**
(43) Date of publication of application: **21.05.2025**
(21) Application number: 23840021.2
(22) Date of filing: 14.07.2023
(51) Int. Cl.: C07K 16/28, A61P 35/00, G01N 33/574, A61K 39/00

(54) **ANTI-ROR1 ANTIBODY, BISPECIFIC ANTIBODY COMPRISING SAME, AND USES THEREOF**

(30) Priority: 15.07.2022 KR 20220087520; 23.03.2023 KR 20230038009
(71) Applicant: ABL Bio, Inc., Seongnam-si, Gyeonggi-do 13488 (KR)
(72) Inventor: LEE, Yangsoon, Seongnam-si, Gyeonggi-do 13488 (KR); JUNG, Jinwon, Seongnam-si, Gyeonggi-do 13488 (KR); KIM, Juhee, Seongnam-si, Gyeonggi-do 13488 (KR); PARK, Kyeongsu, Seongnam-si, Gyeonggi-do 13488 (KR); SUNG, Byungje, Seongnam-si, Gyeonggi-do 13488 (KR); YEOM, Donghoon, Seongnam-si, Gyeonggi-do 13488 (KR); LEE, Bora, Seongnam-si, Gyeonggi-do 13488 (KR); YOO, Byeongmin, Seongnam-si, Gyeonggi-do 13488 (KR); SON, Wonjun, Seongnam-si, Gyeonggi-do 13488 (KR); LEE, Suyoun, Seongnam-si, Gyeonggi-do 13488 (KR); KO, Minji, Seongnam-si, Gyeonggi-do 13488 (KR); RYU, Ilhwan, Seongnam-si, Gyeonggi-do 13488 (KR); LEE, Sunju, Seongnam-si, Gyeonggi-do 13488 (KR); KWON, Jung A, Seongnam-si, Gyeonggi-do 13488 (KR); CHOI, Hyoju, Seongnam-si, Gyeonggi-do 13488 (KR); KIM, Sora, Seongnam-si, Gyeonggi-do 13488 (KR); YOUN, Hyunseong, Seongnam-si, Gyeonggi-do 13488 (KR); PARK, Kyungjin, Seongnam-si, Gyeonggi-do 13488 (KR); SON, Yong-Gyu, Seongnam-si, Gyeonggi-do 13488 (KR)
(74) Representative: Potter Clarkson
(86) International application number: PCT/KR2023/010149
(87) International publication number: WO 2024/014930

(57) **Abstract**

Provided are: a novel anti-ROR1 antibody; a bispecific antibody comprising the anti-ROR1 antibody; and uses thereof.

## Description

### Technical Field

Provided are a novel anti-ROR1 antibody, a bispecific antibody comprising the anti-ROR1 antibody, and uses thereof.

### Background Art

Receptor tyrosine kinase-like orphan receptor (ROR), which is a transmembrane protein in the receptor tyrosine kinase (RTK) family, includes ROR1 and ROR2. ROR1 and ROR2 have 58% amino acid sequence homology with each other, and both have a theoretical molecular weight of about 104 kDa; however, due to multiple N-glycosylation sites, ROR1 has a molecular weight of about 130 kDa. The extracellular domain of the ROR family consists of Ig, cysteine-rich, and kringle domains, while the intracellular domain is composed of tyrosine kinase, serine/threonine (Ser/Thr)-rich, and proline (Pro)-rich domains. In terms of biological characteristics, the ligand for ROR2 is Wnt5a while the ligand for ROR1 has not yet been elucidated.

ROR1 is expressed during embryonic and fetal development, regulating cell polarity, cell migration, and neurite growth. The expression of ROR1 gradually decreases with development, and it is almost not expressed in adults, except for a transient expression during the development of B cells and slight expression in adipocytes. However, the overexpression of ROR1 in various cancer cells has led to its classification as an oncofetal gene. In particular, the discovery that ROR1 is overexpressed in chronic lymphocytic leukemia (CLL) has attracted increased attention on it as a target for cancer antibodies. In addition to CLL, overexpression of ROR1 has been reported in various hematologic malignancies such as B-cell leukemia, lymphoma, acute myeloid leukemia (AML), Burkitt lymphoma, mantle cell lymphoma (MCL), acute lymphoblastic leukemia (ALL), diffuse large B-cell lymphoma (DLBCL), follicular lymphoma (FL), marginal zone lymphoma

(MZL), as well as in a wide range of solid tumors including breast cancer, kidney cancer, ovarian cancer, gastric cancer, liver cancer, lung cancer, colorectal cancer, pancreatic cancer, skin cancer, bladder cancer, testicular cancer, uterine cancer, prostate cancer, non-small cell lung cancer (NSCLC), neuroblastoma, brain cancer, colon cancer, epithelial squamous cell carcinoma, melanoma, multiple myeloma, cervical cancer, thyroid cancer, head and neck cancer, and adrenal cancer. The expression of ROR1 in such cancers is associated with poor prognosis in cancer patients and is known to affect cancer metastasis. It has been shown that mice injected with cancer cells in which ROR1 expression was suppressed had increased survival times and reduced metastasis.

Thus, ROR1, which is specifically expressed in cancer cells, can be an effective target for cancer therapy, necessitating the development of antibodies that specifically recognize ROR1. Moreover, when developing therapeutic antibodies, it is also very important to possess not only target specificity and binding affinity but also favorable properties related to production, storage, and administration, such as physicochemical property like stability and developability.

Multispecific antibodies targeting two or more antigens are being developed in various types and forms, and are expected to be superior therapeutic antibodies compared to single-target antibodies in terms of therapeutic effects.

### Disclosure

### Technical Problem

Provided herein are a novel anti-ROR1 antibody or an antigen-binding fragment thereof, a bispecific antibody comprising the same, and uses thereof.

The anti-ROR1 antibody or antigen-binding fragment thereof may comprise:
HCDR1 comprising the amino acid sequence of SEQ ID NO: 1;
HCDR2 comprising the amino acid sequence of SEQ ID NO: 2;
HCDR3 comprising the amino acid sequence of SEQ ID NO: 3;
LCDR1 comprising the amino acid sequence of SEQ ID NO: 4;
LCDR2 comprising the amino acid sequence of SEQ ID NO: 5; and
LCDR3 comprising the amino acid sequence of SEQ ID NO: 6.

The anti-ROR1 antibody or the antigen-binding fragment thereof may comprise:
heavy chain complementarity-determining regions (CDRs) comprising HCDR1 with the amino acid sequence of SEQ ID NO: 1, HCDR2 with the amino acid sequence of SEQ ID NO: 2, and HCDR3 with the amino acid sequence of SEQ ID NO: 3, or a heavy chain variable region comprising the heavy chain CDRs; and
light chain complementarity-determining regions (CDRs) comprising LCDR1 with the amino acid sequence of SEQ ID NO: 4, LCDR2 with the amino acid sequence of SEQ ID NO: 5, and LCDR3 with the amino acid sequence of SEQ ID NO: 6, or a light chain variable region comprising the light chain CDRs.

In an embodiment, the anti-ROR1 antibody or the antigen-binding fragment thereof may comprise a heavy chain variable region of SEQ ID NO: 7 or SEQ ID NO: 127, and a light chain variable region of SEQ ID NO: 8 or SEQ ID NO: 126. More specifically, the anti-ROR1 antibody or the antigen-binding fragment thereof may comprise the heavy chain variable region of SEQ ID NO: 7 and the light chain variable region of SEQ ID NO: 8, or the heavy chain variable region of SEQ ID NO: 127 and the light chain variable region of SEQ ID NO: 126, but is not limited thereto.

Another aspect provides a bispecific antibody comprising the anti-ROR1 antibody or an antigen-binding fragment thereof.

More specifically, provided is a bispecific antibody or an antigen-binding fragment thereof comprising: a first targeting moiety that targets (specifically binds to and/or recognizes) a first antigen; and a second targeting moiety that targets (specifically binds to and/or recognizes) a second antigen,
where the first antigen and the second antigen are different from each other, and
one of the first targeting moiety and the second targeting moiety comprises the anti-ROR1 antibody or the antigen-binding fragment thereof.

In the bispecific antibody or the antigen-binding fragment thereof according to an embodiment, one of the first antigen and the second antigen may be ROR1 while the other may be selected from a group consisting of cancer cell-expressed antigens, immune checkpoint proteins, and/or T cell surface antigens, for example, from 4-1BB and B7-H3, but with no limitations thereto.

Another aspect provides a conjugate comprising (1) the anti-ROR1 antibody or antigen-binding fragment thereof, or the bispecific antibody, and (2) at least one selected from the group consisting of useful polymers, labels, drugs, etc. The drug may be a cytotoxic drug, for example, an anticancer drug.

Another aspect provides a pharmaceutical composition comprising: the anti-ROR1 antibody or antigen-binding fragment thereof; the bispecific antibody; and/or the conjugate. The pharmaceutical composition may further comprise a pharmaceutically acceptable excipient. The pharmaceutical composition may have anticancer activity.

Another aspect provides a pharmaceutical composition, comprising: the anti-ROR1 antibody or antigen-binding fragment thereof; the bispecific antibody; and/or the conjugate, for the prevention and/or treatment of cancer. The cancer may be a cancer expressing ROR1.

Another aspect provides a method for the prevention and/or treatment of cancer, the method comprising a step of administering the anti-ROR1 antibody or antigen-binding fragment thereof; the bispecific antibody; and/or the conjugate in a pharmaceutically effective amount to a subject in need thereof. The method may further comprise identifying (diagnosing) a subject in need of prevention and/or treatment of cancer prior to the administering step.

Another aspect provides a use of the anti-ROR1 antibody or antigen-binding fragment thereof; the bispecific antibody; and/or the conjugate for the prevention and/or treatment of cancer and/or for the preparation of a medicament (anticancer agent) for the prevention and/or treatment of cancer.

Another aspect provides a composition, comprising the anti-ROR1 antibody or antigen-binding fragment thereof, for the detection of ROR1.

Another aspect provides a method for detecting ROR1 in a biological sample, the method comprising a step of contacting the biological sample with the anti-ROR1 antibody or antigen-binding fragment thereof.

Another aspect provides a composition, comprising the anti-ROR1 antibody or antigen-binding fragment thereof, for the diagnosis of cancer. The cancer may be a cancer expressing ROR1.

Another aspect provides a method for diagnosing cancer in a biological sample or a subject from which the biological sample is derived, or for proving information for cancer diagnosis, the method comprising a step of contacting the anti-ROR1 antibody or antigen-binding fragment thereof with the biological sample.

### Technical Solution

Provided are a novel anti-ROR1 antibody, a bispecific antibody comprising the same, and anticancer uses thereof.

### Definition of Terms

Through this specification, the expression that a polynucleotide (which can be used interchangeably with a gene or a nucleic acid molecule) or a polypeptide (which can be used as interchangeably with a protein, and may mean a CDR, a variable region, a heavy chain, a light chain, and/or an antibody according to the context) "comprises a specific nucleic acid sequence or amino acid sequence" or "consists of or is represented by a specific nucleic acid sequence or amino acid sequence" is intended to mean that the polynucleotide or polypeptide necessarily comprises the specific nucleic acid or amino acid sequence, and may comprise a substantially equivalent sequence to the specific nucleic acid or amino acid sequence, with variations (deletions, substitutions, modifications, and/or additions) that are consistent with maintaining the original and/or intended function of the polynucleotide or polypeptide (or without excluding the possibility of such variations).

In an embodiment, the expression that a polynucleotide or polypeptide "comprises a specific nucleic acid sequence or amino acid sequence" or "consists of or is represented by a specific nucleic acid sequence or amino acid sequence" may mean that a polynucleotide or polypeptide (i) essentially comprises the specific nucleic acid or amino acid sequence, or (ii) consists of or essentially comprises a nucleic acid or amino acid sequence having 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more sequence identity with the specific nucleic acid or amino acid sequence, and retains the original function and/or intended function.

As used herein, the term "sequence homology" or "sequence identity" refers to a degree of match between a given nucleic acid or amino acid sequence, and can be expressed as a percentage (%). For nucleic acid sequence homology, for example, the homology or identity can be determined using algorithms such as BLAST or Pearson's FASTA. Based on these algorithms, programs known as BLASTN or BLASTX have been developed (see: http://www.ncbi.nlm.nih.gov).

Herein, the expression that a protein or polypeptide "comprises a specific amino acid sequence or consists of or is represented by a specific amino acid" may mean cases where the amino acid sequence is essentially comprised, and/or where inconsequential variations (e.g., substitution, deletion, and/or addition of amino acid residues) which do not affect the original activity and/or intended activity (for instance, binding affinity to an antigen, anticancer activity such as inhibition of cancer cell growth, induction of cancer cell apoptosis, etc.) are introduced into the amino acid sequence.

Unless defined otherwise, amino acid positions mentioned in this specification are counted from the N-terminus of the reference amino acid sequence.

As used herein, the term "antibody" refers generally to a protein that specifically binds to a particular antigen and is intended to encompass proteins produced by the immune system in response to an antigen stimulus or proteins chemically synthesized or recombinantly produced, without being specifically limited in type. An antibody may be any isotype of an intact immunoglobulin or an antigen-binding fragment that can compete with an intact antibody for binding to the target antigen. Antibodies are also a type of antigen-binding protein themselves. An intact antibody typically may include at least two full-length heavy chains and two full-length light chains. In this specification, an antibody or an antigen-binding fragment thereof may be unnaturally produced, for example, recombinantly or synthetically. For instance, an antibody or an antigen-binding fragment thereof can be produced by hybridoma technology, recombinant DNA techniques, or enzymatic or chemical cleavage of a complete antibody. Unless otherwise stated in this specification, the term "antibody" encompasses antibodies including two full-length heavy chains and two full-length light chains, as well as derivatives, variants, fragments, and/or mutants thereof. The antibodies may be animal antibodies (e.g., mouse antibodies), chimeric antibodies (e.g., mouse-human antibodies), humanized antibodies, or human antibodies. The antibodies may be monospecific (or monoclonal antibodies) or polyspecific (or polyclonal antibodies).

In the antibodies or their antigen-binding fragments provided herein, portions other than the defined heavy chain CDR and light chain CDR, or heavy chain variable region and light chain variable region, may be derived from all immunoglobulin isotypes (e.g., IgA, IgD, IgE, IgG (IgG1, IgG2, IgG3, or IgG4), IgM, etc.), for example, from the framework regions of all these isotypes of immunoglobulins, and/or from the constant regions of the light and heavy chains. In an embodiment, the antibodies provided in this specification may be of the human IgG type, for example, IgG1, IgG2, IgG3, or IgG4, but are not limited thereto.

An intact antibody (e.g., of the IgG type) has a structure possessing two full-length light chains and two full-length heavy chains, with disulfide linkages therebetween. The constant regions of an antibody are divided into heavy chain constant regions and light chain constant regions, with heavy chain constant regions being of the gamma (γ), mu (µ), alpha (α), delta (δ), or epsilon (ε) types, and subclasses include gamma1 (γ1), gamma2 (γ2), gamma3 (γ3), gamma4 (γ4), alpha1 (α1), or alpha2 (α2). The constant regions of the light chains are of the kappa (κ) or lambda (λ) types.

In an embodiment, the constant region of IgG (e.g., human IgG1) may be of the wild type. In another embodiment, the constant region of IgG may have mutations introduced for a specific purpose. For instance, the constant region of IgG, based on human IgG1, may include known mutations, such as the N297A mutation (where the 297^{th} amino acid residue N is substituted by A), and such variants may exhibit reduced antibody-dependent cellular cytotoxicity (ADCC) compared to the wild type.

The term "heavy chain" is interpreted to encompass a full-length heavy chain and fragments thereof, wherein the full-length heavy chain includes a variable domain VH having a sufficient variable region sequence to confer specificity to an antigen, three constant region domains C_{H1}, C_{H2}, and C_{H3}, and a hinge. The VH domain is located at the N-terminus of the heavy chain, the CH domains are at the C-terminus, and the C_{H3} domain is closest to the C-terminus. The heavy chain may be selected from among the isotypes of IgG (including IgG1, IgG2, IgG3, and IgG4 subtypes), IgA (including IgA1 and IgA2 subtypes), and IgM and IgE.

Furthermore, the term "light chain" is interpreted to encompass a full-length light chain and fragments thereof, wherein the full-length light chain includes a variable domain VL having a sufficient variable region sequence to confer specificity to an antigen, and a constant region domain CL. The variable region domain of the light chain is located at the amino terminus of the light chain. The type of light chain may include kappa (κ) or lambda (λ) chains.

The term "complementarity determining region" (CDR) refers to a part of the antibody variable region that confers binding specificity to an antigen, representing the amino acid sequence of the hypervariable region of the immunoglobulin's heavy and light chains. Both the heavy and light chains can comprise three CDRs (HCDR1 (or CDRH1), HCDR2 (or CDRH2), HCDR3 (or CDRH3), LCDR1 (or CDRL1), LCDR2 (or CDRL2), and LCDR3 (or CDRL3)). The CDRs may provide key contact residues for the antibody to bind to an antigen or epitope. Throughout the specification, the terms "specifically binding" or "specifically recognizing" are understood in the same sense as commonly known to those skilled in the art, meaning that the antigen and antibody specifically interact to elicit an immunological response.

The complementarity determining regions (CDRs) described herein may be determined as defined according to the Kabat system.

Unless specifically stated otherwise, the term "antibody" may be understood to encompass an antigen-binding fragment of an antibody that retain the ability to bind to an antigen.

The term "antigen-binding fragment" used herein refer to a polypeptide in any type, which includes a portion (e.g., 6 CDRs as defined herein) capable of binding to an antigen, and, for example, may be at least one selected from the group consisting of scFv, scFv-Fc, (scFv)₂, Fab, Fab', F(ab')₂, diabody, and minibody, but with no limitations thereto.

Among the antigen-binding fragments, Fab has a structure composed of variable regions of light and heavy chains, the constant region of a light chain, and the first constant region (C_{H1}) of a heavy chain, with one antigen-binding site retained.

Fab' is different from Fab in that the former includes a hinge region having at least one cysteine residue at the C-terminal of the heavy chain C_{H1} domain.

F(ab')₂ antibody is formed through disulfide bridging of the cysteine residues in the hinge region of Fab'.

Fv is a minimal antibody fragment composed of only a heavy chain variable region and a light chain variable region. Recombination techniques of generating an Fv fragment are widely known in the art. Two-chain Fv includes a heavy chain variable region and a light chain variable region which are linked to each other by a non-covalent bond. Single-chain Fv generally includes a heavy-chain variable region and a light-chain variable region which are linked to each other by a covalent bond via a peptide linker or directly (without a linker) linked at the C-terminals to have a dimer structure. scFv-Fc is a conjugate in which Fc is linked to scFV. scFV-Fc-scFv is a structure in which scFv is linked to both terminals (N terminus and C terminus) of Fc. A minibody is structured to have scFv linked with C_{H3}. A diabody comprises two molecules of scFv.

The antigen-binding fragments may be obtained by chemical cleavage or enzymatic cleavage using protease (for example, Fab may be obtained by restrictively cleaving a whole antibody with papain, and an F(ab')₂ fragment may be obtained by cleaving with pepsin), or may be prepared by using a genetic recombination technique.

The term "hinge region" refers to a region between C_{H1} and C_{H2} domains within heavy chain of an antibody, which functions to provide flexibility for the antigen-binding site in the antibody.

Herein, the term "Fc" region refers to a heavy chain fragment that includes C_{H2} and C_{H3} domains in an antibody, and in some cases, may include the hinge region at the N-terminus of the C_{H2} and C_{H3} domains (i.e., the N-terminus of C_{H2} domain). Two Fc regions can be joined to each other by two or more disulfide bonds and/or hydrophobic interactions of the C_{H3} domain.

Herein, "bivalent antibodies or bivalent antigen-binding fragments" refer to those that include two antigen-binding sites, each comprising six CDRs (HCDR1, HCDR2, HCDR3, LCDR1, LCDR2, and LCDR3). The two antigen-binding sites included in the bivalent antibody or bivalent antigen-binding fragment may have the same antigen specificity, or they may have dual specificity, binding to two different antigens. Herein, "monovalent antibodies or monovalent antigen-binding fragments" include one antigen-binding site comprising six CDRs (HCDR1, HCDR2, HCDR3, LCDR1, LCDR2, and LCDR3).

As used herein, "bispecific" antibodies or antigen-binding fragments refer to antibodies or antigen-binding fragments thereof that target two antigens, being hybrid antibodies that bind to two different epitopes. The two epitopes of the bispecific antibody or antigen-binding fragment can be located on the same or different target proteins (antigens).

The antibodies provided herein may be monoclonal antibodies. Monoclonal antibodies can be manufactured using methods well known in the industry, for example, using phage display techniques. Alternatively, the antibodies can be produced as animal-derived monoclonal antibodies (e.g., from mice, rats, rabbits, etc.) using conventional methods or manufactured as humanized antibodies through standard processes.

As used herein, the term "antigen" or "immunogen" refers to a molecule or part of a molecule that can bind to an antigen-binding protein (e.g., an antibody or its immunologically functional antigen-binding fragment) and can be used to generate antibodies capable of binding to the antigen in animals. The antigen may include one or more epitopes that can interact with other antibodies or their fragments.

Herein, an "epitope" is a part of a molecule that is bound or recognized by an antibody or antigen-binding fragment and includes any determinant that can specifically bind to an antigen-binding protein, such as an antibody or T-cell receptor. Amino acid residues responsible for an epitope can be continuous or discontinuous. For example, they might not be consecutive in the polypeptide sequence, however, in an aspect of entire antibody molecule, they may be located in distinct by conformationally adjacent positions, thus being bound by one antigen-binding protein, such as a conformational epitope. The epitope determinants can be chemically activated groups presented on the surface by molecules, such as amino acids, sugar chains, phosphoryl groups, and/or sulfonyl groups, or they can possess specific three-dimensional structural features and/or specific charge characteristics.

Herein, an antibody (for example, CDR, variable region, or heavy chain/light chain, antigen-binding fragment etc.) "comprising (including) a specific amino acid sequence or consisting of a specific amino acid sequence" may refer to all cases in which the amino acid sequence is essentially comprised, and/or an insignificant mutation (for example, substitution, deletion, and/or addition of amino acid residue(s)) that does not affect antibody activity (for example, antigen binding affinity, pharmacological activity, etc.) is introduced into the amino acid sequence.

### Summary of the Invention

Provided according to an embodiment is an anti-ROR1 antibody or an antigen-binding fragment thereof, comprising:
HCDR1 comprising the amino acid sequence of SEQ ID NO: 1;
HCDR2 comprising the amino acid sequence of SEQ ID NO: 2;
HCDR3 comprising the amino acid sequence of SEQ ID NO: 3;
LCDR1 comprising the amino acid sequence of SEQ ID NO: 4;
LCDR2 comprising the amino acid sequence of SEQ ID NO: 5; and
LCDR3 comprising the amino acid sequence of SEQ ID NO: 6.

Another aspect provides an anti-ROR1 antibody or an antigen-binding fragment thereof comprising a heavy chain variable region of SEQ ID NO: 7 or 127, and a light chain variable region of SEQ ID NO: 8 or126.

Another aspect provides a nucleic acid molecule encoding the anti-ROR1 antibody or antigen-binding fragment thereof described above, a recombinant vector carrying same, and/or a recombinant cell comprising the recombinant vector.

Another aspect provides a pharmaceutical composition for the prevention or treatment of cancer, the composition comprising the anti-ROR1 antibody or antigen-binding fragment thereof described above, and a pharmaceutically acceptable excipient. Another aspect provides a method for the prevention or treatment of cancer, the method comprising a step of administering a pharmaceutically effective amount of the anti-ROR1 antibody or antigen-binding fragment thereof described above to a subject in need of the prevention or treatment of cancer. Another aspect provides a use of the anti-ROR1 antibody or antigen-binding fragment thereof described above for preventing or treating cancer or for preparing a pharmaceutical composition for the prevention or treatment of cancer.

In the pharmaceutical composition, method, and/or use, the cancer may be a cancer that expresses ROR1.

Another aspect provides a composition for detecting ROR1, comprising the previously described anti-ROR1 antibody or antigen-binding fragment thereof described above, a method for detecting ROR1 using the same, and a use of the composition for ROR1 detection.

Another aspect provides a composition for the diagnosis of cancer, the composition comprising the anti-ROR1 antibody or antigen-binding fragment thereof described above.

In the composition for the diagnosis of cancer, the cancer may be a cancer that expresses ROR1.

Another aspect provides an anti-ROR1/anti-4-1BB bispecific antibody comprising:
an anti-ROR1 antibody or an antigen-binding fragment thereof; and
an anti-4-1BB antibody or an antigen-binding fragment thereof,
wherein the anti-ROR1 antibody or antigen-binding fragment thereof comprises HCDR1 comprising the amino acid sequence of SEQ ID NO: 1, HCDR2 comprising the amino acid sequence of SEQ ID NO: 2, HCDR3 comprising the amino acid sequence of SEQ ID NO: 3, LCDR1 comprising the amino acid sequence of SEQ ID NO: 4, LCDR2 comprising the amino acid sequence of SEQ ID NO: 5, and LCDR3 comprising the amino acid sequence of SEQ ID NO: 6.

In the anti-ROR1/anti-4-1BB bispecific antibody described above, the anti-ROR1 antibody or antigen-binding fragment thereof may comprise a heavy chain variable region of SEQ ID NO: 7 or SEQ ID NO: 127, and a light chain variable region of SEQ ID NO: 8 or SEQ ID NO: 126.

In the anti-ROR1/anti-4-1BB bispecific antibody described above, the anti-4-1BB antibody or antigen-binding fragment thereof may comprise:
HCDR1 comprising an amino acid sequence of SEQ ID NO: 13, 14, or 15,
HCDR2 comprising an amino acid sequence of SEQ ID NO: 16, 17, or 18,
HCDR3 comprising an amino acid sequence of SEQ ID NO: 19, 20, 21, 22, or 23,
LCDR1 comprising an amino acid sequence of SEQ ID NO: 24 or 25,
LCDR2 comprising an amino acid sequence of SEQ ID NO: 26 or 27, and
LCDR3 comprising an amino acid sequence of SEQ ID NO: 28 or 29.

In the anti-ROR1/anti-4-1BB bispecific antibody described above, the anti-4-1BB antibody or antigen-binding fragment thereof may comprise:
(1) a heavy chain complementarity-determining region selected from:
   (a) HCDR1 comprising the amino acid of SEQ ID NO: 13, HCDR2 comprising the amino acid of SEQ ID NO: 16, and HCDR3 comprising the amino acid of SEQ ID NO: 19,
   (b) HCDR1 comprising the amino acid of SEQ ID NO: 13, HCDR2 comprising the amino acid of SEQ ID NO: 16, and HCDR3 comprising the amino acid of SEQ ID NO: 20,
   (c) HCDR1 comprising the amino acid of SEQ ID NO: 13, HCDR2 comprising the amino acid of SEQ ID NO: 16, and HCDR3 comprising the amino acid of SEQ ID NO: 21,
   (d) HCDR1 comprising the amino acid of SEQ ID NO: 14, HCDR2 comprising the amino acid of SEQ ID NO: 17, and HCDR3 comprising the amino acid of SEQ ID NO: 22, and
   (e) HCDR1 comprising the amino acid of SEQ ID NO: 15, HCDR2 comprising the amino acid of SEQ ID NO: 18, and HCDR3 comprising the amino acid of SEQ ID NO: 23, and
(2) a light chain complementarity-determining region selected from:
   (a) LCDR1 comprising the amino acid of SEQ ID NO: 24, LCDR2 comprising the amino acid of SEQ ID NO: 26, and LCDR3 comprising the amino acid of SEQ ID NO: 28, and
   (b) LCDR1 comprising the amino acid of SEQ ID NO: 24, LCDR2 comprising the amino acid of SEQ ID NO: 26, and LCDR3 comprising the amino acid of SEQ ID NO: 28.

In the anti-ROR1/anti-4-1BB bispecific antibody described above, the anti-4-1BB antibody or antigen-binding fragment thereof may comprise:
a heavy chain variable region comprising an amino acid sequence of SEQ ID NO: 30, 31, 32, 33, 34, 35, 36, 37, 38, 39, 40, or 41; and
a light chain variable region comprising an amino acid sequence of SEQ ID NO: 42, 43, 44, 45, 46, or 47.

Another aspect provides a nucleic acid molecule encoding the anti-ROR1/anti-4-1BB bispecific antibody or antigen-binding fragment thereof described above, a recombinant vector carrying same, and/or a recombinant cell comprising the recombinant vector.

Another aspect provides a pharmaceutical composition for the prevention or treatment of cancer, the composition comprising the anti-ROR1/anti-4-1BB bispecific antibody or antigen-binding fragment thereof described above, and a pharmaceutically acceptable excipient. Another aspect provides a method for the prevention or treatment of cancer, the method comprising a step of administering a pharmaceutically effective amount of the anti-ROR1/anti-4-1BB bispecific antibody or antigen-binding fragment thereof described above to a subject in need of the prevention or treatment of cancer. Another aspect provides a use of the anti-ROR1/anti-4-1BB bispecific antibody or antigen-binding fragment thereof described above for preventing or treating cancer or for preparing a pharmaceutical composition for the prevention or treatment of cancer.

In the pharmaceutical composition, method, and/or use, the cancer may be a cancer that expresses ROR1.

Another aspect provides an anti-ROR1/anti-B7-H3 bispecific antibody comprising:
an anti-ROR1 antibody or an antigen-binding fragment thereof; and
an anti- B7-H3 antibody or an antigen-binding fragment thereof,
wherein the anti-ROR1 antibody or antigen-binding fragment thereof comprises HCDR1 comprising the amino acid sequence of SEQ ID NO: 1, HCDR2 comprising the amino acid sequence of SEQ ID NO: 2, HCDR3 comprising the amino acid sequence of SEQ ID NO: 3, LCDR1 comprising the amino acid sequence of SEQ ID NO: 4, LCDR2 comprising the amino acid sequence of SEQ ID NO: 5, and LCDR3 comprising the amino acid sequence of SEQ ID NO: 6.

In the anti-ROR1/anti-B7-H3 bispecific antibody described above, the anti-ROR1 antibody or antigen-binding fragment thereof may comprise a heavy chain variable region of SEQ ID NO: 7 or SEQ ID NO: 127, and a light chain variable region of SEQ ID NO: 8 or SEQ ID NO: 126.

In the anti-ROR1/anti-B7-H3 bispecific antibody described above, the anti-B7-H3 antibody or antigen-binding fragment thereof may comprise:
HCDR1 comprising an amino acid sequence selected from SEQ ID NOS: 48 to 51;
HCDR2 comprising an amino acid sequence selected from SEQ ID NOS: 52 to 57;
HCDR3 comprising an amino acid sequence selected from SEQ ID NOS: 58 to 62;
LCDR1 comprising an amino acid sequence selected from SEQ ID NOS: 63 to 67;
LCDR2 comprising an amino acid sequence selected from SEQ ID NOS: 68 to 73; and
LCDR3 comprising an amino acid sequence selected from SEQ ID NOS: 74 to 79.

In the anti-ROR1/anti-B7-H3 bispecific antibody described above, the anti-B7-H3 antibody or antigen-binding fragment thereof may comprise:
(i) a heavy chain complementarity-determining region selected from:
   (a) HCDR1 comprising the amino acid of SEQ ID NO: 48, HCDR2 comprising the amino acid of SEQ ID NO: 52, and HCDR3 comprising the amino acid of SEQ ID NO: 58,
   (b) HCDR1 comprising the amino acid of SEQ ID NO: 49, HCDR2 comprising the amino acid of SEQ ID NO: 53, and HCDR3 comprising the amino acid of SEQ ID NO: 59,
   (c) HCDR1 comprising the amino acid of SEQ ID NO: 50, HCDR2 comprising the amino acid of SEQ ID NO: 54, and HCDR3 comprising the amino acid of SEQ ID NO: 60,
   (d) HCDR1 comprising the amino acid of SEQ ID NO: 48, HCDR2 comprising the amino acid of SEQ ID NO: 55, and HCDR3 comprising the amino acid of SEQ ID NO: 61, and
   (e) HCDR1 comprising the amino acid of SEQ ID NO: 51, HCDR2 comprising the amino acid of SEQ ID NO: 56, and HCDR3 comprising the amino acid of SEQ ID NO: 62, and
   (f) HCDR1 comprising the amino acid of SEQ ID NO: 50, HCDR2 comprising the amino acid of SEQ ID NO: 57, and HCDR3 comprising the amino acid of SEQ ID NO: 60, and
(ii) a light chain complementarity-determining region selected from:
   (a) LCDR1 comprising the amino acid of SEQ ID NO: 63, LCDR2 comprising the amino acid of SEQ ID NO: 68, and LCDR3 comprising the amino acid of SEQ ID NO: 74,
   (b) LCDR1 comprising the amino acid of SEQ ID NO: 64, LCDR2 comprising the amino acid of SEQ ID NO: 69, and LCDR3 comprising the amino acid of SEQ ID NO: 75;
   (c) LCDR1 comprising the amino acid of SEQ ID NO: 65, LCDR2 comprising the amino acid of SEQ ID NO: 70, and LCDR3 comprising the amino acid of SEQ ID NO: 76;
   (d) LCDR1 comprising the amino acid of SEQ ID NO: 66, LCDR2 comprising the amino acid of SEQ ID NO: 71, and LCDR3 comprising the amino acid of SEQ ID NO: 77;
   (e) LCDR1 comprising the amino acid of SEQ ID NO: 67, LCDR2 comprising the amino acid of SEQ ID NO: 72, and LCDR3 comprising the amino acid of SEQ ID NO: 78; and
   (f) LCDR1 comprising the amino acid of SEQ ID NO: 65, LCDR2 comprising the amino acid of SEQ ID NO: 73, and LCDR3 comprising the amino acid of SEQ ID NO: 76.

In the anti-ROR1/anti-B7-H3 bispecific antibody described above, the anti-B7-H3 antibody or antigen-binding fragment thereof may comprise:
a heavy chain variable region comprising an amino acid sequence of SEQ ID NO: 80 to 91; and
a light chain variable region comprising an amino acid sequence of SEQ ID NO: 92 to 103.

Another aspect provides a nucleic acid molecule encoding the anti-ROR1/anti-B7-H3 bispecific antibody or antigen-binding fragment thereof described above, a recombinant vector carrying same, and/or a recombinant cell comprising the recombinant vector.

Another aspect provides a pharmaceutical composition for the prevention or treatment of cancer, the composition comprising the anti-ROR1/anti-B7-H3 bispecific antibody or antigen-binding fragment thereof described above, and a pharmaceutically acceptable excipient. Another aspect provides a method for the prevention or treatment of cancer, the method comprising a step of administering a pharmaceutically effective amount of the anti-ROR1/anti-B7-H3 bispecific antibody or antigen-binding fragment thereof described above to a subject in need of the prevention or treatment of cancer. Another aspect provides a use of the anti-ROR1/anti-B7-H3 bispecific antibody or antigen-binding fragment thereof described above for preventing or treating cancer or for preparing a pharmaceutical composition for the prevention or treatment of cancer.

In the pharmaceutical composition, method, and/or use, the cancer may be a cancer that expresses ROR1, B7-H3, or both of them.

Another aspect provides an antibody-drug conjugate (ADC) that comprises the previously described anti-ROR1/anti-B7-H3 bispecific antibody or antigen-binding fragment thereof linked to a cytotoxic drug.

The antibody-drug conjugate may have an antibody-linker-drug structure.

In the antibody-drug conjugate, the cytotoxic drug may be an anticancer agent.

Another aspect provides a pharmaceutical composition for the prevention or treatment of cancer, the composition comprising the antibody-drug conjugate described above and a pharmaceutically acceptable excipient. Another aspect provides a method for the prevention or treatment of cancer, the method comprising a step of administering a pharmaceutically effective amount of the anti-ROR1/anti-B7-H3 bispecific antibody or antigen-binding fragment thereof described above to a subject in need of the prevention or treatment of cancer. Another aspect provides a use of the anti-ROR1/anti-B7-H3 bispecific antibody or antigen-binding fragment thereof described above for preventing or treating cancer or for preparing a pharmaceutical composition for the prevention or treatment of cancer.

In the pharmaceutical composition, method, and/or use, the cancer may be a cancer that expresses ROR1, B7-H3, or both of them.

Hereinafter, a detailed description will be given of the present disclosure:

### Anti-ROR1 antibody or antigen-binding fragment thereof

An aspect provides an anti-ROR1 antibody or an antigen-binding fragment thereof that binds to ROR1. The anti-ROR1 antibody or antigen-binding fragment thereof provided herein can target (specifically recognize and/or bind to) human ROR1 (for example, the extracellular domain). The antibody or antigen-binding fragment thereof not only possesses inhibitory activity against ROR1 but also enhances stability and developability, thereby enjoying the advantages of allowing for the economical production of more effective and safer antibody pharmaceuticals compared to conventional therapeutic antibodies.

The anti-ROR1 antibody or antigen-binding fragment thereof may comprise:
HCDR1 comprising the amino acid sequence of SEQ ID NO: 1,
HCDR2 comprising the amino acid sequence of SEQ ID NO: 2,
HCDR3 comprising the amino acid sequence of SEQ ID NO: 3,
LCDR1 comprising the amino acid sequence of SEQ ID NO: 4,
LCDR2 comprising the amino acid sequence of SEQ ID NO: 5, and
LCDR3 comprising the amino acid sequence of SEQ ID NO: 6.

Herein, the complementarity determining region (CDR) can be determined based on the CDR definitions according to the Kabat system.

In an embodiment, the amino acid sequences (N→C) of the six CDRs (HCDR1, HCDR2, HCDR3, LCDR1, LCDR2, and LCDR3) that may be comprised in the anti-ROR1 antibody or antigen-binding fragment thereof provided herein are outlined as follows:
HCDR1: NYDMS (SEQ ID NO: 1)
HCDR2: AIYHSGSSKYYADSVKG (SEQ ID NO: 2)
HCDR3: GGSGAWDTGFDY (SEQ ID NO: 3)
LCDR1: SGSSSNIGSNDVS (SEQ ID NO: 4)
LCDR2: YENNRPS (SEQ ID NO: 5)
LCDR3: GAWDDSLSGYV (SEQ ID NO: 6)
(HCDR1, HCDR2, and HCDR3 represent the heavy chain complementarity determining regions, and LCDR1, LCDR2, and LCDR3 represent the light chain complementarity determining regions.)

The anti-ROR1 antibody or antigen-binding fragment thereof may comprise:
a heavy chain complementarity-determining region (CDR) or a heavy chain variable region comprising the heavy chain CDR, wherein the heavy chain CDR comprises HCDR1 comprising the amino acid sequence of SEQ ID NO: 1, HCDR2 comprising the amino acid sequence of SEQ ID NO: 2, and HCDR3 comprising the amino acid sequence of SEQ ID NO: 3; and
a light chain complementarity-determining region (CDR) or a light chain variable region comprising the light chain CDR, wherein the light chain CDR comprises LCDR1 comprising the amino acid sequence of SEQ ID NO: 4, LCDR2 comprising the amino acid sequence of SEQ ID NO: 5, and LCDR3 comprising the amino acid sequence of SEQ ID NO: 6.

The heavy chain variable region and light chain variable region of the anti-ROR1 antibody or antigen-binding fragment thereof may be derived from an IgG-derived antibody or an antigen-binding fragment thereof. They may comprise wild-type sequences, such as framework (HFR1, HFR2, HFR3, HFR4, LFR1, LFR2, LFR3, and/or LFR4) regions with a mutation introduced thereinto.

In an embodiment, the mutation may be intended for stabilization and/or disulfide bridge formation. For instance, the antigen-binding fragment may have one or more (e.g., one) G (Gly) → C (Cys) mutations (where G is substituted by C) introduced in the framework region of the variable domain in the antibody, but with no limitations thereto. In a particular embodiment, if the ROR1 antibody is an intact IgG antibody or a part thereof (e.g., Fab or Fab-Fc), the heavy chain variable region and light chain variable region comprised therein may be derived from wild-type IgG. In another embodiment, if the anti-ROR1 antibody is an antigen-binding fragment of anti-ROR1 antibody (e.g., scFv, etc.), the heavy chain variable region and light chain variable region comprised therein may have G→C mutations introduced in the framework region thereof.

In an embodiment, the anti-ROR1 antibody or antigen-binding fragment thereof may comprise a heavy chain variable region of SEQ ID NO: 7 or 127, and a light chain variable region of SEQ ID NO: 8 or 126. More specifically, the anti-ROR1 antibody or antigen-binding fragment thereof may comprise the heavy chain variable region of SEQ ID NO: 7 and the light chain variable region of SEQ ID NO: 8, or the heavy chain variable region of SEQ ID NO: 127 and the light chain variable region of SEQ ID NO: 126, but is not limited thereto.

In certain cases (for example, when produced recombinantly), the heavy chain variable region and/or light chain variable region may further comprise an appropriate signal sequence at the N-terminus.

The amino acid sequences of the heavy chain variable region and light chain variable region that can be comprised in the anti-ROR1 antibody or antigen-binding fragment thereof provided herein are exemplified in Table 1 below:

**TABLE 1**

| Variable region | Amino acid sequence (N→C) | SEQ ID NO: |
|---|---|---|
| Heavy chain variable region (IgG) | EVQLLESGGGLVQPGGSLRLSCAASGFTFSNYDMSWVR QAPGKGLEWVSAIYHSGSSKYYADSVKGRFTISRDNSKN TLYLQMNSLRAEDTAVYYCARGGSGAWDTGFDYWGQ GTLVTVSS | 7 |
| Heavy chain variable region (scFv) | EVQLLESGGGLVQPGGSLRLSCAASGFTFSNYDMSWVR QAPGKCLEWVSAIYHSGSSKYYADSVKGRFTISRDNSKN TLYLQMNSLRAEDTAVYYCARGGSGAWDTGFDYWGQ GTLVTVSS | 127 |
| Light chain variable region (IgG) | QSVLTQPPSASGTPGQRVTISCSGSSSNIGSNDVSWYQQL PGTAPKLLIYYENNRPSGVPDRFSGSKSGTSASLAISGLRS EDEADYYCGAWDDSLSGYVFGGGTKLTVL | 8 |
| Light chain variable region (scFv) | QSVLTQPPSASGTPGQRVTISCSGSSSNIGSNDVSWYQQL PGTAPKLLIYYENNRPSGVPDRFSGSKSGTSASLAISGLRS | 126 |
| | EDEADYYCGAWDDSLSGYVFGCGTKLTVL | |

| | | |
|---|---|---|
| (In Table 1, CDR1, CDR2, and CDR3 of heavy and light chains are underlined in that order) | | |

The receptor tyrosine kinase-like orphan receptor (ROR1), that is an antigen targeted by the anti-ROR1 antibody or antigen-binding fragment thereof provided herein, is a transmembrane protein that belongs to the Receptor Tyrosine Kinase (RTK) family. The anti-ROR1 antibody or antigen-binding fragment thereof can recognize (bind to) portions of the ROR1 protein that are present on the cell membrane or the extracellular domain that is not present on the cell membrane. The ROR1 protein may be a mammalian ROR1 protein, for example, human ROR1 protein. The amino acid sequence of the human ROR1 protein can be found under NCBI Accession No. NP_005003.2 (937 aa), and the nucleic acid sequence encoding same can be under NCBI Accession No. NM_005012.3, but with no limitations thereto. In another embodiment, the ROR1 protein can be represented by mouse ROR1 protein (GenBank Accession No. BAA75480.1).

The antibodies or antigen-binding fragments thereof provided herein may have an antigen binding affinity of 5 nM or less, 1 nM or less, 0.5 nM or less, 0.2 nM or less, 0.1 nM or less, 0.05 nM or less, 0.01 nM or less, 0.005 nM or less, or 0.001 nM or less, for example, 0.001 nM to 5 nM, or 0.001 nM to 1 nM, as measured, for example, for binding affinity (K_{D}) by surface plasmon resonance (SPR) or for EC₅₀ by ELISA, but with no limitations thereto.

The anti-ROR1 antibody or antigen-binding fragment thereof provided herein has functions such as ROR1 inhibition (for example, suppression of ROR 1 expression and/or activity), cancer cell targeting through specific binding to cancer cells (for example, cancer cells expressing ROR1), and anticancer activity (for example, inducing cancer cell apoptosis, inhibiting cancer cell proliferation, etc.). Therefore, the anti-ROR1 antibody or antigen-binding fragment thereof can find advantageous findings in the prevention and/or treatment of cancer.

Another aspect provides a composition or a kit for detecting ROR1, the composition or kit comprising at least one selected from a group consisting of the anti-ROR1 antibody or antigen-binding fragment thereof, and nucleic acid molecules coding therefor.

Another aspect provides a method for detecting ROR1 in a biological sample, the method comprising a step of contacting the biological sample in need of detecting ROR1 with at least one selected from a group consisting of the anti-ROR1 antibody or antigen-binding fragment thereof, and a nucleic acid molecule coding therefor. Following the contacting step, the method may further comprise a step of measuring the binding in the biological sample treated (contacted) with the antibody, antigen-binding fragment, and/or nucleic acid molecule. In an embodiment, the method may be performed in vitro or in vivo.

Another aspect provides a composition or kit for the diagnosis of cancer, the composition or kit comprising at least one selected from a group consisting of the anti-ROR1 antibody or antigen-binding fragment thereof, and a nucleic acid molecule coding therefore. The cancer may be a cancer that expresses ROR1.

Another aspect provides a method for diagnosing cancer in a biological sample or a subject from which the biological sample is derived or isolated or for providing information for cancer diagnosis, the method comprising a step of contacting the biological sample with one or more selected from a group consisting of the anti-ROR1 antibody or antigen-binding fragment thereof and a nucleic acid molecule coding therefor. Following the contacting step, the method may involve measuring the binding in the biological sample treated (contacted) with the antibody, antigen-binding fragment, and/or nucleic acid molecule. Furthermore, if binding is detected in the treated (contacted) biological sample, the method may comprise determining (confirming) the biological sample or the subject derived from (or isolated from) the biological sample as a cancer patient or further assessing that such a subject is likely to respond to treatment with the anti-ROR1 antibody or its antigen-binding fragment. This method can be performed in vitro or in vivo. The cancer may be a cancer that expresses ROR1.

In the compositions and/or methods for detecting ROR1 and/or diagnosing cancer, provided herein, the biological sample may be derived (or isolated) from an animal selected from mammals comprising primates such as humans, monkeys, and the like, and rodents such as rats, mice, etc, and for example, the biological sample may be cells, tissues, bodily fluids (e.g., serum), transformed cells, or cultures thereof, originating from humans or may be human-derived cells, tissues, bodily fluids (e.g., serum).

Another aspect provides a nucleic acid molecule encoding the anti-ROR1 antibody or antigen-binding fragment thereof.

Specifically, an embodiment provides a nucleic acid molecule encoding the heavy chain complementarity-determining region, the heavy chain variable region, or the heavy chain of the anti-ROR1 antibody.

Another aspect provides a nucleic acid molecule encoding the light chain complementarity-determining region, the light chain variable region, or the light chain of the anti-ROR1 antibody.

Another aspect provides a recombinant vector collectively or separately carrying:
(1) a nucleic acid molecule encoding the heavy chain complementarity-determining region, heavy chain variable region, or heavy chain of the anti-ROR1 antibody, and
(2) a nucleic acid molecule encoding the light chain complementarity-determining region, light chain variable region, or light chain of the anti-ROR1 antibody. The recombinant vector may be an expression vector designed for the expression of the nucleic acid molecules.

Another aspect provides a recombinant cell comprising the recombinant vector.

An aspect of the present application provides a bispecific antibody or an antigen-binding fragment thereof, which comprises the anti-ROR1 antibody or antigen-binding fragment thereof.

More specifically, provided is a bispecific antibody or an antigen-binding fragment thereof, which comprises:
a first targeting moiety that targets (specifically binds to and/or recognizes) a first antigen, and
a second targeting moiety that targets (specifically binds to and/or recognizes) a second antigen,
wherein the first antigen and the second antigen are different from each other, and
one of the first targeting moiety and the targeting moiety comprises the anti-ROR1 antibody or antigen-binding fragment thereof.

For instance, in the bispecific antibody or antigen-binding fragment thereof, one of the antigens might be ROR1 and the other may be selected from 4-1BB and B7-H3, but with no limitations thereto. By way of example, one of the antigens may be selected from cancer cell-expressed antigens, immune checkpoint proteins, or T cell surface antigens expressed in different types of cancer cells.

The description provided herein for the anti-ROR1 antibody or antigen-binding fragment thereof can also be applied to the anti-ROR1/4-1BB bispecific antibody, anti-ROR1/B7-H3 bispecific antibody, or antigen-binding fragments thereof which will be described below. Similarly, the description to be given regarding the anti-ROR1/4-1BB bispecific antibody, anti-ROR1/B7-H3 bispecific antibody, or antigen-binding fragments thereof can equally apply to the anti-ROR1 antibody or antigen-binding fragment thereof.

### Anti-ROR1/anti-4-1BB bispecific antibody

An aspect provides an anti-ROR1/anti-4-1BB bispecific antibody comprising:
(1) an anti-ROR1 antibody or an antigen-binding fragment thereof as an ROR1 targeting moiety; and
(2) an anti-4-1BB antibody or an antigen-binding fragment thereof as a 4-1BB targeting moiety. The anti-ROR1/anti-4-1BB bispecific antibody targets (specifically binds to and/or recognizes) both ROR1 and 4-1BB.

The anti-ROR1 antibody or antigen-binding fragment thereof, serving as the ROR1 targeting moiety, is a protein that targets (specifically binds to and/or recognizes) ROR1. More specifically, the ROR1 targeting moiety may be the previously described anti-ROR1 antibody or antigen-binding fragment thereof. The description provided above regarding the anti-ROR1 antibody or antigen-binding fragment thereof may be equally applicable to the anti-ROR1 antibody or antigen-binding fragment thereof comprised in the anti-ROR1/anti-4-1BB bispecific antibody.

The anti-4-1BB antibody or antigen-binding fragment thereof as the 4-1BB-targeting moiety may be a protein that targets (specifically binds to and/or recognizes) 4-1BB.

4-1BB, also known as CD137 or tumor necrosis factor receptor superfamily member 9 (TNFRSF9), is a member of the TNF-receptor superfamily (TNFRSF), functioning as a co-stimulatory molecule expressed following the activation of both innate and adaptive immune cells. 4-1BB plays a crucial role in regulating the activity of various immune cells. The antigen targeted by the anti-4-1BB antibody or antigen-binding fragment thereof provided herein may be mammalian 4-1BB, for example, human 4-1BB. The amino acid sequence of human 4-1BB can be found under NCBI Accession No. NP_001552.2, but with no limitations thereto.

For instance, the anti-4-1BB antibody or antigen-binding fragment thereof may comprise:
CDR-H1 (HCDR1) comprising an amino acid sequence of SEQ ID NO: 13, 14, or 15;
HCDR2 comprising an amino acid sequence of SEQ ID NO: 16, 17, or 18;
HCDR3 comprising an amino acid sequence of SEQ ID NO: 19, 20, 21, 22, or 23;
LCDR1 comprising an amino acid sequence of SEQ ID NO: 24 or 25;
LCDR2 comprising an amino acid sequence of SEQ ID NO: 26 or 27; and
LCDR3 comprising an amino acid sequence of SEQ ID NO: 28 or 29.

The amino acid sequences of the CDRs of the anti-4-1BB antibody or antigen-binding fragment are exemplified in Table 2.

**TABLE 2**

| **SEQ ID NO: #** | **HCDR1** | **SEQ ID NO: #** | **HCDR2** | **SEQ ID NO: #** | **HCDR3** |
|---|---|---|---|---|---|
| 13 | SYDMS | 16 | WISYSGGSI YYADSVKG | 19 | DGQRNSMREF DY |
| | | | | 20 | DAQRNSMREF DY |
| | | | | 21 | DAQRQSMREF DY |
| 14 | GYDMS | 17 | VIYPDDGNT YYADSVKG | 22 | HGGQKPTTKS SSAYGMDG |
| 15 | SYWNM | 18 | EINPGNGHT NYNEKFKS | 23 | SFTTARAFAY |

| **SEQ ID NO: #** | **LCDR1** | **SEQ ID NO: #** | **LCDR2** | **SEQ ID NO: #** | **LCDR3** |
|---|---|---|---|---|---|
| 24 | SGSSSNIG NNYVT | 26 | ADSHRPS | 28 | ATWDYSLSGY V |
| 25 | RASQTISD YLH | 27 | YASQSIS | 29 | QDGHSFPPT |

For example, the anti-4-1BB antibody or antigen-binding fragment thereof may comprise:
HCDR1 comprising the amino acid sequence of SEQ ID NO: 13, HCDR2 comprising the amino acid sequence of SEQ ID NO: 16, HCDR3 comprising the amino acid sequence of SEQ ID NO: 19, LCDR1 comprising the amino acid sequence of SEQ ID NO: 24, LCDR2 comprising the amino acid sequence of SEQ ID NO: 26, and LCDR3 comprising the amino acid sequence of SEQ ID NO: 28;
HCDR1 comprising the amino acid sequence of SEQ ID NO: 13, HCDR2 comprising the amino acid sequence of SEQ ID NO: 16, HCDR3 comprising the amino acid sequence of SEQ ID NO: 20, LCDR1 comprising the amino acid sequence of SEQ ID NO: 24, LCDR2 comprising the amino acid sequence of SEQ ID NO: 26, and LCDR3 comprising the amino acid sequence of SEQ ID NO: 28;
HCDR1 comprising the amino acid sequence of SEQ ID NO: 13, HCDR2 comprising the amino acid sequence of SEQ ID NO: 16, HCDR3 comprising the amino acid sequence of SEQ ID NO: 21, LCDR1 comprising the amino acid sequence of SEQ ID NO: 24, LCDR2 comprising the amino acid sequence of SEQ ID NO: 26, and LCDR3 comprising the amino acid sequence of SEQ ID NO: 28;
HCDR1 comprising the amino acid sequence of SEQ ID NO: 13, HCDR2 comprising the amino acid sequence of SEQ ID NO: 16, HCDR3 comprising the amino acid sequence of SEQ ID NO: 19, LCDR1 comprising the amino acid sequence of SEQ ID NO: 25, LCDR2 comprising the amino acid sequence of SEQ ID NO: 27, and LCDR3 comprising the amino acid sequence of SEQ ID NO: 29;
HCDR1 comprising the amino acid sequence of SEQ ID NO: 13, HCDR2 comprising the amino acid sequence of SEQ ID NO: 16, HCDR3 comprising the amino acid sequence of SEQ ID NO: 20, LCDR1 comprising the amino acid sequence of SEQ ID NO: 25, LCDR2 comprising the amino acid sequence of SEQ ID NO: 27, and LCDR3 comprising the amino acid sequence of SEQ ID NO: 29;
HCDR1 comprising the amino acid sequence of SEQ ID NO: 13, HCDR2 comprising the amino acid sequence of SEQ ID NO: 16, HCDR3 comprising the amino acid sequence of SEQ ID NO: 21, LCDR1 comprising the amino acid sequence of SEQ ID NO: 25, LCDR2 comprising the amino acid sequence of SEQ ID NO: 27, and LCDR3 comprising the amino acid sequence of SEQ ID NO: 29;
HCDR1 comprising the amino acid sequence of SEQ ID NO: 14, HCDR2 comprising the amino acid sequence of SEQ ID NO: 17, HCDR3 comprising the amino acid sequence of SEQ ID NO: 22, LCDR1 comprising the amino acid sequence of SEQ ID NO: 24, LCDR2 comprising the amino acid sequence of SEQ ID NO: 26, and LCDR3 comprising the amino acid sequence of SEQ ID NO: 28;
HCDR1 comprising the amino acid sequence of SEQ ID NO: 14, HCDR2 comprising the amino acid sequence of SEQ ID NO: 17, HCDR3 comprising the amino acid sequence of SEQ ID NO: 22, LCDR1 comprising the amino acid sequence of SEQ ID NO: 25, LCDR2 comprising the amino acid sequence of SEQ ID NO: 27, and LCDR3 comprising the amino acid sequence of SEQ ID NO: 29;
HCDR1 comprising the amino acid sequence of SEQ ID NO: 15, HCDR2 comprising the amino acid sequence of SEQ ID NO: 18, HCDR3 comprising the amino acid sequence of SEQ ID NO: 23, LCDR1 comprising the amino acid sequence of SEQ ID NO: 24, LCDR2 comprising the amino acid sequence of SEQ ID NO: 26, and LCDR3 comprising the amino acid sequence of SEQ ID NO: 28; or
HCDR1 comprising the amino acid sequence of SEQ ID NO: 15, HCDR2 comprising the amino acid sequence of SEQ ID NO: 18, HCDR3 comprising the amino acid sequence of SEQ ID NO: 23, LCDR1 comprising the amino acid sequence of SEQ ID NO: 25, LCDR2 comprising the amino acid sequence of SEQ ID NO: 27, and LCDR3 comprising the amino acid sequence of SEQ ID NO: 29.

In another embodiment, the anti-4-1BB antibody or antigen-binding fragment thereof may comprise:
a heavy chain variable region comprising HCDR1 with an amino acid sequence of SEQ ID NO: 13, 14, or 15, HCDR2 with an amino acid sequence of SEQ ID NO: 16, 17, or 18, and HCDR3 with an amino acid sequence of SEQ ID NO: 19, 20, 21, 22, or 23; and
a light chain variable region comprising LCDR1 with an amino acid sequence of SEQ ID NO: 24 or 25, LCDR2 with an amino acid sequence of SEQ ID NO: 26 or 27, and LCDR3 with an amino acid sequence of SEQ ID NO: 28 or 29.

The heavy and light chain variable regions of the anti-4-1BB antibody or antigen-binding fragment thereof may be derived from IgG antibodies or antigen-binding fragments thereof. The antibodies or antigen-binding fragments thereof may comprise wild-type sequences, such as the framework regions (HFR1, HFR2, HFR3, HFR4, LFR1, LFR2, LFR3, and/or LFR4), with a mutation introduced thereinto for stabilization and/or creation of disulfide bridges. For instance, the antigen-binding fragment may have one or more (e.g., one) G(Gly) to C(Cys) mutations (G substituted by C) introduced into the framework region of the variable domain in the antibody, but with no limitations thereto. In an embodiment, if the 4-1BB targeting moiety is an intact IgG antibody or a part thereof (e.g., Fab or Fc-Fab), the heavy and light chain variable regions comprised therein may be derived from wild-type IgG. In another embodiment, if the 4-1BB targeting moiety is an antigen-binding fragment of the anti-4-1BB antibody (e.g., scFv), the heavy and light chain variable regions comprised therein may have a G to C mutation introduced in the framework region.

In an embodiment, the anti-4-1BB antibody or antigen-binding fragment thereof may comprise:
a heavy chain variable region comprising an amino acid sequence of SEQ ID NO: 30, 31, 32, 33, 34, 35, 36, 37, 38, 39, 40, or 41; and
a light chain variable region comprising an amino acid sequence of SEQ ID NO: 42, 43, 44, 45, 46, or 47.
The amino acid sequences (N→C) of the variable regions of the anti-4-1BB antibody or antigen-binding fragment thereof are exemplified in Table 3.

**TABLE 3**

| **SEQ ID NO: #** | **Heavy chain variable region targeting 4-1BB** |
|---|---|
| 30 | EVQLLESGGGLVQPGGSLRLSCAASGFTFSSYDMSWVRQAPGKGLE WVSWISYSGGSIYYADSVKGRFTISRDNSKNTLYLQMNSLRAEDTAV YYCARDGQRNSMREFDYWGQGTLVTVSS |
| 31 | EVQLLESGGGLVQPGGSLRLSCAASGFTFSSYDMSWVRQAPGKGLE |
| | WVSWISYSGGSIYYADSVKGRFTISRDNSKNTLYLQMNSLRAEDTAV YYCARDAQRNSMREFDYWGQGTLVTVSS |
| 32 | EVQLLESGGGLVQPGGSLRLSCAASGFTFSSYDMSWVRQAPGKGLE WVSWISYSGGSIYYADSVKGRFTISRDNSKNTLYLQMNSLRAEDTAV YYCARDAQRQSMREFDYWGQGTLVTVSS |
| 33 | EVQLLESGGGLVQPGGSLRLSCAASGFTFSGYDMSWVRQAPGKGLE WVSVIYPDDGNTYYADSVKGRFTISRDNSKNTLYLQMNSLRAEDAA VYYCAKHGGQKPTTKSSSAYGMDGWGQGTLVTVSS |
| 34 | EVQLLESGGGLVQPGGSLRLSCAASGFTFSGYDMSWVRQAPGKGLE WVSVIYPDDGNTYYADSVKGRFTISRDNSKNTLYLQMNSLRAEDTA VYYCAKHGGQKPTTKSSSAYGMDGWGQGTLVTVSS |
| 35 | QVQLQQSGAEVIKPGASVKLSCKASGYTFSSYWMHWVRQAPGQGLE WIGEINPGNGHTNYNEKFKSRATLTGDTSTSTVYMELSSLRSEDTAVY YCARSFTTARAFAYWGQGTLVTVSS |
| 36 | EVQLLESGGGLVQPGGSLRLSCAASGFTFSSYDMSWVRQAPGKCLE WVSWISYSGGSIYYADSVKGRFTISRDNSKNTLYLQMNSLRAEDTAV YYCARDGQRNSMREFDYWGQGTLVTVSS |
| 37 | EVQLLESGGGLVQPGGSLRLSCAASGFTFSSYDMSWVRQAPGKCLE WVSWISYSGGSIYYADSVKGRFTISRDNSKNTLYLQMNSLRAEDTAV YYCARDAQRNSMREFDYWGQGTLVTVSS |
| 38 | EVQLLESGGGLVQPGGSLRLSCAASGFTFSSYDMSWVRQAPGKCLE WVSWISYSGGSIYYADSVKGRFTISRDNSKNTLYLQMNSLRAEDTAV YYCARDAQRQSMREFDYWGQGTLVTVSS |
| 39 | EVQLLESGGGLVQPGGSLRLSCAASGFTFSGYDMSWVRQAPGKCLE WVSVIYPDDGNTYYADSVKGRFTISRDNSKNTLYLQMNSLRAEDAA VYYCAKHGGQKPTTKSSSAYGMDGWGQGTLVTVSS |
| 40 | EVQLLESGGGLVQPGGSLRLSCAASGFTFSGYDMSWVRQAPGKCLE WVSVIYPDDGNTYYADSVKGRFTISRDNSKNTLYLQMNSLRAEDTA VYYCAKHGGQKPTTKSSSAYGMDGWGQGTLVTVSS |
| 41 | QVQLQQSGAEVIKPGASVKLSCKASGYTFSSYWMHWVRQAPGQCLE WIGEINPGNGHTNYNEKFKSRATLTGDTSTSTVYMELSSLRSEDTAVY YCARSFTTARAFAYWGQGTLVTVSS |

| **SEQ ID NO: #** | **Light chain variable region targeting 4-1BB** |
|---|---|
| 42 | QSVLTQPPSASGTPGRRVTISCSGSSSNIGNNYVTWYQQLPGTAPKLLI YADSHRPSGVPDRFSGSKSGTSASLAISGLRSEDEADYYCATWDYSLS |
| | GYVFGGGTKLTVL |
| 43 | QSVLTQPPSASGTPGQRVTISCSGSSSNIGNNYVTWYQQLPGTAPKLLI YADSHRPSGVPDRFSGSKSGTSASLAISGLRSEDEADYYCATWDYSLS GYVFGGGTKLTVL |
| 44 | DIVMTQSPAFLSVTPGEKVTITCRASQTISDYLHWYQQKPDQAPKLLI KYASQSISGIPSRFSGSGSGTDFTFTISSLEAEDAATYYCQDGHSFPPTF GQGTKLEIKR |
| 45 | QSVLTQPPSASGTPGRRVTISCSGSSSNIGNNYVTWYQQLPGTAPKLLI YADSHRPSGVPDRFSGSKSGTSASLAISGLRSEDEADYYCATWDYSLS GYVFGCGTKLTVL |
| 46 | QSVLTQPPSASGTPGQRVTISCSGSSSNIGNNYVTWYQQLPGTAPKLLI YADSHRPSGVPDRFSGSKSGTSASLAISGLRSEDEADYYCATWDYSLS GYVFGCGTKLTVL |
| 47 | DIVMTQSPAFLSVTPGEKVTITCRASQTISDYLHWYQQKPDQAPKLLI KYASQSISGIPSRFSGSGSGTDFTFTISSLEAEDAATYYCQDGHSFPPTF GCGTKLEIKR |

In another embodiment, the anti-4-1BB antibody or antigen-binding fragment thereof may be a single-chain variable fragment (scFv) that comprises:
a heavy chain variable region comprising HCDR1 with an amino acid sequence of SEQ ID NO: 13, 14, or 15, HCDR2 with amino acid sequences from SEQ ID NO: 16, 17, or 18, and HCDR3 with amino acid sequences from SEQ ID NO: 19, 20, 21, 22, or 23; and
a light chain variable region comprising LCDR1 with an amino acid sequence of SEQ ID NO: 24 or 25, LCDR2 with an amino acid sequence of SEQ ID NO: 26 or 27, and LCDR3 with an amino acid sequence of SEQ ID NO: 28 or 29.

In an embodiment, the anti-4-1BB scFv may comprise:
a heavy chain variable region comprising an amino acid sequence of SEQ ID NO: 30, 31, 32, 33, 34, 35, 36, 37, 38, 39, 40, or 41 (more specifically, SEQ ID NO: 36, 37, 38, 39, 40, or 41); and
a light chain variable region comprising an amino acid sequence of SEQ ID NO: 42, 43, 44, 45, 46, or 47 (more specifically, SEQ ID NO: 45, 46, or 47).

In this regard, the heavy chain and light chain variable regions may be directly connected or linked through a peptide linker in any order.

In the anti-4-1BB scFv, the heavy and light chain variable regions may be connected directly (e.g., without a linker) or through a peptide linker in any order. More specifically, the anti-4-1BB scFv may comprise, in order from the N-terminus to the C-terminus, a light chain variable region and a heavy chain variable region; a heavy chain variable region and a light chain variable region; a light chain variable region, a peptide linker, and a heavy chain variable region; or a heavy chain variable region, a peptide linker, and a light chain variable region.

### Anti-ROR1/anti-B7-H3 bispecific antibody

An aspect provides an anti-ROR1/anti-B7-H3 bispecific antibody comprising:
(1) an anti-ROR1 antibody or an antigen-binding fragment thereof as an ROR1 targeting moiety, and
(2) an anti-B7-H3 antibody or an antigen-binding fragment thereof as a B7-H3 targeting moiety. The anti-ROR1/anti-B7-H3 bispecific targets (specifically binds to and/or recognizes) both ROR1 and B7-H3.

The anti-ROR1 antibody or antigen-binding fragment thereof as an ROR1 targeting moiety may be a protein that targets (specifically binds to and/or recognizes) ROR1. More specifically, the ROR1 targeting moiety may be the anti-ROR1 antibody or antigen-binding fragment described above. The description provided above regarding the anti-ROR1 antibody or antigen-binding fragment thereof may also be applied to the anti-ROR1 antibody or antigen-binding fragment thereof comprised in the anti-ROR1/anti-B7-H3 bispecific antibody.

The anti- B7-H3 antibody or antigen-binding fragment thereof as a B7-H3 targeting moiety may be a protein that targets (specifically binds to and/or recognizes) B7-H3.

B7-H3 (B7 Homolog 3, CD276), which is a transmembrane protein belonging to the immunoglobulin (Ig) superfamily, may include an extracellular domain, a transmembrane domain, and/or an intracellular domain. The B7-H3 targeted by the anti-B7-H3 antibody or antigen-binding fragment thereof may be the extracellular domain present on or not located on the cell membrane. B7-H3 may be mammalian B7-H3, such as human B7-H3(e.g., NCBI Accession No. NP_001019907.1 (534 aa)), monkey B7-H3(e.g., NCBI Accession No. XP_005560056.1), or mouse B7-H3(e.g., NCBI Accession No. NP_598744.1), but is not limited thereto.

For instance, the anti-B7-H3 antibody or antigen-binding fragment thereof may comprise:
HCDR1 comprising an amino acid sequence selected from SEQ ID NOS: 48 to 51;
HCDR2 comprising an amino acid sequence selected from SEQ ID NOS: 52 to 57;
HCDR3 comprising an amino acid sequence selected from SEQ ID NOS: 58 to 62;
LCDR1 comprising an amino acid sequence selected from SEQ ID NOS: 63 to 67;
LCDR2 comprising an amino acid sequence selected from SEQ ID NOS: 68 to 73; and
LCDR3 comprising an amino acid sequence selected from SEQ ID NOS: 74 to 79.

The amino acid sequences of the CDRs of the anti-B7-H3 antibody or antigen-binding fragment thereof are exemplified in Table 4.

**TABLE 4**

| **HCDR1** | |
|---|---|
| **Sequence** | **SEQ ID NO: #** |
| DYAMS | 48 |
| GYYMS | 49 |
| SYSMS | 50 |
| SYGMS | 51 |

| **HCDR2** | |
|---|---|
| **Sequence** | **SEQ ID NO: #** |
| SISSGSGSIYYADSVKG | 52 |
| LISPSSGSIYYADSVKG | 53 |
| GIYSDGSNTYYADSVKG | 54 |
| GISPGGSNTYYADSVKG | 55 |
| GIYSGGSSKYYADSVKG | 56 |
| GIYSDASNTYYADSVKG | 57 |

| **HCDR3** | |
|---|---|
| **Sequence** | **SEQ ID NO: #** |
| NLIPLDY | 58 |
| GLTKFDY | 59 |
| MLHRFDY | 60 |
| DAWIARLLLFDY | 61 |
| NRLRFDY | 62 |

| **LCDR1** | |
|---|---|
| **Sequence** | **SEQ ID NO: #** |
| SGSSSNIGSNAVS | 63 |
| TGSSSNIGSNDVS | 64 |
| SGSSSIVIGSNSVT | 65 |
| SGSSSNIGSNAVT | 66 |
| TGSSSNIGSNSVT | 67 |

| **LCDR2** | |
|---|---|
| **Sequence** | **SEQ ID NO: #** |
| YNSHRPS | 68 |
| ANSHRPS | 69 |
| ADSQRPS | 70 |
| YNNKRPS | 71 |
| SDSHRPS | 72 |
| ADVQRPS | 73 |

| **LCDR3** | |
|---|---|
| **Sequence** | **SEQ ID NO: #** |
| GSWDASLNA YV | 74 |
| GSWDDSLSGYV | 75 |
| GTWDSSLNAYV | 76 |
| GTWDDSLSGYV | 77 |
| GTWDASLNAYV | 78 |
| GSWDASLNA YV | 79 |

The heavy chain variable region and light chain variable region of the anti-B7-H3 antibody or antigen-binding fragment thereof may be derived from an IgG-derived antibody or an antigen-binding fragment thereof. They may comprise wild-type sequences, such as framework (HFR1, HFR2, HFR3, HFR4, LFR1, LFR2, LFR3, and/or LFR4) regions with a mutation introduced thereinto. In an embodiment, the mutation may be intended for stabilization and/or disulfide bridge formation. For instance, the antigen-binding fragment may have one or more (e.g., one) G (Gly) → C (Cys) mutations (where G is substituted by C) introduced in the framework region of the variable domain in the antibody, but with no limitations thereto. In a particular embodiment, if the B7-H3 targeting moiety is an intact IgG antibody or a part thereof (e.g., Fab or Fab-Fc), the heavy chain variable region and light chain variable region comprised therein may be derived from wild-type IgG. In another embodiment, if the B7-H3 targeting moiety is an antigen-binding fragment of anti-ROR1 antibody (e.g., scFv, etc.), the heavy chain variable region and light chain variable region comprised therein may have G→C mutations introduced into the framework region thereof.

In an embodiment, the anti-B7-H3 antibody or antigen-binding fragment thereof comprises:
a heavy chain variable region comprising an amino acid sequence selected from SEQ ID NOS: 80 to 91; and
a light chain variable region comprising an amino acid sequence selected from SEQ ID NOS: 92 to 103.

The sequences (N→C) of the variable regions of the anti-B7-H3 antibody or antigen-binding fragment thereof are exemplified in Table 5.

**TABLE 5**

| **Heavy chain variable region (VH) sequence** | **SEQ ID NO: #** |
|---|---|
| EVQLLESGGGLVQPGGSLRLSCAASGFTFSDYAMSWVRQAPGKGLEW VSSISSGSGSIYYADSVKGRFTISRDNSKNTLYLQMNSLRAEDTAVYYC AKNLIPLDYWGQGTLVTVSS | 80 |
| EVQLLESGGGLVQPGGSLRLSCAASGFTFSGYYMSWVRQAPGKGLEW VSLISPSSGSIYYADSVKGRFTISRDNSKNTLYLQMNSLRAEDTAVYYC AKGLTKFDYWGQGTLVTVSS | 81 |
| EVQLLESGGGLVQPGGSLRLSCAASGFTFSSYSMSWVRQAPGKGLEW VSGIYSDGSNTYYADSVKGRFTISRDNSKNTLYLQMNSLRAEDTAVYY CAKMLHRFDYWGQGTLVTVSS | 82 |
| EVQLLESGGGLVQPGGSLRLSCAASGFTFSDYAMSWVRQAPGKGLEW VSGISPGGSNTYYADSVKGRFTISRDNSKNTLYLQMNSLRAEDTAVYY CAKDAWIARLLLFDYWGQGTLVTVSS | 83 |
| EVQLLESGGGLVQPGGSLRLSCAASGFTFSSYGMSWVRQAPGKGLEW VSGIYSGGSSKYYADSVKGRFTISRDNSKNTLYLQMNSLRAEDTAVYY CAKNRLRFDYWGQGTLVTVSS | 84 |
| EVQLLESGGGLVQPGGSLRLSCAASGFTFSSYSMSWVRQAPGKGLEW VSGIYSDASNTYYADSVKGRFTISRDNSKNTLYLQMNSLRAEDTAVYY | 85 |
| CAKMLHRFDYWGQGTLVTVSS | |
| EVQLLESGGGLVQPGGSLRLSCAASGFTFSDYAMSWVRQAPGKCLEW VSSISSGSGSIYYADSVKGRFTISRDNSKNTLYLQMNSLRAEDTAVYYC AKNLIPLDYWGQGTLVTVSS | 86 |
| EVQLLESGGGLVQPGGSLRLSCAASGFTFSGYYMSWVRQAPGKCLEW VSLISPSSGSIYYADSVKGRFTISRDNSKNTLYLQMNSLRAEDTAVYYC AKGLTKFDYWGQGTLVTVSS | 87 |
| EVQLLESGGGLVQPGGSLRLSCAASGFTFSSYSMSWVRQAPGKCLEW VSGIYSDGSNTYYADSVKGRFTISRDNSKNTLYLQMNSLRAEDTAVYY CAKMLHRFDYWGQGTLVTVSS | 88 |
| EVQLLESGGGLVQPGGSLRLSCAASGFTFSDYAMSWVRQAPGKCLEW VSGISPGGSNTYYADSVKGRFTISRDNSKNTLYLQMNSLRAEDTAVYY CAKDAWIARLLLFDYWGQGTLVTVSS | 89 |
| EVQLLESGGGLVQPGGSLRLSCAASGFTFSSYGMSWVRQAPGKCLEW VSGIYSGGSSKYYADSVKGRFTISRDNSKNTLYLQMNSLRAEDTAVYY CAKNRLRFDYWGQGTLVTVSS | 90 |
| EVQLLESGGGLVQPGGSLRLSCAASGFTFSSYSMSWVRQAPGKCLEW VSGIYSDASNTYYADSVKGRFTISRDNSKNTLYLQMNSLRAEDTAVYY CAKMLHRFDYWGQGTLVTVSS | 91 |

| **Light chain variable region (VL) sequence** | **SEQ ID NO: #** |
|---|---|
| QSVLTQPPSASGTPGQRVTISCSGSSSNIGSNAVSWYQQLPGTAPKLLIY YNSHRPSGVPDRFSGSKSGTSASLAISGLRSEDEADYYCGSWDASLNA YVFGGGTKLTVL | 92 |
| QSVLTQPPSASGTPGQRVTISCTGSSSNIGSNDVSWYQQLPGTAPKLLIY ANSHRPSGVPDRFSGSKSGTSASLAISGLRSEDEADYYCGSWDDSLSGY VFGGGTKLTVL | 93 |
| QSVLTQPPSASGTPGQRVTISCSGSSSNIGSNSVTWYQQLPGTAPKLLIY ADSQRPSGVPDRFSGSKSGTSASLAISGLRSEDEADYYCGTWDSSLNAY VFGGGTKLTVL | 94 |
| QSVLTQPPSASGTPGQRVTISCSGSSSNIGSNAVTWYQQLPGTAPKLLIY YNNKRPSGVPDRFSGSKSGTSASLAISGLRSEDEADYYCGTWDDSLSG YVFGGGTKLTVL | 95 |
| QSVLTQPPSASGTPGQRVTISCTGSSSNIGSNSVTWYQQLPGTAPKLLIY SDSHRPSGVPDRFSGSKSGTSASLAISGLRSEDEADYYCGTWDASLNAY VFGGGTKLTVL | 96 |
| QSVLTQPPSASGTPGQRVTISCSGSSSNIGSNSVTWYQQLPGTAPKLLIY | 97 |
| ADVQRPSGVPDRFSGSKSGTSASLAISGLRSEDEADYYCGTWDSSLNA YVFGGGTKLTVL | |
| QSVLTQPPSASGTPGQRVTISCSGSSSNIGSNAVSWYQQLPGTAPKLLIY YNSHRPSGVPDRFSGSKSGTSASLAISGLRSEDEADYYCGSWDASLNA YVFGCGTKLTVL | 98 |
| QSVLTQPPSASGTPGQRVTISCTGSSSNIGSNDVSWYQQLPGTAPKLLIY ANSHRPSGVPDRFSGSKSGTSASLAISGLRSEDEADYYCGSWDDSLSGY VFGCGTKLTVL | 99 |
| QSVLTQPPSASGTPGQRVTISCSGSSSNIGSNSVTWYQQLPGTAPKLLIY ADSQRPSGVPDRFSGSKSGTSASLAISGLRSEDEADYYCGTWDSSLNAY VFGCGTKLTVL | 100 |
| QSVLTQPPSASGTPGQRVTISCSGSSSNIGSNAVTWYQQLPGTAPKLLIY YNNKRPSGVPDRFSGSKSGTSASLAISGLRSEDEADYYCGTWDDSLSG YVFGCGTKLTVL | 101 |
| QSVLTQPPSASGTPGQRVTISCTGSSSNIGSNSVTWYQQLPGTAPKLLIY SDSHRPSGVPDRFSGSKSGTSASLAISGLRSEDEADYYCGTWDASLNAY VFGCGTKLTVL | 102 |
| QSVLTQPPSASGTPGQRVTISCSGSSSNIGSNSVTWYQQLPGTAPKLLIY ADVQRPSGVPDRFSGSKSGTSASLAISGLRSEDEADYYCGTWDSSLNA YVFGCGTKLTVL | 103 |

For example, the anti-B7-H3 antibody or antigen-binding fragment thereof may comprise:
a heavy chain variable region comprising an amino acid sequence of SEQ ID NO: 80 or 86 and a light chain variable region comprising an amino acid sequence of SEQ ID NO: 92 or 98;
a heavy chain variable region comprising an amino acid sequence of SEQ ID NO: 81 or 87 and a light chain variable region comprising an amino acid sequence of SEQ ID NO: 93 or 99;
a heavy chain variable region comprising an amino acid sequence of SEQ ID NO: 82 or 88 and a light chain variable region comprising an amino acid sequence of SEQ ID NO: 94 or 100;
a heavy chain variable region comprising an amino acid sequence of SEQ ID NO: 83 or 89 and a light chain variable region comprising an amino acid sequence of SEQ ID NO: 95 or 101;
a heavy chain variable region comprising an amino acid sequence of SEQ ID NO: 84 or 90 and a light chain variable region comprising an amino acid sequence of SEQ ID NO: 96 or 102; or
a heavy chain variable region comprising an amino acid sequence of SEQ ID NO: 85 or 91 and a light chain variable region comprising an amino acid sequence of SEQ ID NO: 97 or 103;

In another embodiment, the anti-B7-H3 antibody or antigen-binding fragment thereof may be a single-chain variable fragment (scFv) comprising:
a heavy chain variable region comprising HCDR1 with an amino acid sequence selected from SEQ ID NOS: 48 to 51, HCDR2 with an amino acid sequence selected from SEQ ID NOS: 52 to 57, and HCDR3 with an amino acid sequence selected SEQ ID NOS: 58 to 62; and
a light chain variable region comprising LCDR1 with an amino acid sequence selected from SEQ ID NOS: 63 to 67, LCDR2 with an amino acid sequence selected from SEQ ID NOS: 68 to 73, and LCDR3 with an amino acid sequence selected from SEQ ID NOS: 74 to 79.

In an embodiment, the anti-B7-H3 scFv may comprise:
a heavy chain variable region comprising an amino acid sequence selected from SEQ ID NOS: 80 to 91 (more specifically, SEQ ID NOS: 86 to 91); and
a light chain variable region comprising an amino acid sequence selected from SEQ ID NOS: 92 to 103 (more specifically, SEQ ID NOS: 98 to 103).

In this regard, the heavy chain and light chain variable regions may be directly connected or linked through a peptide linker in any order.

In an embodiment, the anti-B7-H3 scFv may comprise:
a heavy chain variable region comprising an amino acid sequence of SEQ ID NO: 80 or 86 and a light chain variable region comprising an amino acid sequence of SEQ ID NO: 92 or 98;
a heavy chain variable region comprising an amino acid sequence of SEQ ID NO: 81 or 87 and a light chain variable region comprising an amino acid sequence of SEQ ID NO: 93 or 99;
a heavy chain variable region comprising an amino acid sequence of SEQ ID NO: 82 or 88 and a light chain variable region comprising an amino acid sequence of SEQ ID NO: 94 or 100;

a heavy chain variable region comprising an amino acid sequence of SEQ ID NO: 83 or 89 and a light chain variable region comprising an amino acid sequence of SEQ ID NO: 95 or 101;
a heavy chain variable region comprising an amino acid sequence of SEQ ID NO: 84 or 90 and a light chain variable region comprising an amino acid sequence of SEQ ID NO: 96 or 102; or
a heavy chain variable region comprising an amino acid sequence of SEQ ID NO: 85 or 91 and a light chain variable region with an amino acid sequence of SEQ ID NO: 97 or 103.

In the anti-B7-H3 scFv, the heavy and light chain variable regions may be connected directly (e.g., without a linker) or through a peptide linker in any order. More specifically, the anti-B7-H3 scFv may comprise, in order from the N-terminus to the C-terminus, a light chain variable region and a heavy chain variable region; a heavy chain variable region and a light chain variable region; a light chain variable region, a peptide linker, and a heavy chain variable region; or a heavy chain variable region, a peptide linker, and a light chain variable region.

### Bispecific antibody comprising anti-ROR1 antibody

In the anti-ROR1/anti-4-1BB bispecific antibody and/or anti-ROR1/anti-B7-H3 bispecific antibody, provided herein, the anti-ROR1 antibody or antigen-binding fragment thereof, serving as an ROR1 targeting moiety, is capable of targeting (specifically binding to and/or recognizing) ROR1.

In an embodiment, if the ROR1 targeting moiety is an intact IgG antibody or a part thereof (e.g., Fab or Fab-Fc), the heavy and light chain variable regions comprised therein may be derived from wild-type IgG. In another embodiment, if the ROR1 targeting moiety is an antigen-binding fragment of the anti-ROR1 antibody (e.g., scFv), the heavy and light chain variable regions comprised therein may have a mutation introduced in the framework region.

In an embodiment, the anti-ROR1 antibody or antigen-binding fragment thereof comprised in the bispecific antibody provided herein may be an antibody in an intact immunoglobulin form (e.g., in the form of IgG comprising 2 heavy chains and 2 light chains), a part thereof (e.g., Fab or Fab-Fc), or an antigen-binding fragment derived therefrom (such as an scFv, etc.). The anti-ROR1 scFv may additionally incorporate a peptide linker connecting the heavy and light chain variable regions.

The anti-ROR1/anti-4-1BB bispecific antibody or anti-ROR1/anti-B7-H3 bispecific antibody provided herein may comprise at least one copy of the ROR1 targeting moiety and at least one copy of the B7-H3 or 4-1BB targeting moiety, with no particular limitations imparted to the linkage form and structure therebetween.

In an embodiment, both the ROR1 targeting moiety and the 4-1BB or B7-H3 targeting moiety within the anti-ROR1/anti-4-1BB bispecific antibody or anti-ROR1/anti-B7-H3 bispecific antibody may be a bivalent antigen-binding antibody or bivalent antigen-binding fragment (for example, intact antibody (e.g., IgG), Fab-Fc or Fab'-Fc dimers linked at the Fc, or scFv-Fc-scFv). For example, a bispecific antibody may comprise an anti-ROR1 IgG antibody and anti-4-1BB or anti-B7-H3 scFv grafted thereto, where the scFv is directly connected to either the N-terminus or C-terminus of one or both of the heavy or light chains of the IgG antibody, either through a peptide linker or directly without a linker.

In another embodiment, one of the ROR1 targeting moiety and the 4-1BB or B7-H3 targeting moiety in the anti-ROR1/anti-4-1BB or anti-ROR1/anti-B7-H3 bispecific antibody may be a bivalent antigen or antigen-binding fragment (such as an intact IgG, a dimer of Fab-Fc or Fab'-Fc linked at the Fc, or scFv-Fc-scFv) and the other may be a monovalent antibody or antigen-binding fragment (e.g., scFv, scFv-Fc, Fab, Fab-Fc, Fab'-Fc, etc.). The monovalent antibody or antigen-binding fragment may be linked to either or both of the N-terminus and C-terminus of one or both of the heavy or light chains of the bivalent antibody or antigen-binding fragment or linked between the Fc regions, either through a peptide linker or directly without any linker. For example, the bispecific antibody may comprise an anti-ROR1 IgG antibody and an anti-4-1BB or anti-B7-H3 scFv grafted thereto, where the scFv is connected to the N-terminus or C-terminus of one of the two heavy chain or two light chains of the IgG antibody, through a peptide linker or directly without a linker.

In another embodiment, the bispecific antibody may comprise an anti-ROR1 Fab-Fc and an anti-B7-H3 Fab-Fc, Fab-Fc-scFv, scFv-Fc-scFv, or scFv-Fc linked thereto. The scFvs may be linked either through a peptide linker or directly without any linker.

For instance, in one embodiment, the ROR1 targeting moiety within an anti-ROR1/anti-4-1BB bispecific antibody may be an anti-ROR1 IgG antibody, and the 4-1BB targeting moiety may be an anti-4-1BB scFv.

For B7-H3 antibodies, there exists a potential for toxicity when the binding affinity to the antigen is excessively high. Therefore, for the anti-ROR1/anti-B7-H3 bispecific antibodies provided herein, it may be preferable to use clones that possess an appropriate level of binding affinity while also having a reduced risk of toxicity for targeting B7-H3.

In another embodiment, the ROR1 targeting moiety and the 4-1BB or B7-H3 targeting moiety within the anti-ROR1/anti-4-1BB or anti-ROR1/anti-B7-H3 bispecific antibody may each be a monovalent antibody or antigen-binding fragment having one antigen binding site (e.g., scFv, scFv-Fc, Fab, Fab-Fc, Fab'-Fc, etc.). These antibodies or antigen-binding fragments may be linked either through a peptide linker or directly without any linker and may be connected to the terminus of F c, or heavy or light chain constant region, the inside of the heavy or light chain constant region, or the terminus of the heavy or light chains variable region, or linked between Fc regions.

In one embodiment, scFv may comprise variable regions of the heavy and light chains, and optionally a linker, in any order. For example, the scFv may comprise, in sequence from the N-terminus to the C-terminus, the variable region of the light chain and the variable region of the heavy chain; the variable region of the heavy chain and the variable region of the light chain; the variable region of the light chain, a peptide linker, and the variable region of the heavy chain; or the variable region of the heavy chain, a peptide linker, and the variable region of the light chain.

In the anti-ROR1/anti-4-1BB bispecific antibody or anti-ROR1/anti-B7-H3 bispecific antibody provided herein, the light chain variable region, the heavy chain variable region, scFv and Fc, and/or the ROR1 targeting moiety and the 4-1BB or B7-H3 targeting moiety may be linked through a peptide linker. In an embodiment, the peptide linker may be an oligopeptide comprising 1 to 100 amino acids, particularly 2 to 50 amino acids, each of which may be any type of amino acid without limitation. Any conventional peptide linker can be used, with or without appropriate modifications, depending on the specific purpose. In certain embodiments, the peptide linker may include residues such as Gly, Asn, and/or Ser, and may also comprise neutral amino acids like Thr and/or Ala. Suitable amino acid sequences for the peptide linker are well known in the related art. The length of the peptide linker can be determined appropriately within the limit that does not affect the function of the polypeptides and/or scFv. For example, the peptide linker may be composed of a total of about 1 to about 100 amino acids, about 2 to about 50 amino acids, or about 5 to about 25 amino acids (for instance, 1, 2, 3, 4, 5, 6, 7, 8, 9, 10, 11, 12, 13, 14, 15, 16, 17, 18, 19, 20, 21, 22, 23, 24, or 25 amino acids), each independently selected from a group consisting of Gly, Asn, Ser, Thr, and Ala. In one embodiment, the peptide linker can be represented as (GₘSₗ)n (where m, l, and n represent the number of "G", "S", and "(GₘSₗ)" units, respectively, each independently selected from an integer of about 1 to about 10, particularly, 1, 2, 3, 4, 5, 6, 7, 8, 9, or 10). In an embodiment, the peptide linker may be amino acids such as (GGGGS)₂, (GGGGS)₃, (GGGGS)₄, or (GS)₉, but is not limited thereto.

### Use of Anti-ROR1 antibody and/or bispecific antibody comprising the same

The anti-ROR1 antibody provided herein exhibits inhibitory activity against ROR1 (e.g., suppression of ROR1 expression and/or activity inhibition). Additionally, the anti-ROR1/anti-4-1BB bispecific antibodies comprising the same have both ROR1 inhibitory activity and T cell activation effects, while the anti-ROR1/anti-B7-H3 bispecific antibodies have functions of targeting cancer cells (cancer cells expressing ROR1 and/or B7-H3) through specifically binding to the cancer cells, anticancer activity (e.g., inhibiting cancer cell proliferation, and inducing cancer cell death), and the like. Therefore, these anti-ROR1 antibodies, anti-ROR1/anti-4-1BB bispecific antibodies, or anti-ROR1/anti-B7-H3 bispecific antibodies can be effectively used for the prevention and/or treatment of cancer. Moreover, with excellent cell internalization capability (see Example 3.4), the anti-ROR1/anti-B7-H3 bispecific antibodies provided herein can achieve superior cancer cell killing (anticancer) effects when used in conjunction with cytotoxic drugs, such as in antibody-drug conjugates (ADCs).

As used herein, the term "conjugate" refers to a cell-binding agent that is covalently bonded to one or more molecules of a cytotoxic compound. Here, the "cell-binding agent" is a molecule with affinity for a biological target, such as ligands, proteins, antibodies, or their antigen-binding fragments, and functions to guide biologically active compounds to the biological target. In an embodiment of the present disclosure, the conjugate may be designed to target cancer cells through cell surface antigens. The antigen may be a cell surface antigen that is overexpressed or expressed on abnormal cell types. Specifically, the target antigen can be predominantly expressed on proliferative cells (e.g., cancer cells). Target antigens may be selected based on their differential expression between proliferative tissues and normal tissues.

The anti-ROR1 antibody, anti-ROR1/anti-4-1BB bispecific antibody, anti-ROR1/anti-B7-H3 bispecific antibody, or antigen-binding fragments thereof provided herein may be used in the form of conjugates in which one or more selected from a group consisting of useful polymers, labels, drugs, etc. are conjugated thereto.

The useful polymer may be a polymer, for example, that can increase the half-life of polypeptides, antibodies, and/or antigen-binding fragments thereof in vivo, and may be selected from a group consisting of polyethylene glycol (PEG) (with molecular weights of, for example, 2 kDa, 5 kDa, 10 kDa, 12 kDa, 20 kDa, 30 kDa, or 40 kDa), dextran, and monomethoxypolyethylene glycol (mPEG), but with no limitations thereto.

The label may be selected from fluorescent or chemiluminescent small molecule compounds, radioactive isotopes, etc., typically used in the industry, but are not limited thereto.

In the present disclosure, the drug may be at least one selected from the group consisting of cytotoxic drugs (e.g., anticancer agents, antibiotics, etc.) and contrast agents. These cytotoxic drugs may have cytotoxicity against cell, especially cancer cells, and toxicity against bacteria/viruses and may include, for example, tubulin inhibitors, DNA alkylating agents, topoisomerase inhibitors, RNA polymerase inhibitors, ribosome inactivating proteins, NAMPT inhibitors, and immunomodulators. Examples of the drugs include maytansine-based drugs (e.g., mertansine (DM1), ravtansine (DM4)), auristatin-based drugs (e.g., monomethyl auristatin E (MMAE), monomethyl auristatin F (MMAF), etc.), calicheamicin-based drugs, pyrrolobenzodiazepine-based drugs, duocarmycin, docetaxel, doxorubicin, carboplatin (paraplatin), cyclophosphamide, ifosfamide, nidran, nitrogen mustard, mechlorethamine HCL, bleomycin, mitomycin C, cytarabine, flurouracil, gemcitabine, trimetrexate, methotrexate, etoposide, vinblastine, vinorelbine, alimta, altretamine, procarbazine, paclitaxel (taxol), taxotere, topotecan, irinotecan, exatecan, and analogs thereof. In this case, it may be used in the form of an antibody-drug conjugate (ADC), wherein the drug may be preferably a maitansine-based drug, an auristatin-based drug, a calicheamicin-based drug, a pyrrolobenzodiazepine-based drug, duocarmycin, irinotecan, or exatecan. In the conjugates, the antibody and the cytotoxic drug (e.g., anticancer agent) may be linked to each other via chemical bond, for instance, a covalent bond. For example, the antibody and the cytotoxic drug can be linked through thiol coupling (SH coupling) or amine coupling (NH₂ coupling). To this end, any amino acid residue in the heavy chain and/or light chain (e.g., within the constant region) of the antibody may be modified to or substituted with cysteine so as to enable thiol or amine coupling, and/or the drug can be derivatized to possess a functional group that allows for thiol or amine coupling. Alternatively, the antibody and the cytotoxic drug can be linked to each other through glycosylation of the asparagine (N) residue in the Fc portion.

As used herein, the term "drug-to-antibody ratio" (DAR) refers to the average number of therapeutic moieties, such as drugs, bound per a conjugate according to the present disclosure. The drug-to-antibody ratio can be measured by methods such as liquid chromatography-mass analysis. The drug-to-antibody ratio of the conjugates according to the present disclosure may be from 1 to 10, in particular, from 2 to 8, but is not limited thereto, and can be appropriately adjusted depending on the type and characteristics of the drug or linker used.

As used herein, the "antibody-drug conjugate" (ADC) can deliver the drug to cells to which the antibody can specifically bind; for example, as the drug is conjugated with the antibody of the present disclosure that can specifically bind to ROR1 and/or B7-H3, the conjugate can selectively deliver the drug to cells expressing ROR1 and/or B7-H3.

In the present disclosure, the conjugation between the antibody and the drug may be achieved by a direct chemical bond or an indirect bond through a linker or a secondary antibody bound to the antibody, but is not limited thereto.

The antibody-drug conjugate may comprise a linker, which refers to a compound that covalently binds an active agent to a ligand. For example, the linker can be cleavable, non-cleavable, hydrophilic, or hydrophobic, but with no limitations thereto. For instance, the linker sequence can be attached to the Fc sequence of the antibody according to the present disclosure, and the linker and drug can be connected to prepare the form of an antibody-linker-drug.

Another aspect provides a pharmaceutical composition comprising the anti-ROR1 antibody, the anti-ROR1/anti-4-1BB bispecific antibody, the anti-ROR1/anti-B7-H3 bispecific antibody, or the antigen-binding fragment thereof; and/or the antibody-drug conjugate. The pharmaceutical composition may additionally comprise a pharmaceutically acceptable excipient. The pharmaceutical composition may have anticancer activity.

Another aspect provides a pharmaceutical composition for the prevention and/or treatment of cancer, the composition comprising the anti-ROR1 antibody, the anti-ROR1/anti-4-1BB bispecific antibody, the anti-ROR1/anti-B7-H3 bispecific antibody, or the antigen-binding fragment thereof; and/or the conjugate.

Another aspect provides a method for the prevention and/or treatment of cancer, the method comprising a step of administering an effective amount of the anti-ROR1 antibody, the anti-ROR1/anti-4-1BB bispecific antibody, the anti-ROR1/anti-B7-H3 bispecific antibody, or the antigen-binding fragment thereof; and/or the antibody-drug conjugate to a subject in need of the prevention and/or treatment of cancer. The method may further comprise a step of identifying (diagnosing) the subject in need of the prevention and/or treatment of cancer before the administering step.

Another aspect provides a use of the anti-ROR1 antibody, the anti-ROR1/anti-4-1BB bispecific antibody, the anti-ROR1/anti-B7-H3 bispecific antibody, or the antigen-binding fragment thereof; and/or the antibody-drug conjugate for the prevention and/or treatment of cancer, and/or for the preparation of a medicament (anticancer drug) for the prevention and/or treatment of cancer.

The cancer may be a solid cancer or blood cancer and, for example, may be characterized by the expression (or overexpression) of ROR1 and/or B7-H3 on cell surface, but with no limitations thereto. Examples of the cancer includes, but are not limited thereto, breast cancer, breast cancer, lung cancer, prostate cancer, ovarian cancer, brain cancer, liver cancer, colorectal cancer, cervical cancer, endometrial cancer, kidney cancer, skin cancer, nasopharyngeal cancer, head and neck cancer, bone cancer, esophageal cancer, bladder cancer, stomach cancer, pancreatic cancer, testicular cancer, thyroid cancer, oral cancer, renal cell carcinoma, adrenal cancer, melanoma, multiple myeloma, mesothelioma, osteosarcoma, myelodysplastic syndromes, soft tissue sarcomas, gastrointestinal stromal tumors, malignant peripheral nerve sheath tumors (MPNST), Ewing's sarcoma, leiomyosarcoma, chondrosarcoma, lymphoma, fibrosarcoma, rhabdomyosarcoma, teratoma, neuroblastoma, glioma, leukemia, Burkitt lymphoma, mantle cell lymphoma, diffuse large B-cell lymphoma, follicular lymphoma, marginal zone lymphoma, and others. Lung cancer, for example, can be small cell lung carcinoma (SCLC) or non-small cell lung carcinoma (NSCLC). Leukemias may include acute myeloid leukemia (AML), chronic myelogenous leukemia (CML), acute lymphoblastic leukemia (ALL), or chronic lymphocytic leukemia (CLL). These cancers can be primary or metastatic.

The anticancer activity or the prophylactic and/or therapeutic effects on cancer means any anticancer and/or anti-tumor action to eliminate (kill) cancer cells, inhibit the occurrence and/or growth of cancer cells, prevent or reduce the worsening of cancer due to migration, invasion, metastasis, alleviate or palliate symptoms, and/or partially or completely eradicate the cancer.

The subject to which the anti-ROR1 antibody, anti-ROR1/anti-4-1BB bispecific antibody, anti-ROR1/anti-B7-H3 bispecific antibody, antigen-binding fragment thereof, antibody-drug conjugate, and/or pharmaceutical composition comprising the same, provided herein, is administered may be any animal or cell. Specifically, the subject may include animals selected from mammals including primates such as humans and monkeys, rodents such as rats and mice, and the like, or cells, tissues, bodily fluids (e.g., serum), or cultures derived from (isolated from) these animals, particularly, humans, or cells, tissues, bodily fluids (e.g., serum) isolated from humans.

The pharmaceutical composition may comprise a pharmaceutically acceptable excipient and/or carrier in addition to the active ingredient(s) such as the anti-ROR1 antibody, anti-ROR1/anti-4-1BB bispecific antibody, anti-ROR1/anti-B7-H3 bispecific antibody, antigen-binding fragment thereof, or conjugate, and the excipient and/or carrier can be selected by those skilled in the art from those conventionally used in the formulation of protein drugs, and one or more types can be appropriately chosen for use.

The administration of the anti-ROR1 antibody, anti-ROR1/anti-4-1BB bispecific antibody, anti-ROR1/anti-B7-H3 bispecific antibody, antigen-binding fragment thereof, conjugate, and/or pharmaceutical composition may be performed through oral or non-oral routes.

The content of the effective ingredients the anti-ROR1 antibody, anti-ROR1/anti-4-1BB bispecific antibody, anti-ROR1/anti-B7-H3 bispecific antibody, antigen-binding fragment thereof, and conjugate, in the pharmaceutical composition, may be variably prescribed depending on factors including the method of formulation, mode of administration, the patient's age, weight, gender, pathological condition, food intake, timing of administration, interval between administrations, route of administration, rate of excretion, and response sensitivity.

For more detailed information on excipients and/or carriers, routes of administration, dosage, etc., in the pharmaceutical composition, reference may be made to Korean Patent No. 10-2551365.

Another aspect provides a nucleic acid molecule encoding the anti-ROR1 antibody, anti-ROR 1/anti-4-1BB bispecific antibody, or anti-ROR1/anti-B7-H3 bispecific antibody.

Another aspect provides a recombinant vector carrying the nucleic acid molecule. The recombinant vector may be an expression vector for expressing the nucleic acid molecule.

Another aspect provides a recombinant cell comprising the recombinant vector.

In cases where the antibodies or antigen-binding fragments thereof are recombinantly produced, they may be linked with conventional signal peptides, cleavage sites, tags, etc., for purification purposes. Therefore, in a non-limiting example, the antibodies or antigen-binding fragments thereof provided herein may comprise one or more elements selected from a group consisting of signal peptides, cleavage sites, tags (for example, His tag, GST (glutathione S-transferase) tag, MBP (maltose binding protein) tag, etc.) commonly used in the recombinant production process of proteins, or they may be in a purified form with these elements removed.

The term "vector" refers to a means to express a target gene in a host cell. Examples include plasmid vectors, cosmid vectors, bacteriophage vectors, lentivirus vectors, adenovirus vectors, retrovirus vectors, and adeno-associated virus vectors. Vectors that can be used as recombinant vectors may be constructed by manipulating commonly used plasmids in the art (e.g., pBR series, pUC series, pBluescript II series, pGEM series, pGEX series, pTZ series, pCL, pcDNA series, pET series, etc.; more specifically, pSC101, pGV1106, pACYC177, ColE1, pKT230, pME290, pBR322, pUC8/9, pUC6, pBD9, pHC79, pIJ61, pLAFR1, pHV14, pDZ, pACYC177, pACYC184, pCL, pECCG117, pUC19, pBR322, pMW118, pCC1BAC, pcDNA3.1, pcDNA3.3, etc.), phages (e.g., λgt4λB, λ-Charon, λΔz1, M13, etc.), or viruses (e.g., SV40), but with no limitations thereto.

In the recombinant vector, the nucleic acid molecule may be operatively linked to a promoter. The term "operatively linked" is intended to pertain to a functional linkage between a nucleotide sequence of interest and an expression regulatory sequence (for example, a promoter sequence). When being "operatively linked", the regulatory element can control the transcription and/or translation of a polynucleotide of interest.

The recombinant vector may be constructed typically as a cloning vector or an expression vector. For recombinant expression vectors, a vector generally available in the relevant art for expressing a foreign protein in plant, animal, or microbial cells may be employed. Various methods well known in the art may be used for the construction of recombinant vectors.

The recombinant vector may be constructed using prokaryotic or eukaryotic cells as hosts. For example, when a vector is constructed as an expression vector for use in a prokaryotic cell as host, the vector typically includes a strong promoter for transcription (e.g., a pL^{λ} promoter, a CMV promoter, a *trp* promoter, a *lac* promoter, a *tac* promoter, a T7 promoter, etc.), a ribosomal binding site for initiating translation, and transcriptional/translational termination sequences. On the other hand, an expression vector for use in a eukaryotic cell as host typically includes an origin of replication operable in a eukaryotic cell, such as an f1 origin of replication, an SV40 origin of replication, a pMB1 origin of replication, an adeno origin of replication, an AAV origin of replication, and a BBV origin of replication, but is not limited thereto. In addition, the expression vector typically includes a promoter derived from genomes of mammalian cells (for example, metallothionein promoter) or from mammalian viruses (for example, adenovirus late promoter, vaccinia virus 7.5K promoter, SV40 promoter, cytomegalovirus promoter, tk promoter of HSV, etc.), and a polyadenylation sequence as a transcription termination sequence.

The recombinant cell may be prepared by introducing the recombinant vector into a suitable host cell. As long as it allows the sequential cloning and expression of the recombinant vector in a stable manner, any host cell known in the art may be employed in the present disclosure. Examples of the prokaryotic host cell available for the present disclosure may be selected from E. coli such as *E. coli* JM109, *E. coli* BL21, *E. coli* RR1, *E. coli* LE392, *E. coli B, E. coli X* 1776, *E*. *coli* W3110, Bacillus spp. such as *Bacillus subtilis* and *Bacillus thuringiensis,* and enterobacteriaceae strains such as *Salmonella typhimurium, Serratia marcescens* and various Pseudomonas species. Eukaryotic host cells that may be used for transformation may selected from, but are not limited to, *Saccharomyces cerevisiae,* insect cells, plant cells and animal cells, such as Sp2/0, CHO (Chinese hamster ovary) K1, CHO DG44, CHO S, CHO DXB11, CHO GS-KO, PER.C6, W138, BHK, COS-7, 293, HepG2, Huh7, 3T3, RIN, MDCK, etc.

The nucleic acid molecule or a recombinant vector carrying the same may be delivered (introduced) into a host cell using a method well known in the relevant art. For example, this delivery may be carried out using, but not limited to, a CaCl₂ or electroporation method when the host cell is prokaryotic, while for eukaryotic host cells, the genetic introduction may be achieved using, but not limited to, microinjection, calcium phosphate precipitation, electroporation, liposome-mediated transfection, or particle bombardment.

To select a transformed host cell, advantage may be taken of a phenotype associated with a selection marker according to methods well known in the art. For example, when the selection marker is a gene conferring resistance to a certain antibiotic, the host cells may be grown in the presence of the antibiotic in a medium to select a transformant of interest.

Another aspect provides a method for producing the anti-ROR1 antibody, anti-ROR 1/anti-4-1BB bispecific antibody, or anti-ROR1/anti-B7-H3 bispecific antibody, the method comprising a step of expressing the nucleic acid molecules in a host cell. The step of expressing the nucleic acid molecule in a host cell may be a step in which a cell comprising the nucleic acid molecules or a recombinant vector carrying same is cultured. The method may further comprise a step of separating and/or purifying an antibody or antigen-binding fragment from the medium after the culture step.

### Advantageous Effects

The present disclosure provides an improved anti-ROR1 antibody, a bispecific antibody comprising the ROR1 antibody and a T cell engaging antibody (T cell engager), and/or a bispecific antibody comprising the anti-ROR1 antibody and an antibody specific to a tumor-associated antigen (TAA, for example, B7-H3), thus enabling the achievement of superior anticancer effects.

Specifically, designed to provide an anti-ROR1 antibody with superior binding capabilities and enhanced stability and developability and a bispecific antibody comprising same, the present disclosure allows for the more effective and safer production of antibody medicines, compared to conventional therapeutic antibodies, in a cost-effective manner.

### Description of Drawings

FIGS. 1a to 1c are graphs showing the binding affinity of an anti-ROR1 antibody to ROR1 according to an embodiment.
FIG. 2a and 2b are graphs showing the binding affinity of an anti-ROR1/anti-4-1BB bispecific antibody to 4-1BB and/or ROR1 according to an embodiment.
FIG. 2c is a graph showing the binding affinity of an anti-ROR1/anti-4-1BB bispecific antibody to cells expressing 4-1BB on their surface according to an embodiment.
FIG. 2d is a graph showing the binding affinity of an anti-ROR1/anti-4-1BB bispecific antibody to cells expressing ROR1 on their surface according to an embodiment.
FIGS. 3a to 3d are graphs showing the serum stability of an anti-ROR1/anti-4-1BB bispecific antibody according to an embodiment (a and b: in human serum; c and d: in monkey serum).
FIG. 4a is a graph showing 4-1BB signaling activation by an anti-ROR1/anti-4-1BB bispecific antibody in the presence of ROR1 according to an embodiment.
FIG. 4b is a graph showing ROR1-dependent 4-1BB signaling activation by an anti-ROR1/anti-4-1BB bispecific antibody according to an embodiment.
FIG. 4c is a graph showing the correlation between ROR1-dependent 4-1BB signaling activation and ROR1 expression by an anti-ROR1/anti-4-1BB bispecific antibody according to an embodiment.
FIG. 4d is a graph showing FcγRI-dependent 4-1BB signaling activation by an anti-ROR1/anti-4-1BB bispecific antibody according to an embodiment.

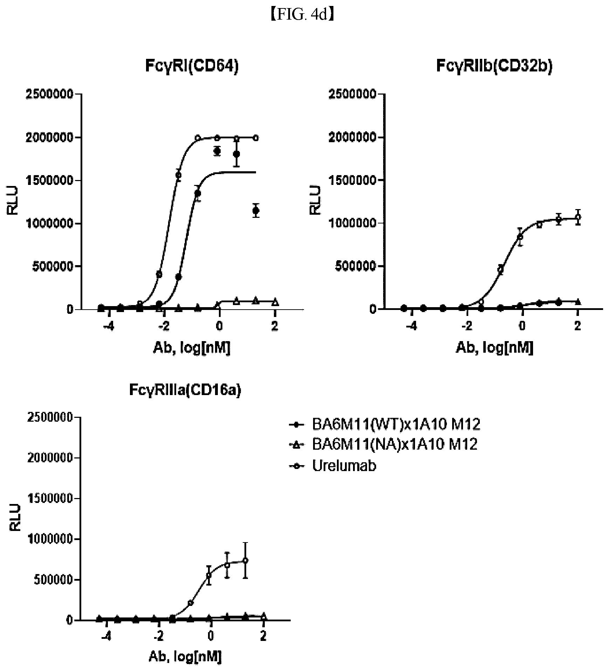

FIG. 4d is a graph showing FcγRI-dependent 4-1BB signaling activation by an anti-ROR1/anti-4-1BB bispecific antibody according to an embodiment.

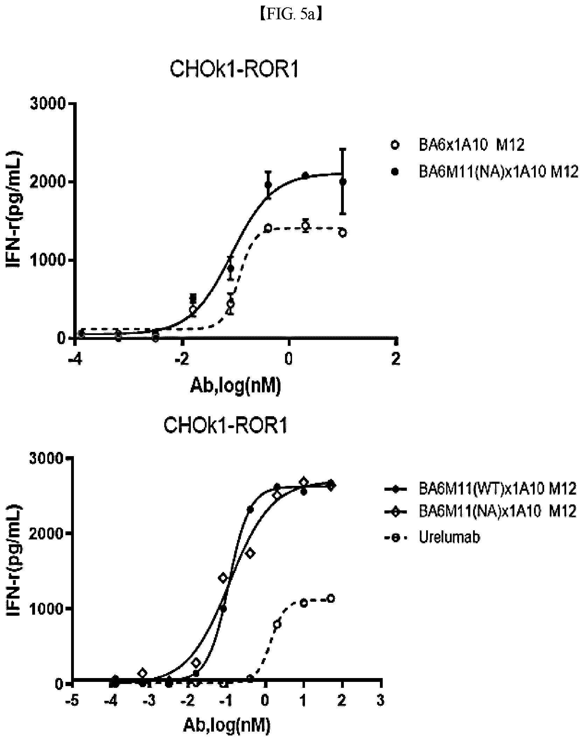

FIG. 5a is a graph demonstrating the activation of PBMCs and the induction of cytokine (interferon-gamma) release by an anti-ROR1/anti-4-1BB bispecific antibody in CHO-K1 cells overexpressing ROR1 according to an embodiment.

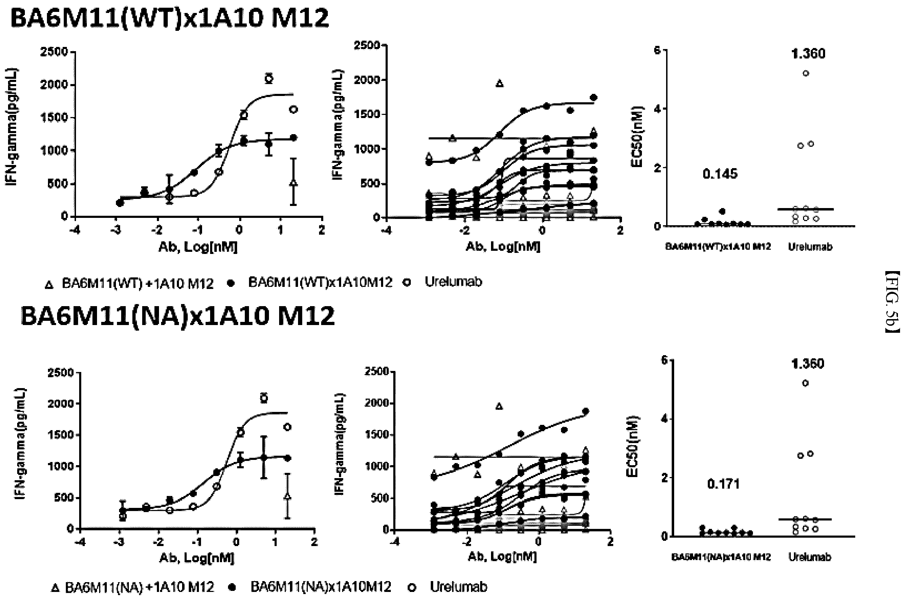

FIG. 5b are graphs illustrating the activation of PBMCs and the induction of cytokine (interferon-gamma) release by an anti-ROR1/anti-4-1BB bispecific antibody in gastric cancer cell lines expressing ROR1 according to an embodiment.

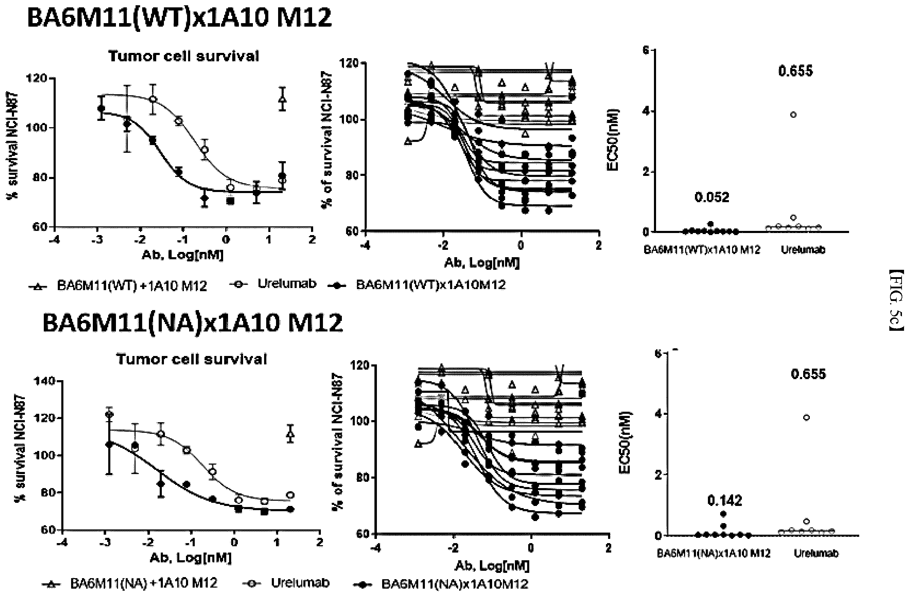

FIG. 5c are graphs illustrating the activation of PBMCs and the induction of cytokine (interferon-gamma) release by an anti-ROR1/anti-4-1BB bispecific antibody in gastric cancer cell lines expressing ROR1 according to an embodiment.

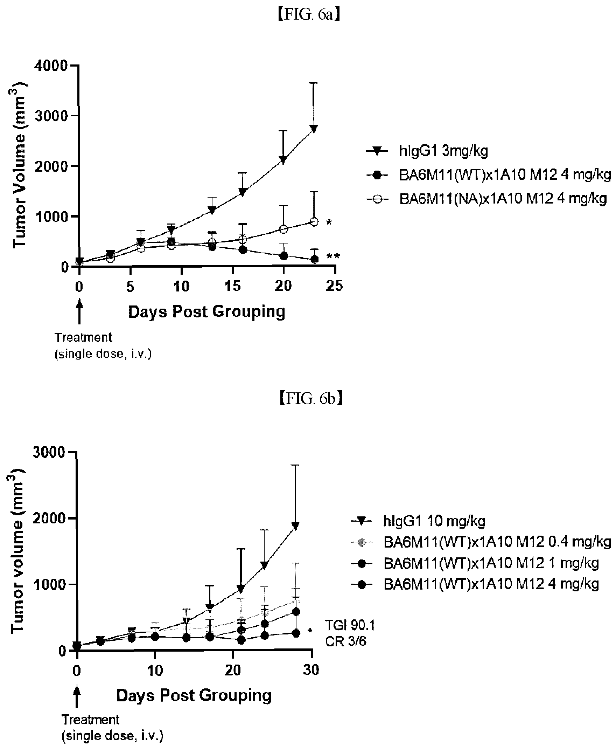

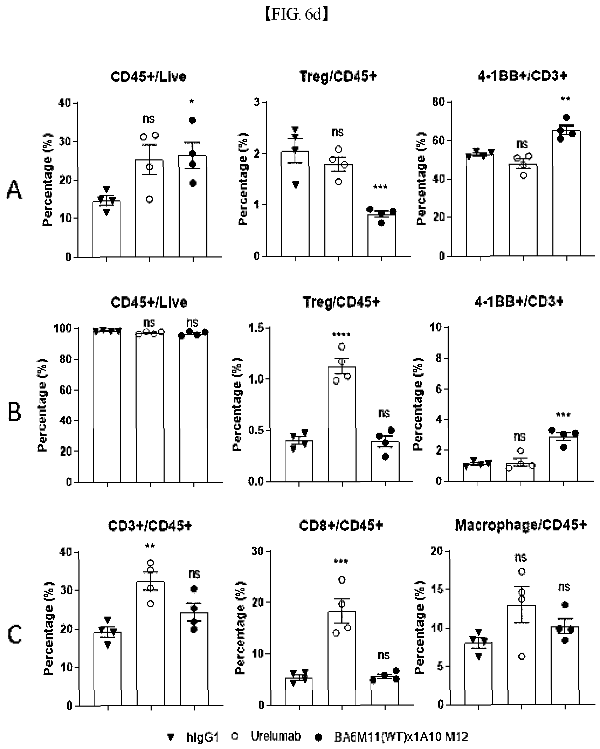

FIGS. 6a and 6b are graphs showing the anti-tumor effect (in vivo) of an anti-ROR1/anti-4-1BB bispecific antibody according to an embodiment.

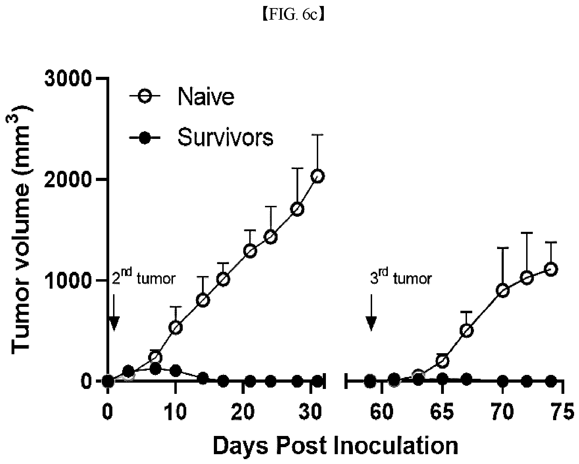

FIG. 6c is a graph depicting the long-lasting anti-tumor memory response by an anti-ROR1/anti-4-1BB bispecific antibody according to an embodiment.
FIG. 6d is a graph presenting the results of measuring immune modulation activity of an anti-ROR1/anti-4-1BB bispecific antibody in tumor, blood, and liver according to an embodiment.

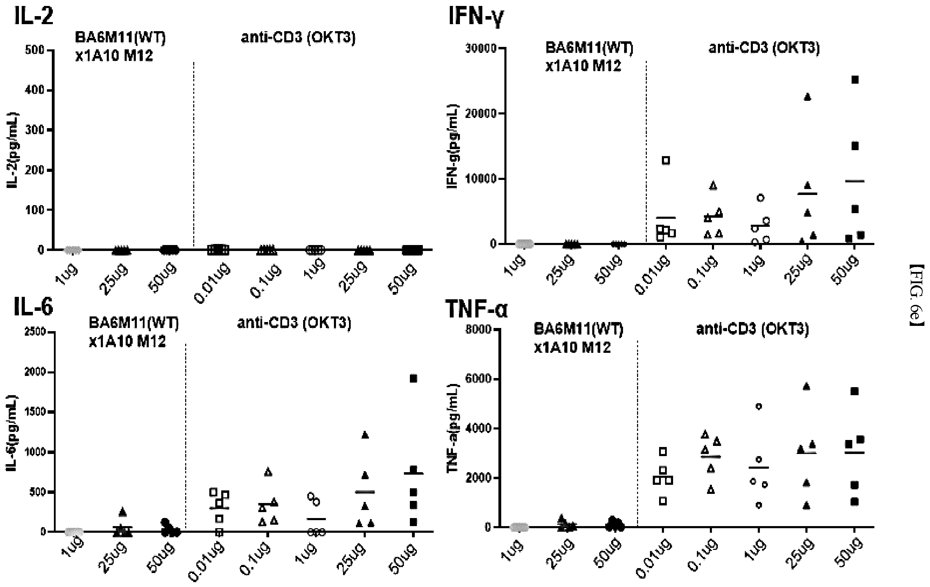

FIG. 6e is a graph showing the results of measuring immune modulation activity of an anti-ROR1/anti-4-1BB bispecific antibody in PBMCs not expressing ROR1 according to an embodiment.

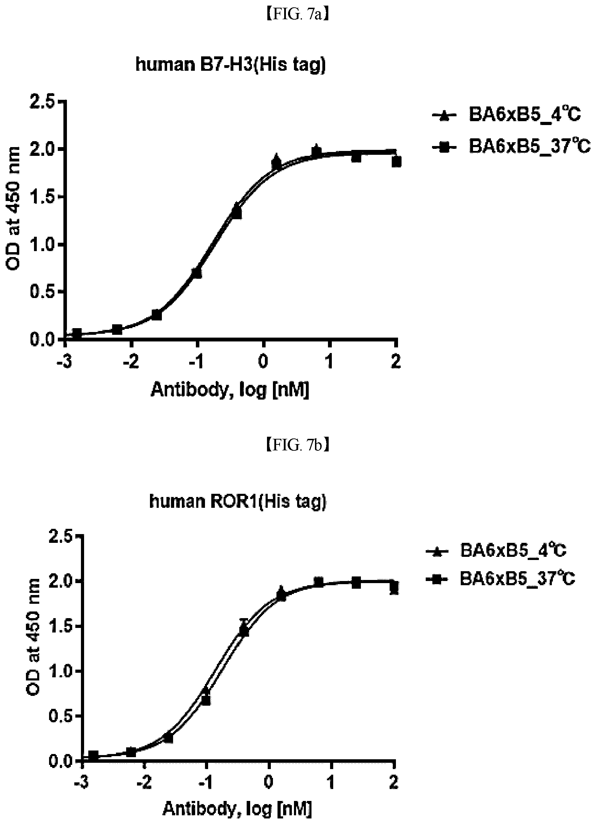

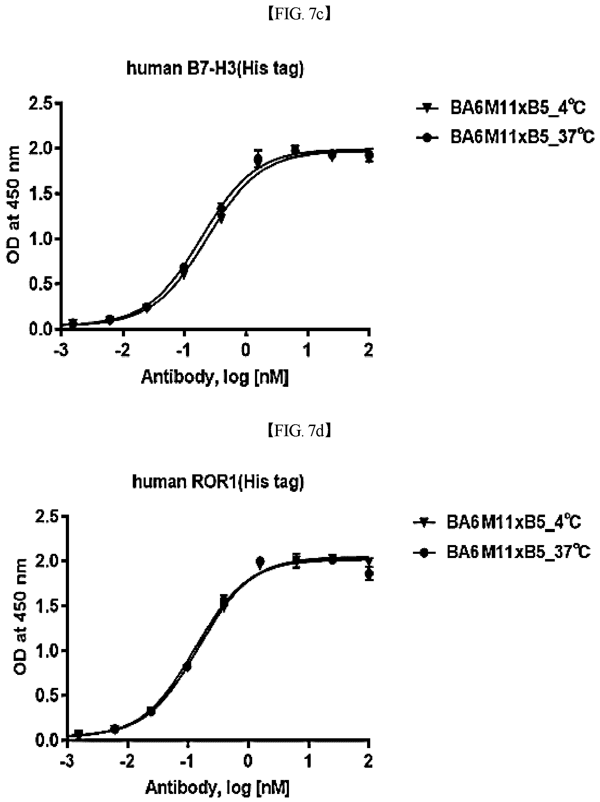

FIGS. 7a-7d are graphs showing the binding affinity of an anti-ROR1/anti-B7-H3 bispecific antibody to ROR1 or B7-H3 according to an embodiment.

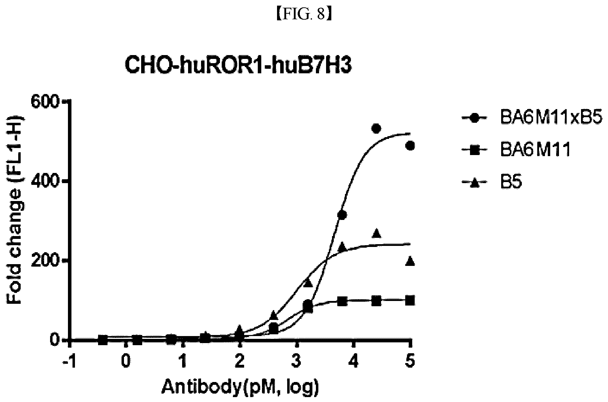

FIG. 8 is a graph demonstrating the binding affinity of an anti-ROR1/anti-B7-H3 bispecific antibody to cells expressing ROR1 and B7-H3 on their surface according to an embodiment.

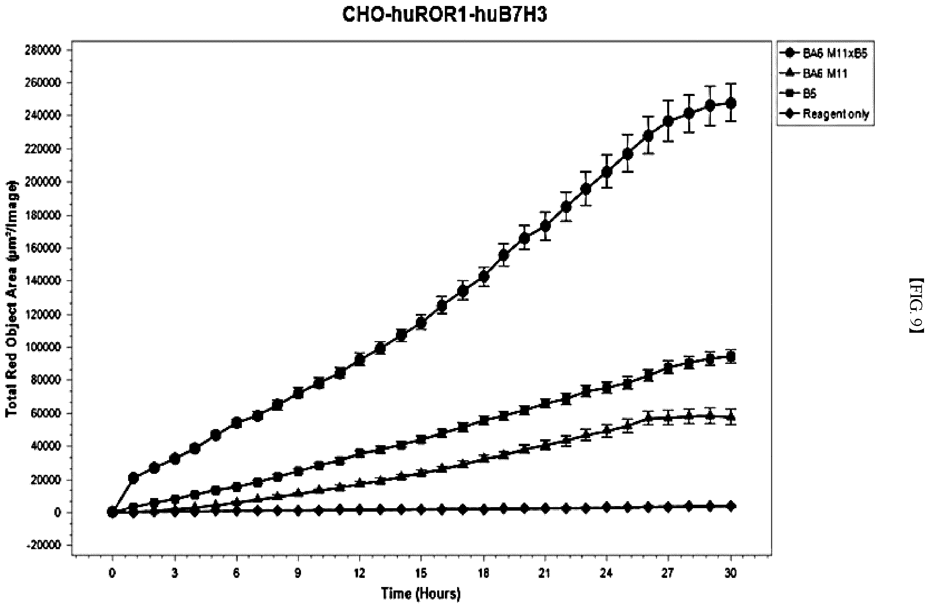

FIG. 9 is a graph illustrating the degree of cellular internalization of an anti-ROR1/anti-B7-H3 bispecific antibody according to an embodiment.

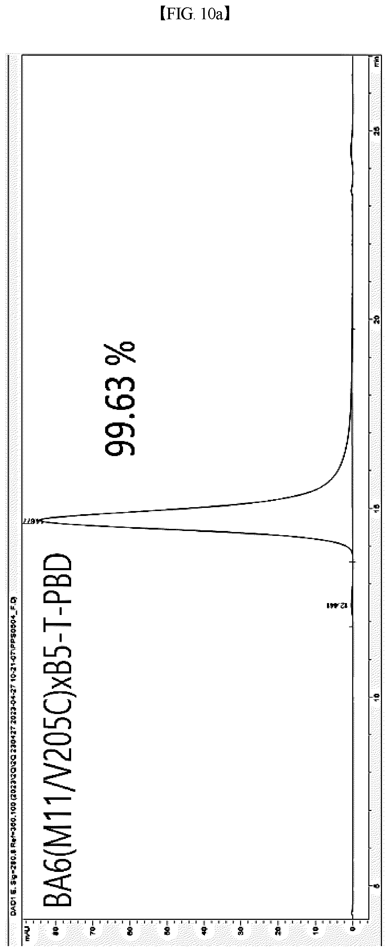

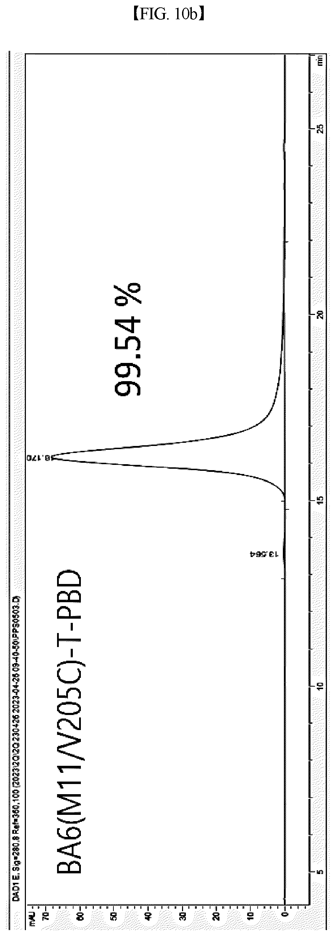

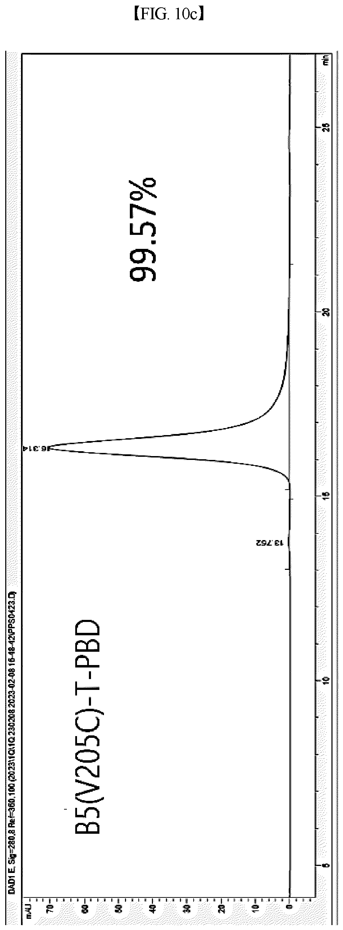

FIGS. 10a to 10c are graphs resenting the results of measuring the purity of an antibody-drug conjugate (ADC) comprising an anti-ROR1/anti-B7-H3 bispecific antibody using size-exclusion high-performance liquid chromatography (SE-HPLC) according to an embodiment. The peak values (min.) shown in FIGS. 10a to 10c are as follows: 10a: 12.441, 14.677; 10b: 13.564, 16.170; 10c: 13.752, 16.314.

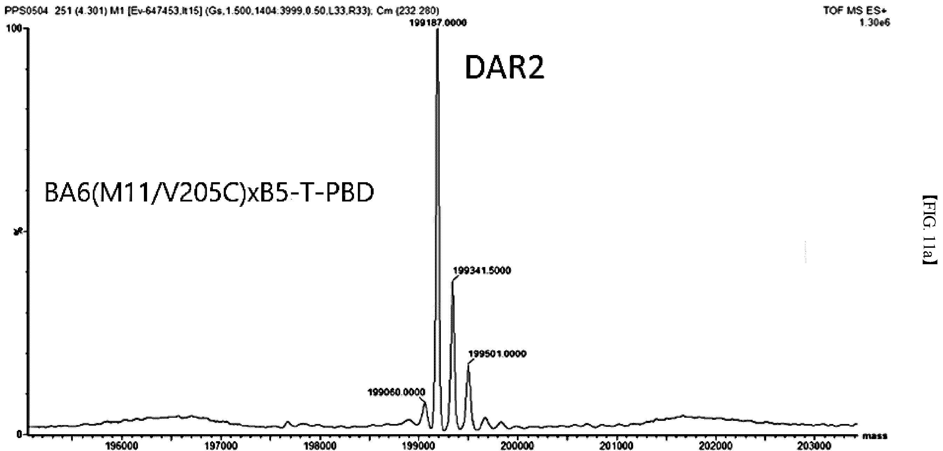

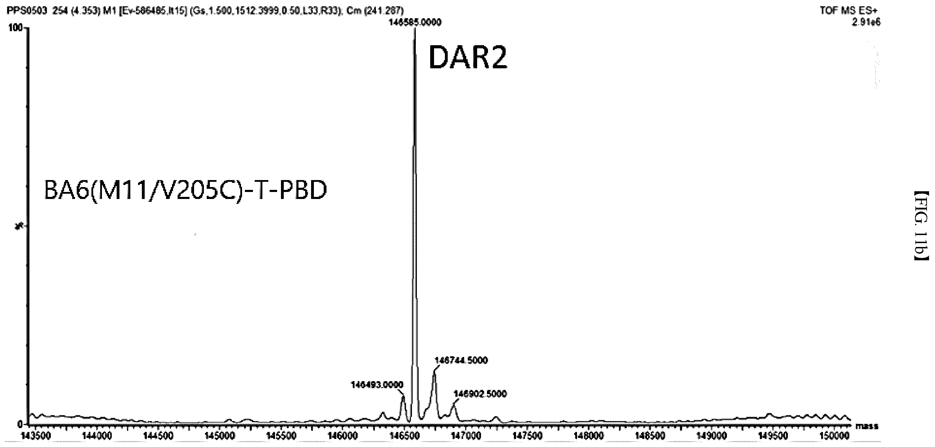

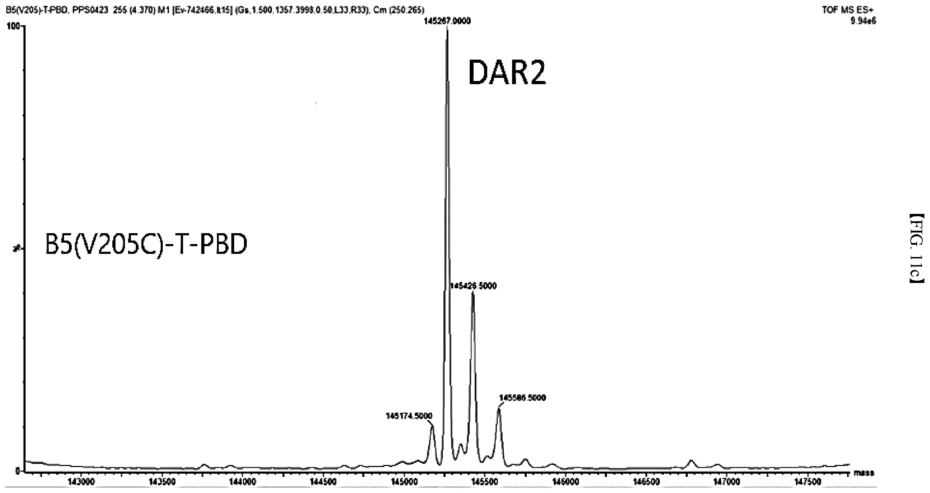

FIG. 11 is a graph showing the results of measuring the drug-to-antibody ratio (DAR) in an ADC comprising an anti-ROR1/anti-B7-H3 bispecific antibody using size-exclusion liquid chromatography-mass spectrometry (LC/MS) according to an embodiment.

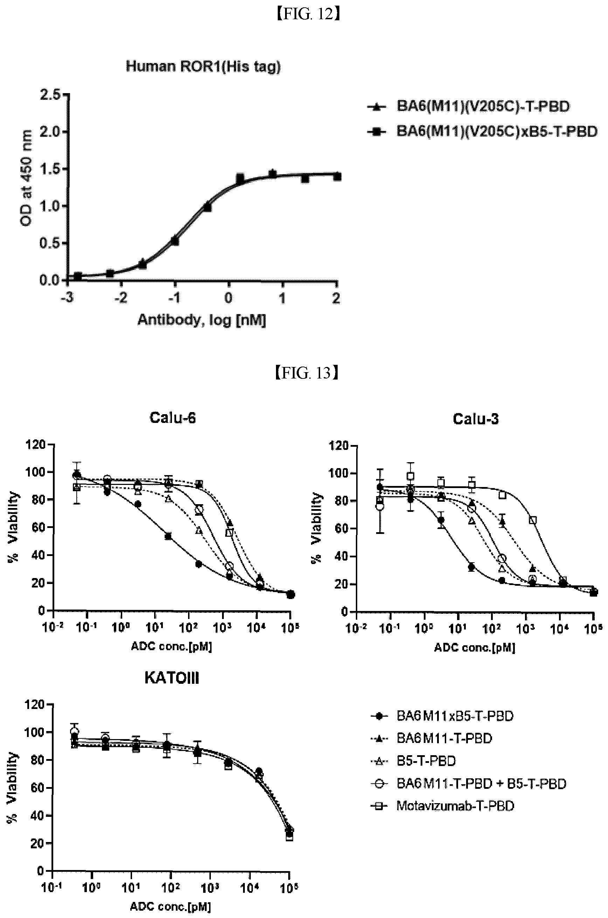

FIG. 12 is a graph demonstrating the binding capability of an ADC comprising an anti-ROR1/anti-B7-H3 bispecific antibody to the antigen (ROR1) according to an embodiment.
FIG. 13 is a graph illustrating the cytotoxicity of an ADC comprising an anti-ROR1/anti-B7-H3 bispecific antibody against cancer cells expressing ROR1 and B7-H3 according to an embodiment.

### Mode for Invention

Hereinafter, the following examples will be described in detail to aid in the understanding of the present invention. However, these examples are provided to illustrate the present invention and are not to be construed as limiting the scope of the present invention. The Examples of the present application are provided to more fully describe the present application to the person with ordinary skill in the art.

### EXAMPLE 1. Anti-ROR1 Antibody

### 1.1. Manufacture of anti-ROR1 antibody

From the anti-ROR1 antibody clone sequence disclosed in International Publication No. WO2019-225992 A1, peptide mapping (Accurate Determination of Succinimide Degradation Products Using High Fidelity Trypsin Digestion Peptide Map Analysis, Anal. Chem. 2011, 83(15): 5912-5919) was employed to identify posttranslational modification (PTM) sites and introduce mutations to remove these sites, thereby manufacturing antibodies with improved physicochemical properties and developability. Among the anti-ROR1 antibodies disclosed in WO2019-225992 A1, the BA6 antibody (WT) (based on IgG1) was chosen as the target for PTM site removal, and the amino acid sequences of the complementarity-determining regions (CDRs) of the heavy chain CDR1 (HCDR1), CDR2 (HCDR2), and CDR3 (HCDR3), as well as the light chain CDR1 (LCDR1), CDR2 (LCDR2), and CDR3 (LCDR3) of the BA6 antibody, are summarized in Table 6 below.

**TABLE 6**

| | Amino acid sequence (N-C) | SEQ ID NO: # |
|---|---|---|
| HCDR1 | NYDMS | 1 |
| HCDR2 | AIYHSGSSKYYADSVKG | 2 |
| HCDR3 | GGNGAWDTGFDY | 9 |
| LCDR1 | SGSSSNIGSNDVS | 4 |
| LCDR2 | YDNNRPS | 10 |
| LCDR3 | GAWDDSLSGYV | 6 |
| VH | EVQLLESGGGLVQPGGSLRLSCAASGFTFSNYDMSWVRQ APGKGLEWVSAIYHSGSSKYYADSVKGRFTISRDNSKNTL YLQMNSLRAEDTAVYYCARGGNGAWDTGFDYWGQGTL VTVSS | 11 |
| VL | QSVLTQPPSASGTPGQRVTISCSGSSSNIGSNDVSWYQQLPG TAPKLLIYYDNNRPSGVPDRFSGSKSGTSASLAISGLRSEDE ADYYCGAWDDSLSGYVFGGGTKLTVL | 12 |

Peptide mapping identified PTMs in LCDR2 and HCDR3. To pinpoint the actual hot spots of PTM within the BA6 antibody sequence, peptide mapping was conducted using the BA6 antibody. After storing the antibody at 4°C and 37°C for two weeks, the samples were digested and fragmented with Immunoglobulin G-degrading enzyme of Streptococcus pyogenes (IdeS; Genovis) and dithiothreitol (DTT), followed by LC/MS analysis, which confirmed the occurrence of PTMs in CDRL2 and CDRH3. Since there were two PTM hot spots, the approach involved dividing them into two groups, inducing single mutations, and then, if the binding activity was found to be similar to the wild-type antibody, combining mutations to create double, triple mutants, etc.

A total of 10 PTM mutants were constructed to verify ligand binding activity. Specifically, to obtain IgGs of each anti-ROR1 antibody clone with PTM removal mutations, mutagenesis primer sets(Macrogen, South Korea) targeting CDRL2 and CDRL3, the PTM hot spots of the above clone, to introduce mutation sequences, were synthesized, and nucleic acid sequences coding for the light and heavy chains were obtained through site-directed mutagenesis using PCR techniques.

For transfection, ExpiCHO-S cells (Thermo Fisher) were prepared at a concentration of 6.0x10⁶ cells/mL in a 500mL flask. Vectors expressing the heavy and light chains (each cloned into separate pcDNA3.4 vectors (Thermo Fisher) for plasmid preparation before use in transfection) and ExpiFectamine (Thermo Fisher) were diluted in OptiPRO medium (Thermo Fisher) to form concentrations of 1 µg/mL and 3.2 µL/mL, respectively, then the diluted vector and ExpiFectamine mixture were mixed and allowed to stand at room temperature for 5 minutes, then added to the flask containing the prepared cells, then the mixture was agitated well and cultured under conditions of 8% CO₂, 37°C, and 120 rpm. The day after performing the transfection, enhancer (Thermo Fisher) at 600µL and feed (Thermo Fisher) at 24mL were each added, and the culture was continued under conditions of 8% CO₂, 37°C, and 120 rpm for 7 days. For detailed experimental protocols, reference was made to the manufacturer's guide of the ExpiCHO expression system provided by Thermo Fisher.

Through the method, a total of 10 mutants (M1-M10) were yielded and used as candidate clones for the anti-ROR1 antibody. These clones each comprised mutations confirmed in PTM sites CDRL2 or CDRH3. Clones M1 to M4 had mutations introduced at L51 in CDRL2 and clones M5-M10 had mutations introduced in CDRH3, and Clone M11 incorporated mutations from M3 (mutation at L51 in CDRL2) and M9 (mutation at H97 in CDRH3), which were selected as leading clones among M1 to M10. The specific information for these candidate clones is summarized in Table 7 below.

**TABLE 7**

| | | | | | |
|---|---|---|---|---|---|
| **PTM** | **deN & IsoD at L51/52** | | **deD at H97** | | **Pass** |
| **Hot spot** | **Yes** | | **Yes** | | |
| **CDR or FR region** | **CDRL2** | | **CDRH3** | | |
| **Kabat numbering** | **L51** | **L52** | **H97** | **H98** | |
| **Location** | **n** | **n+1** | **n** | **n+1** | |
| **Residue** | **D** | **N** | **N** | **G** | |
| M1 | A | | | | O |
| M2 | T | | | | X |
| **M3** | **E** | | | | **O** |
| M4 | S | | | | X |
| M5 | | | | Y | X |
| M6 | | | | V | X |
| M7 | | | | R | X |
| M8 | | | G | | X |
| **M9** | | | **S** | | **O** |
| M10 | | | Y | | X |
| M11 | E | | S | | |

| | | | | | |
|---|---|---|---|---|---|
| (deD: Asp Deamidation, deN: Asn Deamidation, isoD: Aspartate Isomerization; Pass: evaluated based on ligand(antigen) binding activity) | | | | | |

### 1.2. Assay for Binding Specificity of Candidate Clones to Extracellular Domain of ROR1 (ELISA)

Given that the PTM hotspots in the BA6 antibody were identified within the CDR regions, removing PTMs could potentially decrease the binding activity to the antigen compared to WT (wild-type, BA6 antibody) due to sequence changes in the CDR regions, which in turn could reduce the antibody's potency. Thus, the ultimate goal of PTM engineering was to identify clones with equal or superior binding activity to WT. Therefore, the specific binding ability of candidate clones M1 to M11 produced in Example 1.1 to the ROR1 antigen was analyzed as follows.

The binding affinity of the anti-ROR1 antibody-antigen was evaluated using an ELISA-based solution binding assay. Specifically, 96-well microtiter plates (Nunc-Immuno Plates, NUNC) were coated with ROR1 protein (ROR1-His; sino biological, Catalog # 13968-H08H) at a concentration of 1 µg/ml in PBS solution at 4°C for 16 hours, and non-specific binding sites were blocked with 1%(v/v) BSA (bovine serum albumin) for 2 hours.

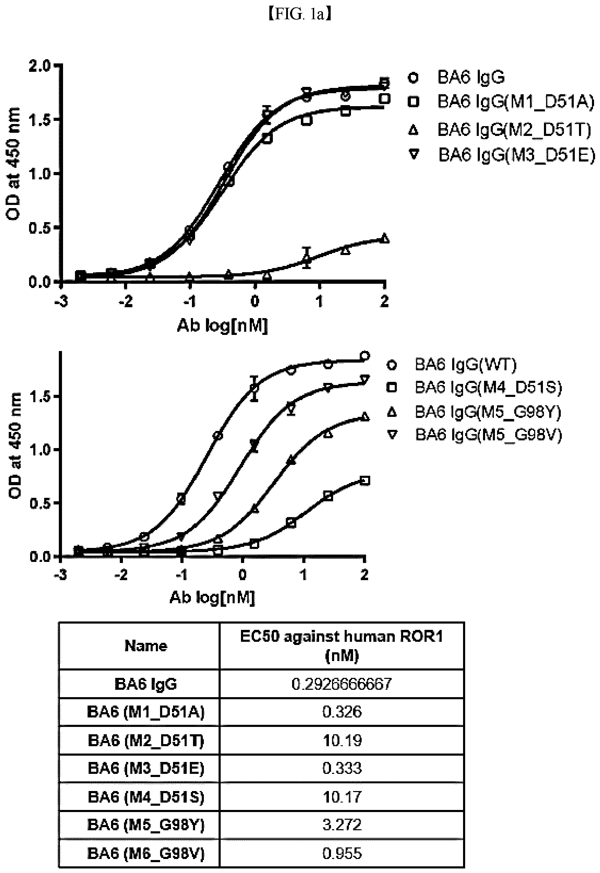

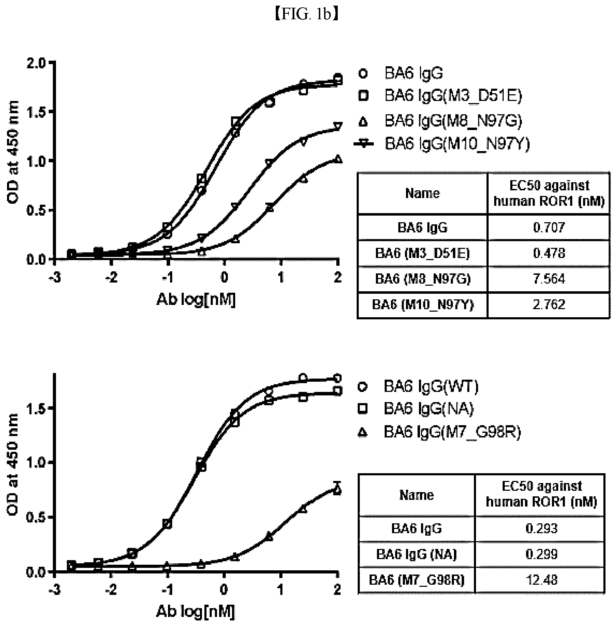

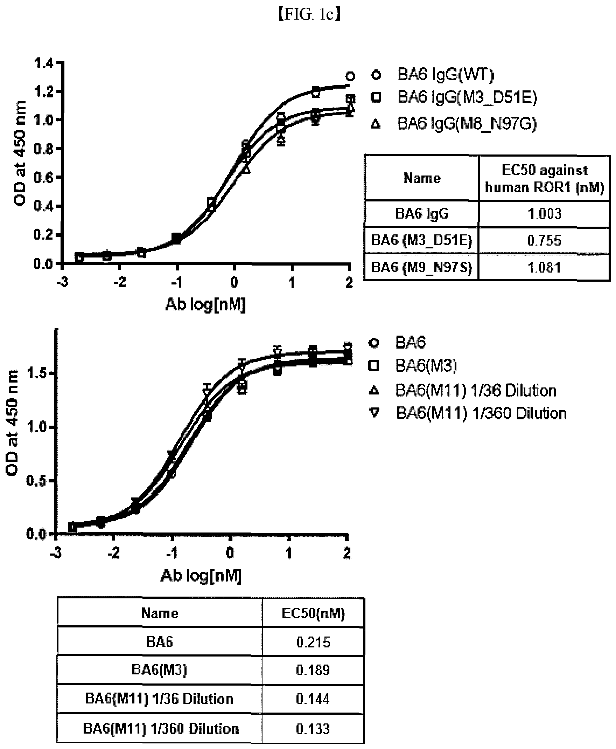

Subsequently, on the 96-well microtiter plates, anti-ROR1 antibodies at concentrations stated in FIGS. 1a to 1c were added to the microtiter plates, and their binding capabilities were analyzed by ELISA as follows. Specifically, after incubating at 37°C for 2 hours, the plates were washed five times with PBS containing 0.05%(v/v) Tween 20, then the HRP-conjugated Fab polyclonal antibody reagent (Pierce, Catalog # 31414) was diluted at a 1:30,000 ratio and added to the washed microtiter plates, then reacted for 1 hour at 37°C, then detected the bound anti-ROR1 antibodies. After reaction, the color was developed using TMB (tetramethylbenzidine, Sigma, T0440). The enzymatic reaction was stopped with 0.5mol/L sulfuric acid, and the absorbance was read at 450nm and 650nm using a microplate reader (Molecular Devices).

The results obtained were presented in FIGS. 1a to 1c. As understood from data of FIGS. 1a to 1c, based on ELISA analysis, M1 and M3 showed similar ligand (antigen) binding activity compared to WT, however, M1 was excluded from the final clone selection due to a lower saturation point than WT. Additionally, none of M7, M8, and M10 showed comparable binding activity to WT. M3 and M9 exhibited comparable binding activity to WT. Consequently, a double mutant M11 clone (D51E (LC) + N97S (HC)) combining M3 and M9 was produced using the same method as in the Example. The M11 clone thus produced was confirmed to exhibit similar binding activity compared to WT and M3, leading to its selection as the final candidate clone.

### 1.3. Analysis for developability of candidate clone

The clone BA6M11 (also denoted as BA6M11) clone (derived from BA6 through PTM engineering to remove PTM liability) the efficacy of which was confirmed in Example 1.2 was assayed for stability under stressed conditions, compared to BA6. More specifically, after storing both the BA6 antibody and the BA6M11 antibody at 4°C (normal conditions) and 37°C (stressed conditions) for two weeks, SE-HPLC, icIEF, and LC/MS analyses were conducted on each sample to check for purity and impurity content. Furthermore, to determine whether changes in the content of the substance also affected efficacy, an antigen-antibody binding ability analysis (ELISA; see Example 1.2) was conducted.

The results thus obtained are summarized in Table 8, below.

**TABLE 8**

| **Content** | **Method** | | **Acceptance Criteria** | **BA6 IgG (WT)** | | **BA6 IgG (M11)** | |
|---|---|---|---|---|---|---|---|
| | | | | **4 °C** | **37 °C** | **4 °C** | **37 °C** |
| **Purity (%)** | **SEC** | | **Δpurity% ≤1.5%** | 99.6 | 98.4 | 99.1 | 98.2 |
| | | | | 1.2 | | 0.9 | |
| **Charge heterogeneity** | **icIEF** | **Basic** | **Information only** | 27.7 | 24.1 | 10.2 | 17.0 |
| | | **Major** | | 35.3 | 27.1 | 74.5 | 69.0 |
| | | **Acidic** | | 37.0 | 48.9 | 15.3 | 14.0 |
| | | **ΔMajor%** | | 8.2 | | 5.5 | |
| **Protein Binding** | **ELISA** | **SACE (EC50 to Human ROR1), nM)** | **70%≤Ratio≤130%, similar curve fit** | 0.217 | 0.261 | 0.166 | 0.129 |
| | | | | 83.1% | | 128.6% | |
| **PTM** | **LC/MS** | | **Report result (Δsuccinimide D %)** | 30.1 | 47.1 | **N/D | **N/D |
| | | | | 17.0 | | *N/A | |

| | | | | | | | |
|---|---|---|---|---|---|---|---|
| (* N/A : Not applicable; ** N/D : Not detected) | | | | | | | |

As shown in Table 8, it was observed that the purity of the BA6M11 clone remained high and comparable to BA6 when stored at both 4°C and 37°C. Moreover, compared to BA6, during the production process, the formation of charge variants in the BA6M11 clone significantly decreased in both basic and acidic conditions, and the formation of charge variants under stressed conditions was also significantly reduced (ΔMajor%). In the BA6M11 clone, peptide mapping analysis confirmed the disappearance of succinimide formation at problematic residues, leading to a reduction in charge variants. The decrease in charge variants indicates a reduced risk of antibody quality variation during production and storage/distribution processes and enhances the reliability of purification processes using ion exchange resins. It also reduces the risk of increased immunogenicity when administered to humans by preventing the hydrolysis of succinimide intermediates, which could lead to antibody chain cleavage, lower potency, and increased immunogenicity. Furthermore, the antigen-antibody binding capacity, verified using human recombinant ROR1 protein (ROR1-His; Sino biological, Catalog # 13968-H08H), showed no significant difference in binding ability between samples stored for two weeks at 4°C and 37°C. These results confirmed that BA6M11 maintained potency comparable to BA6 while improving physicochemical properties in terms of developability.

Ultimately, the BA6M11 clone not only showed comparable binding to WT but also clearly improved developability through PTM engineering.

The amino acid sequences of the novel anti-ROR1 antibody, BA6M11, in the IgG form are listed in Tables 9 and 10.

**TABLE 9**

| | Amino acid sequence (N→C) | SEQ ID NO: # |
|---|---|---|
| BA6_HCDR1 | NYDMS | 1 |
| BA6_HCDR2 | AIYHSGSSKYYADSVKG | 2 |
| BA6_HCDR3 | GGNGAWDTGFDY | 9 |
| BA6_VH | EVQLLESGGGLVQPGGSLRLSCAASGFTFSNYDMSW VRQAPGKGLEWVSAIYHSGSSKYYADSVKGRFTISRD NSKNTLYLQMNSLRAEDTAVYYCARGGNGAWDTGF DYWGQGTLVTVSS | 11 |
| BA6M11_HCD R1 | NYDMS | 1 |
| BA6M11_HCD R2 | AIYHSGSSKYYADSVKG | 2 |
| BA6M11_HCD R3 | GGSGAWDTGFDY | 3 |
| BA6M11_VH | EVQLLESGGGLVQPGGSLRLSCAASGFTFSNYDMSW VRQAPGKGLEWVSAIYHSGSSKYYADSVKGRFTISRD NSKNTLYLQMNSLRAEDTAVYYCARGGSGAWDTGF DYWGQGTLVTVSS | 7 |
| Heavy Chain constant region (WT) | ASTKGPSVFPLAPSSKSTSGGTAALGCLVKDYFPEPVT VSWNSGALTSGVHTFPAVLQSSGLYSLSSVVTVPSSS LGTQTYICNVNHKPSNTKVDKKVEPKSCDKTHTCPPC PAPELLGGPSVFLFPPKPKDTLMISRTPEVTCVVVDVS HEDPEVKFNWYVDGVEVHNAKTKPREEQYNSTYRV VSVLTVLHQDWLNGKEYKCKVSNKALPAPIEKTISK AKGQPREPQVYTLPPSREEMTKNQVSLTCLVKGFYPS DIAVEWESNGQPENNYKTTPPVLDSDGSFFLYSKLTV DKSRWQQGNVFSCSVMHEALHNHYTQKSLSLSPGK | 104 |
| Heavy Chain constant region (NA) | ASTKGPSVFPLAPSSKSTSGGTAALGCLVKDYFPEPVT VSWNSGALTSGVHTFPAVLQSSGLYSLSSVVTVPSSS LGTQTYICNVNHKPSNTKVDKKVEPKSCDKTHTCPPC PAPELLGGPSVFLFPPKPKDTLMISRTPEVTCVVVDVS HEDPEVKFNWYVDGVEVHNAKTKPREEQYASTYRV VSVLTVLHQDWLNGKEYKCKVSNKALPAPIEKTISK AKGQPREPQVYTLPPSREEMTKNQVSLTCLVKGFYPS DIAVEWESNGQPENNYKTTPPVLDSDGSFFLYSKLTV | 105 |
| | DKSRWQQGNVFSCSVMHEALHNHYTQKSLSLSPGK | |

| | Nucleic acid sequence (5'→3') | SEQ ID NO: # |
|---|---|---|
| BA6M11_VH | GAGGTGCAGCTGCTGGAGTCCGGCGGCGGCCTGGT GCAGCCCGGCGGCTCCCTGCGGCTGTCCTGCGCCG CCTCCGGCTTCACCTTCTCCAACTACGACATGTCCT GGGTGCGGCAGGCCCCCGGCAAGGGCCTGGAGTG GGTGTCCGCCATCTACCACTCCGGCTCCTCCAAGTA CTACGCCGACTCCGTGAAGGGCCGGTTCACCATCT CCCGGGACAACTCCAAGAACACCCTGTACCTGCAG ATGAACTCCCTGCGGGCCGAGGACACCGCCGTGTA CTACTGCGCCCGGGGCGGCTCCGGCGCCTGGGACA CCGGCTTCGACTACTGGGGCCAGGGCACCCTGGTG ACCGTGTCCTCC | 106 |
| Heavy Chain constant region (WT) | GCCTCCACCAAGGGCCCCTCCGTGTTCCCCCTGGCC CCCTCCTCCAAGTCCACCTCCGGCGGCACCGCCGC CCTGGGCTGCCTGGTGAAGGACTACTTCCCCGAGC CCGTGACCGTGTCCTGGAACTCCGGCGCCCTGACC TCCGGCGTGCACACCTTCCCCGCCGTGCTGCAGTCC TCCGGCCTGTACTCCCTGTCCTCCGTCGTGACCGTG CCCTCCTCCTCCCTGGGCACCCAGACCTACATCTGC AACGTGAACCACAAGCCCTCCAACACCAAGGTGGA CAAGAAGGTGGAGCCCAAGTCCTGCGACAAGACC CACACCTGCCCTCCCTGCCCCGCCCCCGAGCTGCTG GGCGGCCCCTCCGTGTTCCTGTTCCCTCCTAAGCCC AAGGACACCCTGATGATCTCCCGGACCCCCGAGGT GACTTGCGTGGTGGTGGACGTGTCCCACGAGGACC CCGAGGTGAAGTTCAACTGGTACGTGGACGGCGTG GAGGTGCACAACGCCAAGACCAAGCCCCGGGAGG AGCAGTACAACTCCACCTACCGGGTGGTGTCCGTG CTGACCGTGCTGCACCAGGACTGGCTGAACGGCAA GGAGTACAAGTGCAAGGTGTCCAACAAGGCCCTGC CCGCCCCCATCGAGAAGACCATCTCCAAGGCCAAG GGCCAGCCCCGGGAGCCCCAGGTGTACACCCTGCC CCCCTCCCGGGAGGAGATGACCAAGAACCAGGTGT | 107 |
| | CCCTGACCTGCCTGGTGAAGGGCTTCTACCCCTCCG ACATCGCCGTGGAGTGGGAGTCCAACGGCCAGCCC GAGAACAACTACAAGACCACCCCCCCCGTGCTGGA CTCCGACGGCTCCTTCTTCCTGTACTCCAAGCTGAC CGTGGACAAGTCCCGGTGGCAGCAGGGCAACGTGT TCTCCTGCTCCGTGATGCACGAGGCCCTGCACAAC CACTACACCCAGAAGTCCCTGTCCCTGTCCCCCGGC AAG | |

**TABLE 10**

| | Amino acid sequence (N→C) | SEQ ID NO: # |
|---|---|---|
| BA6_LCDR1 | SGSSSNIGSNDVS | 4 |
| BA6_LCDR2 | YDNNRPS | 10 |
| BA6_LCDR3 | GAWDDSLSGYV | 6 |
| BA6_VL | QSVLTQPPSASGTPGQRVTISCSGSSSNIGSNDVSWYQ QLPGTAPKLLIYYDNNRPSGVPDRFSGSKSGTSASLAI SGLRSEDEADYYCGAWDDSLSGYVFGGGTKLTVL | 12 |
| BA6M11_LCD R1 | SGSSSNIGSNDVS | 4 |
| BA6M11_LCD R2 | YENNRPS | 5 |
| BA6M11_LCD R3 | GAWDDSLSGYV | 6 |
| BA6M11_VL | QSVLTQPPSASGTPGQRVTISCSGSSSNIGSNDVSWYQ QLPGTAPKLLIYYENNRPSGVPDRFSGSKSGTSASLAI SGLRSEDEADYYCGAWDDSLSGYVFGGGTKLTVL | 8 |
| Light Chain constant region | GQPKAAPSVTLFPPSSEELQANKATLVCLISDFYPGAV TVAWKADSSPVKAGVETTTPSKQSNNKYAASSYLSL TPEQWKSHRSYSCQVTHEGSTVEKTVAPAECS | 108 |

| | Nucleic acid sequence (5'→3') | SEQ ID NO: # |
|---|---|---|
| BA6M11_VL | CAGTCCGTGCTGACCCAGCCCCCCTCCGCCTCCGGC ACCCCCGGCCAGCGGGTGACCATCTCCTGCTCCGG | 109 |
| | CTCCTCCTCCAACATCGGCTCCAACGACGTGTCCTG GTACCAGCAGCTGCCCGGCACCGCCCCCAAGCTGC TGATCTACTACGAAAACAACCGGCCCTCCGGCGTG CCCGACCGGTTCTCCGGCTCCAAGTCCGGCACCTCC GCCTCCCTGGCCATCTCCGGCCTGCGGTCCGAGGA CGAGGCCGACTACTACTGCGGCGCCTGGGACGACT CCCTGTCCGGCTACGTGTTCGGCGGCGGCACCAAG CTGACCGTGCTG | |
| Light Chain constant region | GGCCAGCCCAAGGCCGCCCCCTCCGTGACCCTGTT CCCCCCCTCCTCCGAGGAGCTGCAGGCCAACAAGG CCACCCTGGTGTGCCTGATCTCCGACTTCTACCCCG GCGCCGTGACCGTGGCCTGGAAGGCCGACTCCTCC CCCGTGAAGGCCGGCGTGGAGACCACCACCCCCTC CAAGCAGTCCAACAACAAGTACGCCGCCTCCTCCT ACCTGTCCCTGACCCCCGAGCAGTGGAAGTCCCAC CGGTCCTACTCCTGCCAGGTGACCCACGAGGGCTC CACCGTGGAGAAGACCGTGGCCCCCGCCGAGTGCT CC | 110 |

### EXMAPLE 2. Anti-ROR1/Anti-4-1BB Bispecific Antibody

### 2.1. Manufacture of improved anti-ROR1/anti-4-1BB bispecific antibody

An anti-ROR1/anti-4-1BB bispecific antibody was manufactured based on the improved anti-ROR1 antibody clone BA6M11, produced and selected in Example 1, and one of the anti-4-1BB antibody clones disclosed in International Publication Number WO2020/111913 A1, namely 41B01.03 (also referred to as 1A10M12).

In this Example, a bispecific antibody comprising an anti-ROR1 IgG (full-length) antibody and a 4-1BB scFv connected to the C-terminus of the heavy chain of the anti-ROR1 IgG (full-length) antibody was constructed.

To construct the anti-ROR1/anti-4-1BB bispecific antibody, DNA segment 1 having a nucleotide sequence coding for the heavy chain of the anti-ROR1 IgG antibody (BA6M11) was inserted into pcDNA 3.4 (Invitrogen, A14697; plasmid 1) while DNA segment 2 having a nucleotide sequence coding for the light chain of the IgG antibody in the anti-ROR1/anti-4-1BB bispecific antibody was inserted into pcDNA 3.4 (Invitrogen, A14697; plasmid 2).

Subsequently, DNA segment 3 encoding the scFv was fused at the portion corresponding to the C-terminal of the Fc region of the IgG antibody inserted in plasmid 1, using DNA segment 4 encoding a 20 amino acid-length peptide linker composed of (GGGGS)4 (SEQ ID NO: 114), or DNA segment 5 encoding an 18 amino acid-length linker peptide composed of (GS)9 (SEQ ID NO: 113), to construct a vector for the expression of the bispecific antibody.

Additionally, the anti-4-1BB scFv (named 1A10M12) took a form in which VL103-VH44 (VL103: VL with a G(Gly)→C(Cys) mutation introduced at position 103; VH44: VH with a G→C mutation introduced at position 44) at the C-termini of the light chain variable region (VL) and heavy chain variable region (VH) respectively, were fused through the linker ((GGGGS)4, SEQ ID NO: 114). These mutations stabilized the scFv and enable the formation of disulfide bridges.

The amino acid sequences of the bispecific antibody thus prepared are listed in Table 11 below.

**TABLE 11**

| | Amino acid sequence (N→C) | SEQ ID NO: # |
|---|---|---|
| Light chain BA6M11 | QSVLTQPPSASGTPGQRVTISCSGSSSNIGSNDVSWY QQLPGTAPKLLIYYENNRPSGVPDRFSGSKSGTSASL AISGLRSEDEADYYCGAWDDSLSGYVFGGGTKLTVL GQPKAAPSVTLFPPSSEELQANKATLVCLISDFYPGA VTVAWKADSSPVKAGVETTTPSKQSNNKYAASSYL SLTPEQWKSHRSYSCQVTHEGSTVEKTVAPAECS | 111 |

Heavy chain BA6M11(WT)x1A10M12

| | | |
|---|---|---|
| Heavy chain BA6M11(WT) | EVQLLESGGGLVQPGGSLRLSCAASGFTFSNYDMSW VRQAPGKGLEWVSAIYHSGSSKYYADSVKGRFTISR DNSKNTLYLQMNSLRAEDTAVYYCARGGSGAWDT GFDYWGQGTLVTVSSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVD KKVEPKSCDKTHTCPPCPAPELLGGPSVFLFPPKPKD | 112 |
| | TLMISRTPEVTCVVVDVSHEDPEVKFNWYVDGVEV HNAKTKPREEQYNSTYRVVSVLTVLHQDWLNGKEY KCKVSNKALPAPIEKTISKAKGQPREPQVYTLPPSREE MTKNQVSLTCLVKGFYPSDIAVEWESNGQPENNYK TTPPVLDSDGSFFLYSKLTVDKSRWQQGNVFSCSVM HEALHNHYTQKSLSLSPGK | |
| Linker1 | GSGSGSGSGSGSGSGSGS | 113 |
| 1A10M12 (VL) | QSVLTQPPSASGTPGQRVTISCSGSSSNIGNNYVTWY QQLPGTAPKLLIYADSHRPSGVPDRFSGSKSGTSASL AISGLRSEDEADYYCATWDYSLSGYVFGCGTKLTVL | 46 |
| Linker2 | GGGGSGGGGSGGGGSGGGGS | 114 |
| 1A10M12 (VH) | EVQLLESGGGLVQPGGSLRLSCAASGFTFSSYDMSW VRQAPGKCLEWVSWISYSGGSIYYADSVKGRFTISR DNSKNTLYLQMNSLRAEDTAVYYCARDAQRNSMR EFDYWGQGTLVTVSS | 37 |
| Full-length | EVQLLESGGGLVQPGGSLRLSCAASGFTFSNYDMSW VRQAPGKGLEWVSAIYHSGSSKYYADSVKGRFTISR DNSKNTLYLQMNSLRAEDTAVYYCARGGSGAWDT GFDYWGQGTLVTVSSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVD KKVEPKSCDKTHTCPPCPAPELLGGPSVFLFPPKPKD TLMISRTPEVTCVVVDVSHEDPEVKFNWYVDGVEV HNAKTKPREEQYNSTYRVVSVLTVLHQDWLNGKEY KCKVSNKALPAPIEKTISKAKGQPREPQVYTLPPSREE MTKNQVSLTCLVKGFYPSDIAVEWESNGQPENNYK TTPPVLDSDGSFFLYSKLTVDKSRWQQGNVFSCSVM HEALHNHYTQKSLSLSPGKGSGSGSGSGSGSGSGS QSVLTQPPSASGTPGQRVTISCSGSSSNIGNNYVTWYQQL PGTAPKLLIYADSHRPSGVPDRFSGSKSGTSASLAISGLRS EDEADYYCATWDYSLSGYVFGCGTKLTVLGGGGSGGG GSGGGGSGGGGSEVQLLESGGGLVQPGGSLRLSCAA SGFTFSSYDMSWVRQAPGKCLEWVSWISYSGGSIYY ADSVKGRFTISRDNSKNTLYLQMNSLRAEDTAVYYC ARDAQRNSMREFDYWGQGTLVTVSS | 115 |
| Heavy chain BA6M11(NA)x1A10M12 | | |
| Heavy chain BA6M11(NA) | EVQLLESGGGLVQPGGSLRLSCAASGFTFSNYDMSW VRQAPGKGLEWVSAIYHSGSSKYYADSVKGRFTISR DNSKNTLYLQMNSLRAEDTAVYYCARGGSGAWDT GFDYWGQGTLVTVSSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVD KKVEPKSCDKTHTCPPCPAPELLGGPSVFLFPPKPKD TLMISRTPEVTCVVVDVSHEDPEVKFNWYVDGVEV HNAKTKPREEQYASTYRVVSVLTVLHQDWLNGKEY KCKVSNKALPAPIEKTISKAKGQPREPQVYTLPPSREE MTKNQVSLTCLVKGFYPSDIAVEWESNGQPENNYK TTPPVLDSDGSFFLYSKLTVDKSRWQQGNVFSCSVM HEALHNHYTQKSLSLSPGK | 116 |
| Linker1 | GSGSGSGSGSGSGSGSGS | 113 |
| 1A10M12 (VL) | QSVLTQPPSASGTPGQRVTISCSGSSSNIGNNYVTWY QQLPGTAPKLLIYADSHRPSGVPDRFSGSKSGTSASL 4 AISGLRSEDEADYYCATWDYSLSGYVFGCGTKLTVL | 46 |
| Linker2 | GGGGSGGGGSGGGGSGGGGS | 114 |
| 1A10M12 (VH) | EVQLLESGGGLVQPGGSLRLSCAASGFTFSSYDMSW VRQAPGKCLEWVSWISYSGGSIYYADSVKGRFTISR DNSKNTLYLQMNSLRAEDTAVYYCARDAQRNSMR EFDYWGQGTLVTVSS | 37 |
| Full-length | EVQLLESGGGLVQPGGSLRLSCAASGFTFSNYDMSW VRQAPGKGLEWVSAIYHSGSSKYYADSVKGRFTISR DNSKNTLYLQMNSLRAEDTAVYYCARGGSGAWDT GFDYWGQGTLVTVSSASTKGPSVFPLAPSSKSTSGGT AALGCLVKDYFPEPVTVSWNSGALTSGVHTFPAVLQ SSGLYSLSSVVTVPSSSLGTQTYICNVNHKPSNTKVD KKVEPKSCDKTHTCPPCPAPELLGGPSVFLFPPKPKD TLMISRTPEVTCVVVDVSHEDPEVKFNWYVDGVEV HNAKTKPREEQYASTYRVVSVLTVLHQDWLNGKEY KCKVSNKALPAPIEKTISKAKGQPREPQVYTLPPSREE MTKNQVSLTCLVKGFYPSDIAVEWESNGQPENNYK | 117 |
| | TTPPVLDSDGSFFLYSKLTVDKSRWQQGNVFSCSVM HEALHNHYTQKSLSLSPGKGSGSGSGSGSGSGSGS QSVLTQPPSASGTPGQRVTISCSGSSSNIGNNYVTWYQQL PGTAPKLLIYADSHRPSGVPDRFSGSKSGTSASLAISGLRS EDEADYYCATWDYSLSGYVFGCGTKLTVLGGGGSGGG GSGGGGSGGGGSEVQLLESGGGLVQPGGSLRLSCAA SGFTFSSYDMSWVRQAPGKCLEWVSWISYSGGSIYY ADSVKGRFTISRDNSKNTLYLQMNSLRAEDTAVYYC ARDAQRNSMREFDYWGQGTLVTVSS | |

| | | |
|---|---|---|
| (Linker1: BA6M11(IgG) linked to 1A10M12(scFv) (C-terminus of heavy chain of BA6M11 linked to N-terminus of VL of 1A10M12); Linker2: VL linked to VH in 1A10M12 (scFv) (C-terminus of VL linked to N-terminus of VH) | | |

The coding nucleic acid sequence (5'→3') of the bispecific antibody is as follows:
[Heavy chain_BA6M11(WT)x1A10M12] (SEQ ID NO: 118) 
gaggtgcagctgctggagtccggcggcggcctggtgcagcccggcggctccctgcggctgtcctgcgccgcctcc
ggcttcaccttctccaactacgacatgtcctgggtgcggcaggcccccggcaagggcctggagtgggtgtccgcc
atctaccactccggctcctccaagtactacgccgactccgtgaagggccggttcaccatctcccgggacaactcc
aagaacaccctgtacctgcagatgaactccctgcgggccgaggacaccgccgtgtactactgcgcccggggcggc
tccggcgcctgggacaccggcttcgactactggggccagggcaccctggtgaccgtgtcctctgcttctaccaag
ggaccctctgtgttccctctggctccttccagcaagtctacctctggtggaaccgctgctctgggctgcctggtc
aaggattactttcctgagcctgtgacagtgtcctggaactctggtgctctgacctctggcgtgcacacctttcca
gctgtgctgcagtcctctggcctgtactctctgtcctctgtcgtgaccgtgccatcttctagcctgggcacccag
acctacatctgcaacgtgaaccacaagccttccaacaccaaggtggacaagaaggtggaacccaagtcctgcgac
aagacccacacctgtcctccatgtcctgctccagaactgctcggcggtccctccgttttcctgtttccacctaag
cctaaggacaccctgatgatctctcggacccctgaagtgacctgcgtggtggtggatgtgtctcacgaggatccc
gaagtgaagttcaattggtacgtggacggcgtggaagtgcacaacgccaagaccaagcctagagaggaacagtac
aactccacctacagagtggtgtctgtgctgaccgtgctgcaccaggattggctgaacggcaaagagtacaagtgc
aaggtgtccaacaaggccctgcctgctcctatcgaaaagaccatctccaaggctaagggccagcctcgggaacct
caagtgtacaccctgcctcctagcagagaagagatgaccaagaaccaggtgtccctgacctgcctggtcaagggc
ttctacccttccgatatcgccgtggaatgggagtccaatggccagcctgagaacaactacaagaccacacctcct
gtgctggactccgacggctcattcttcctgtactccaagctgaccgtggacaagtccagatggcagcagggcaac
gtgttctcctgctccgtgatgcacgaggccctgcacaatcactacacccagaagtccctgtctctgtcccctggc
aaaggctccggatctggttctggatccggaagcggttctggcagcggatctggatctcagtccgtgctgactcag
cctccttctgcttctggcacccctggccaaagagtgacaatctcttgctccggctcctcctccaacatcggcaac
aactacgtgacctggtatcagcagctgcccggcacagctcccaaactgctgatctacgccgactctcacagacct
tccggcgtgcccgatagattctccggctctaagtctggcacctctgccagcctggctatctccggcctgagatct
gaggacgaggccgactactactgcgccacctgggattattccctgtccggctacgtgttcggctgcggcacaaaa
ctgacagtgcttggcggcggaggatctggcggaggtggaagcggaggcggaggaagcggtggcggcggatctgaa
gttcagctgttggaaagtggcggaggcctggttcaacctggcggatctctgagactgtcttgtgccgcctccggc
tttaccttctcctcctacgacatgtcctgggtccgacaggctcctggcaagtgtctggaatgggtttcctggatc
tcctactccggcggctccatctactacgccgattccgtgaagggcagattcaccatcagccgggacaactccaag
aacaccctgtacctgcagatgaactccctgagagccgaggacaccgccgtgtactactgtgctagagatgcccag
cggaacagcatgagagagttcgactattggggccagggcaccctggtcacagtctcttct
[Light chain_BA6M11] (SEQ ID NO: 119)

cagtccgtgctgacccagcccccctccgcctccggcacccccggccagcgggtgaccatctcctgctccggctcc
tcctccaacatcggctccaacgacgtgtcctggtaccagcagctgcccggcaccgcccccaagctgctgatctac
tacgaaaacaaccggccctccggcgtgcccgaccggttctccggctccaagtccggcacctccgcctccctggcc
atctccggcctgcggtccgaggacgaggccgactactactgcggcgcctgggacgactccctgtccggctacgtg
ttcggcggcggcaccaagctgaccgtgctgggccagcccaaggccgccccctccgtgaccctgttccccccctcc
tccgaggagctgcaggccaacaaggccaccctggtgtgcctgatctccgacttctaccccggcgccgtgaccgtg
gcctggaaggccgactcctcccccgtgaaggccggcgtggagaccaccaccccctccaagcagtccaacaacaag
tacgccgcctcctcctacctgtccctgacccccgagcagtggaagtcccaccggtcctactcctgccaggtgacc
cacgagggctccaccgtggagaagaccgtggcccccgccgagtgctcc

The coding DNA of the bispecific antibody cloned as described in the foregoing were transiently expressed using the ExpiCHO system (Thermo Fisher) according to the manufacturer's protocol. The culture with expressed antibodies was cleared of suspensions using centrifugation and filtration, and the antibody was purified using affinity chromatography with Mabselect Sure^{™} resin and size exclusion chromatography with Superdex200 resin. The purity of the purified antibody was analyzed using HPLC with TSKgel SuperSW3000, and its purity was found to be excellent, exceeding 95%.

### 2.2. Evaluation of anti-ROR1/anti-4-1BB bispecific antibody

### 2.2.1. Dual-antigen capture ELISA (DACE)

The binding affinities of the anti-ROR1/anti-4-1BB bispecific antibodies comprising either the BA6M11 clone (PTMs removal clone, test group) or the BA6 clone (parental clone, control group) to ROR1 and 4-1BB were compared and analyzed using the ELISA method. Two versions of the test group BA6M11 clone were used: BA6M11(NA), which includes the N297A (NA) mutation that reduces ADCC, and BA6M11(WT), which does not include such mutation.

Sequences of the anti-ROR1/anti-4-1BB bispecific antibody comprising BA6 clone used as control are as follows:
Light chain BA6 (SEQ ID NO: 124) 
QSVLTQPPSASGTPGQRVTISCSGSSSNIGSNDVSWYQQLPGTAPKLLIYYDN
NRPSGVPDRFSGSKSGTSASLAISGLRSEDEADYYCGAWDDSLSGYVFGGGTKLTV
LGQPKAAPSVTLFPPSSEELQANKATLVCLISDFYPGAVTVAWKADSSPVKAGVETT
TPSKQSNNKYAASSYLSLTPEQWKSHRSYSCQVTHEGSTVEKTVAPAECS
Heavy chain BA6x1A10M12 (SEQ ID NO: 125)

EVQLLESGGGLVQPGGSLRLSCAASGFTFSNYDMSWVRQAPGKGLEWVSAI
YHSGSSKYYADSVKGRFTISRDNSKNTLYLQMNSLRAEDTAVYYCARGGNGAWDT
GFDYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKKV

EPKSCDKTHTCPPCPAPELLGGPSVFLFPPKPKDTLMISRTPEVTCVVVDVSHEDPEV
KFNWYVDGVEVHNAKTKPREEQYASTYRVVSVLTVLHQDWLNGKEYKCKVSNK
ALPAPIEKTISKAKGQPREPQVYTLPPSREEMTKNQVSLTCLVKGFYPSDIAVEWESN
GQPENNYKTTPPVLDSDGSFFLYSKLTVDKSRWQQGNVFSCSVMHEALHNHYTQK
SLSLSPGKGSGSGSGSGSGSGSGSGSQSVLTQPPSASGTPGQRVTISCSGSSSNIGNNYVT
WYQQLPGTAPKLLIYADSHRPSGVPDRFSGSKSGTSASLAISGLRSEDEADYYCATWDYSLS
GYVFGCGTKLTVLGGGGSGGGGSGGGGSGGGGSEVQLLESGGGLVQPGGSLRLSCA
ASGFTFSSYDMSWVRQAPGKCLEWVSWISYSGGSIYYADSVKGRFTISRDNSKNTL
YLQMNSLRAEDTAVYYCARDAQRNSMREFDYWGQGTLVTVSS

Briefly, 96-well microtiter plates (NUNC, 446612) were coated overnight at 4°C with human ROR1-Fc protein (Sinobio, 13968-H02H1) in PBS at 10 ng/well, then the plates were incubated with 1% BSA buffer (1xPBS, 1%(v/v) BSA) at 200 µl/well for 2 hours at 37°C to prevent nonspecific binding. After blocking nonspecific binding, the wells were washed, and serial dilutions of the bispecific antibody test materials (starting at 100 nM) were added to each well. After incubation for 2 hours at 37°C, the plates were washed with PBS-T (1xPBS/0.05%(v/v) Tween-20). Then, human 4-1BB-His protein (Sinobio, 10041-H08H) dissolved in 1%(v/v) BSA was added to each well at 100 ng/well and incubated for 1 hour at 37°C. After washing the plates with PBS-T, anti-His HRP (Roche, Cat: 11965085001) was added and incubated for 1 hour at 37°C. After washing, color development was conducted TMB (tetramethylbenzidine, Sigma, T0440) and the reaction was stopped with 0.5M sulfuric acid (Samchun Chemicals, S1410), followed by reading absorbance (OD) at 450-650nm in a spectrophotometer. Four-parameter logistic curve analysis was performed using GraphPad software.

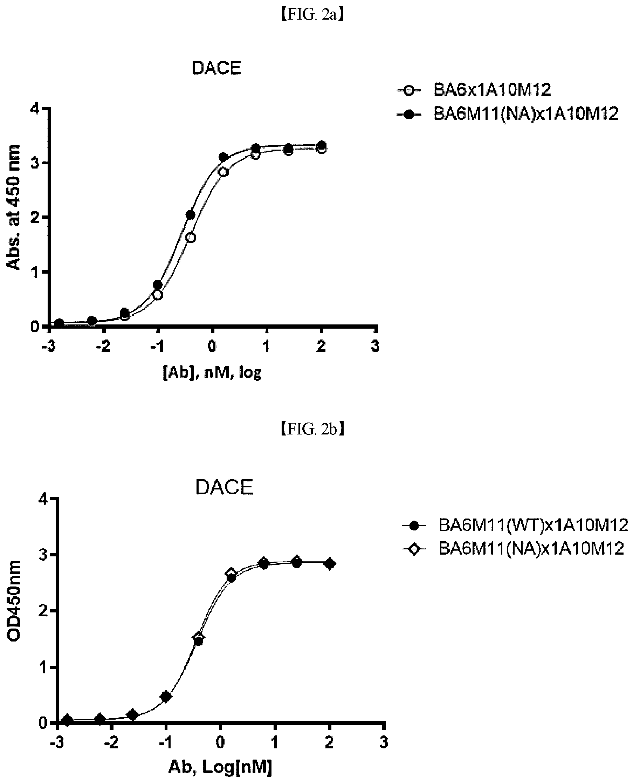

The results obtained through the experiments are presented in Table 12 and FIGS. 2a and 2b.

**TABLE 12**

| ROR1x4-1BB | BA6x1A10M12 | BA6M11(NA)x1A10M1 2 |
|---|---|---|
| EC50(nM) | 0.386 | 0.268 |

As shown in FIG. 2a and Table 12, the bispecific antibody comprising BA6M11 did not show any decrease in binding affinity compared to the parental clone BA6, and instead, an improvement in binding affinity was observed as indicated by the lower EC50.

Additionally, when measuring the difference in binding affinity due to the presence or absence of the NA mutation, BA6M11(WT)x1A10M12 and BA6M11(NA)x1A10M12 showed no difference in binding activity to human ROR1 and 4-1BB.

Considering the fact that it is often challenging to maintain the binding affinity of a parental clone upon changes to its CDR sequences, the finding that the antigen-binding affinity of BA6M11, which had its sequence altered by PTM removal, was equivalent to or better than that of the parental clone BA6, demonstrates desirable characteristics of BA6M11 and its potential and advantages for further development as a therapeutic antibody.

### 2.2.2. Binding assay to cell surface antigen (Flow cytometry)

Using various cells expressing human ROR1 or human 4-1BB on their surface, the binding affinity of anti-ROR1/anti-4-1BB bispecific antibodies comprising either the BA6M11(WT) or BA6M11(NA) clone was compared by measuring with a flow cytometry device (BD).

In particular, Jurkat cells overexpressing human 4-1BB (Promega) and NCI-N87 cells, a human ROR1 positive cell line (ATCC), were seeded into V-bottom 96-well plates (96 Well Plate-RV, Bioneer, 910D09) and washed with 200 µl/well of 1% BSA buffer. Fourfold dilutions of the test materials (bispecific antibodies) starting from 100 nM were added to each well and incubated for 1 hour at 4°C. The plates were then washed with 1% BSA buffer. To each well, FITC-conjugated anti-human IgG (Fc specific) (Sigma, F9512) dissolved in 1% BSA buffer was added and incubated for 1 hour at 4°C. The plates were washed again with 1% BSA buffer. The mean fluorescence intensity (MFI) of FITC was measured by flow cytometry. Four-parameter logistic curve analysis was performed on the obtained MFI values using GraphPad software.

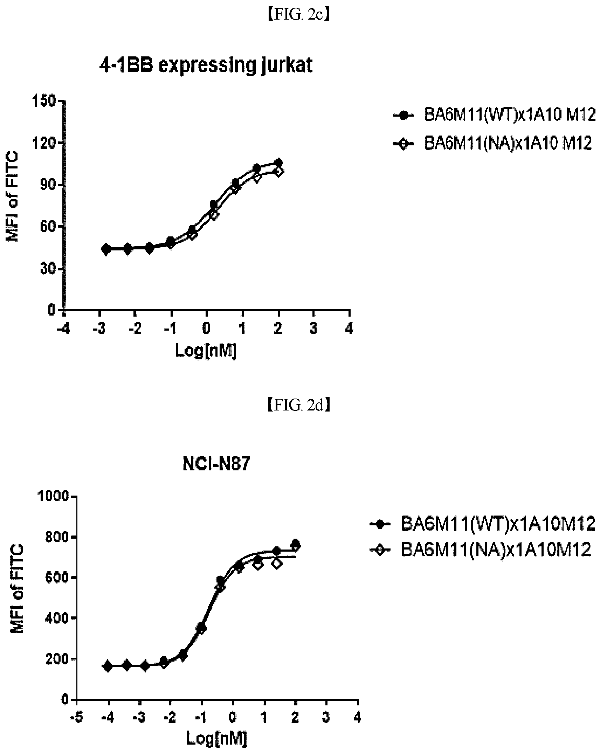

As shown in FIGS. 2c and 2d, both BA6M11(WT)x1A10M12 and BA6M11(NA)x1A10M12 bound similarly to Jurkat cells overexpressing human 4-1BB and ROR1-positive NCI-N87 cells, demonstrating that there is no difference in binding activity to human 4-1BB and human ROR1.

### 2.2.3. Binding affinity assay using surface plasmon resonance (SPR)

The binding affinity of both BA6M11(WT)x1A10M12 and BA6M11(NA)x1A10M12 clones, which are anti-ROR1/anti-4-1BB bispecific antibodies comprising BA6M11 clone, was assessed using surface plasmon resonance (SPR). The antigens used for this assessment were human ROR1 protein (13968-H02H1, Fc tag) from Sino Biological and human 4-1BB protein (9220-4B-100, his tag) from R&D Systems. The measurements were conducted on a Biacore T200 instrument (Cytiva).

Candidate materials were measured for binding affinity for human ROR1 as follows.

The CM5 chip (Cytiva, BR100530) was coated with a 5 µg/mL human ROR1 protein dilution in Acetate 4.0 (Cytiva, BR100349) using the amine coupling method (Cytiva, BR100050) to achieve a surface density of about 80 RU. The BA6M11(WT)x1A10M12 and BA6M11(NA)x1A10M12 samples were two-fold serially diluted in HBS-EP buffer (1x) (Cytiva, BR100669) to form concentrations of 100 nM, 50 nM, 25 nM, 12.5 nM, 6.25 nM, and 0 nM. These diluted antibody samples were then flowed over the human ROR1 protein-immobilized CM5 chip for approximately 60 seconds to conjugate the sample to the antigen, followed by flowing HBS-EP buffer (1x) over the sample-antigen complex for 180 seconds to dissociate the sample from the antigen. The chip surface was regenerated by flowing Glycine-HCl pH1.5 (Cytiva, BR100354) over the chip for 30 seconds. The assay was performed with HBS-EP (1x) as the running buffer, at a flow rate of 30 µL/min and at a temperature of 25°C. Binding affinity was analyzed using the bivalent analyte model to derive the primary association rate constant (Ka1) and primary dissociation rate constant (Kd1). The equilibrium dissociation constant (KD) was calculated by dividing the primary dissociation rate constant by the primary association rate constant, and this value was used to represent the final binding affinity.

Candidate materials were measured for binding affinity for human 4-1BB as follows: 10 µg/mL anti-Fc antibody (Invitrogen, 31125) diluted in 5.0 (Cytiva, BR100351)was fixed at about 15000 RU onto a CM5 chip (Cytiva, BR100530) using the amine coupling method. BA6M11(WT)x1A10M12 and BA6M11(NA)x1A10M12 diluted in HBS-EP (1x) buffer were then bound at 300 RU to the anti-Fc antibody-immobilized CM5 chip. Human 4-1BB antigen was serially two-fold diluted in HBS-EP (1x) buffer to form concentrations of125 nM, 62.5 nM, 31.25 nM, 15.625 nM, 7.8125 nM, and 0 nM. These diluted human 4-1BB samples were then flowed over the CM5 chip for approximately 60 seconds to conjugate the sample to the antigen, followed by flowing HBS-EP buffer (1x) over the sample-antigen complex for 180 seconds to dissociate the sample from the antigen. The chip surface was regenerated by flowing Glycine-HCl pH1.5 (Cytiva, BR100354) over the chip for 30 seconds. The assay was performed with HBS-EP (1x) as the running buffer, at a flow rate of 30 µL/min and at a temperature of 25°C. The binding affinity was determined using a 1:1 binding model to derive the association rate constant (Ka) and dissociation rate constant (Kd). The equilibrium dissociation constant (KD) was calculated by dividing the dissociation rate constant by the association rate constant, and this value was used as the final measure of binding affinity.

The results thus obtained are summarized in Table 13, below:

**TABLE 13**

| Antigen | Antibody sample | Ka (1/Ms, x10⁵) | Kd (1/s, x10⁻³) | KD (M, x10-⁹) | Rmax (RU) |
|---|---|---|---|---|---|
| Human ROR1 | BA6M11(WT) x1A10M12 | 2.408 ± 0.001 | 1.456 ± 0.066 | 6.049 ±0.277 | 40.56 ± 0.74 |
| | BA6M11(NA) x1A10M12 | 2.289 ±0.047 | 1.522 ± 0.129 | 6.654 ± 0.701 | 38.87 ± 0.14 |

| Antigen | Antibody sample | Ka (1/Ms, x10⁵) | Kd (1/s, x10⁻⁴) | KD (M, x10-⁹) | Rmax (RU) |
|---|---|---|---|---|---|
| Human 4-1BB | BA6M11(WT) x1A10M12 | 2.236 ± 0.022 | 10.12 ± 0.31 | 4.527 ± 0.098 | 43.52 ± 0.48 |
| | BA6M11(NA) x1A10M12 | 2.207 ± 0.027 | 9.924 ± 0.292 | 4.497 ± 0.078 | 39.77 ± 0.43 |

As indicated in Table 13, both BA6M11(WT)x1A10M12 and BA6M11(NA)x1A10M12 exhibited similar levels of binding to human ROR1 and human 4-1BB, confirming that there was no difference in antigen-binding activity between the WT and NA cell lines.

### 2.2.4. Assay for developability of anti-ROR1/4-1BB bispecific antibody

Anti-ROR1/4-1BB bispecific antibodies comprising BA6 clone or BA6M11 clone were assayed for developability.

For the analysis of developability, capillary isoelectric focusing (cIEF) or imaged capillary isoelectric focusing (icIEF) were conducted. Both methods analyze changes in charge variants using the isoelectric point (pI) value.

Samples for the developability analysis were prepared by exposing them to temperatures of 4°C and 37°C (or 40°C) for two weeks. The storage temperatures were 4°C or 37°C for the BA6x1A10M12 clone and 4°C or 40°C for both BA6M11(WT)x1A10M12 and BA6M11(NA)x1A10M12 clones.

The cIEF analysis was carried out using a PA 800 plus instrument from Beckman Coulter, equipped with a UV detector. A neutral capillary cartridge (50 µm I.D.) was crafted to a total length of 30.2 cm and mounted in the instrument.

The bispecific antibody samples used for analysis were diluted with a formulation buffer (20 mM histidine, 7% (w/v) trehalose, pH 6.0) to for a final concentration of 5 mg/mL. The master mix solution was prepared using a 3M urea solution including gel (Urea: GE Healthcare, 17-1319-01; cIEF Gel: Beckman Coulter, 477497), ampholyte 3-10 (Pharmalyte 3-10; GE Healthcare, 17-0456-01), an anodic stabilizer solution (200 mM iminodiacetic acid; Sigma, 220000), a cathodic stabilizer solution (500 mM L-arginine; Sigma, A5006), and synthetic peptide isoelectric point markers (10, 9.5, 7.0, 5.5, 4.1; cIEF Peptide Marker Kit; Beckman Coulter, A58481). 10 µL of analysis sample(bispecific antibody) was mixed with 239 µL of the master mix solution and centrifuged. 200 µL of the mixture was transferred to a PCR tube, placed in a plastic vial, and prepared on the sample tray. Distilled water, anolyte solution (200 mM phosphoric acid; Sigma, 345-245), catholyte solution (300 mM sodium hydroxide; Sigma, 79724), capillary cleaning solution (4.3 M urea; GE Healthcare, 17-1319-01), cIEF gel solution (Beckman Coulter, 477497), and chemical mobilizer solution (350 mM acetic acid; Sigma, 695092) were prepared in individual plastic vials on the buffer tray for analysis.

For the icIEF analysis, the Maurice instrument and capillary cartridge from ProteinSimple were utilized. The Maurice cIEF Cartridge (ProteinSimple, PS-MC02-C) was filled with 2 mL of catholyte solution (100 mM sodium hydroxide in 0.1% methyl cellulose; ProteinSimple) and 2 mL of anolyte solution (0.08 M phosphoric acid in 0.1% methyl cellulose; ProteinSimple) and then mounted in the instrument.

The bispecific antibody samples for analysis were diluted in distilled water to form a final concentration of 1 mg/mL. The master mix solution was prepared using an 8 M Urea solution (Sigma, U6504), 1% methyl cellulose (Methyl Cellulose; ProteinSimple), ampholytes (pH 3-10) (Pharmalyte pH 3-10; ProteinSimple, 042-684 or GE Healthcare, 17-0456-01), distilled water (UltraPure^{™} DNase/RNase-Free Distilled Water; Invitrogen, 10977015), and peptide isoelectric point markers (5.85, 9.50; ProteinSimple). 20 µL of the analysis sample(bispecific antibody) was mixed with 80 µL of the master mix solution, centrifuged, and then transferred to a 96-well plate for analysis.

Distilled water, 0.5% methyl cellulose (ProteinSimple), fluorescence calibration standard (ProteinSimple), and air were prepared in individual vials and placed in their respective positions for analysis.

The results of this analysis are presented in Table 14, below:

**TABLE 14**

| Content | Method | | BA6x1A10M12 | | BA6M11(WT)x1A 10M12 | | BA6M11(NA)x1A 10M12 | |
|---|---|---|---|---|---|---|---|---|
| | | | 4 °C | 37 °C | 4 °C | 40 °C | 4 °C | 40°C |
| Charge heterogeneity | cIEF or icIEF | Acidic | 24.7 | 27.3 | 22.5 | 36.7 | 21.0 | 35.8 |
| | | Main | 51.6 | 34.6 | 75.4 | 59.3 | 77.5 | 60.2 |
| | | Basic | 23.7 | 38.1 | 2.1 | 3.9 | 1.5 | 4.0 |

The developability assessment results presented in Table 14 indicate that the main peak content of BA6x1A10M12 was about 52% under storage conditions at 4°C. This clone showed a decrease of about 17% in the content of the main peak under storage conditions at 37°C compared to 4°C, while the basic peak content increased by about 14%.

Assay results for developability of the two BA6M11 clone variants with modified BA6 clone are as follows: The main peak content at 4°C was approximately 75% for BASM11(WT)x1A10M12 and about 78% for BA6M11(NA)x1A10M12. Both clones showed a decrease of about 16-17% in the content of the main peak under storage conditions at 40°C compared to 4°C, with an increase of about 2-3% in the content of the basic peaks.

The BA6M11 clones tested showed improved characteristics in the content of basic charge variants compared to BA6. A decrease in the content of basic charge variants was confirmed through the analysis of samples stored at 4°C, indicating a reduction during production as well, and the rate of change under high-temperature storage conditions (40°C) was also reduced, and the two mutant clones, based on samples stored at 4°C, showed that the WT and NA forms have similar main peak contents. From these results, it was confirmed that the BA6M11 clones possess superior developability compared to the parent BA6 clone.

### 2.2.5. Analysis for serum stability of anti-ROR1/anti-4-1BB bispecific antibody

To evaluate the serum stability of the anti-ROR1/anti-4-1BB bispecific antibodies comprising either the BA6 clone (parental clone) or the BA6M11 clone (PTMs removed), they were stored in human and monkey serum and then analyzed and compared using the ELISA method.

In this analysis, the anti-ROR1/anti-4-1BB bispecific antibodies were stored in human and monkey serum at 37°C for 2 days, 7 days, and 14 days.

A 96-well microtiter plate (NUNC, 446612) was coated overnight at 4°C with human ROR1-Fc protein (Sinobio, 13968-H02H1) at 100 ng/well in PBS. The plate was then incubated with 1% BSA buffer (1xPBS, 1% (v/v) BSA) at 200 µl/well at 37°C for 2 hours to block non-specific binding. Thereafter, the blocking solution was discarded and fourfold dilutions of each bispecific antibody test material (starting from 100nM) were added to the wells. The plate was incubated at 37°C for 2 hours and then washed with PBS-T (1xPBS/0.05% (v/v) Tween-20). Subsequently, human 4-1BB-His protein (Sinobio, 10041-H08H) dissolved in 1% BSA buffer was added at 100ng/well and incubated at 37°C for 1 hour. After washing with PBS-T, the plate was incubated with anti-His HRP (Roche, Cat: 11965085001) at 37°C for 1 hour. After washing, color development was performed with TMB (tetramethylbenzidine, Sigma, T0440) and the color development was stopped with 0.5M sulfuric acid (Samchun Chemicals, S1410), and the absorbance (OD) at 450-650nm was read using a spectrophotometer. Four-parameter logistic curve analysis was performed using GraphPad software.

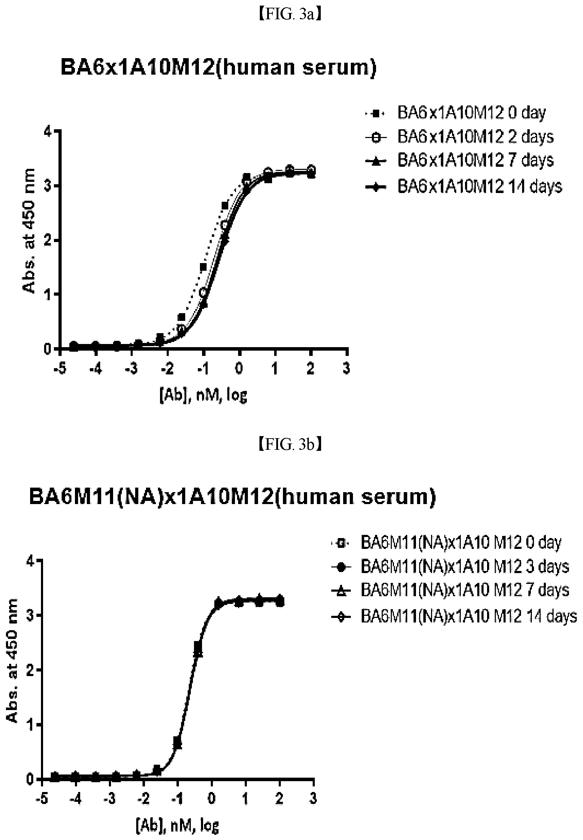

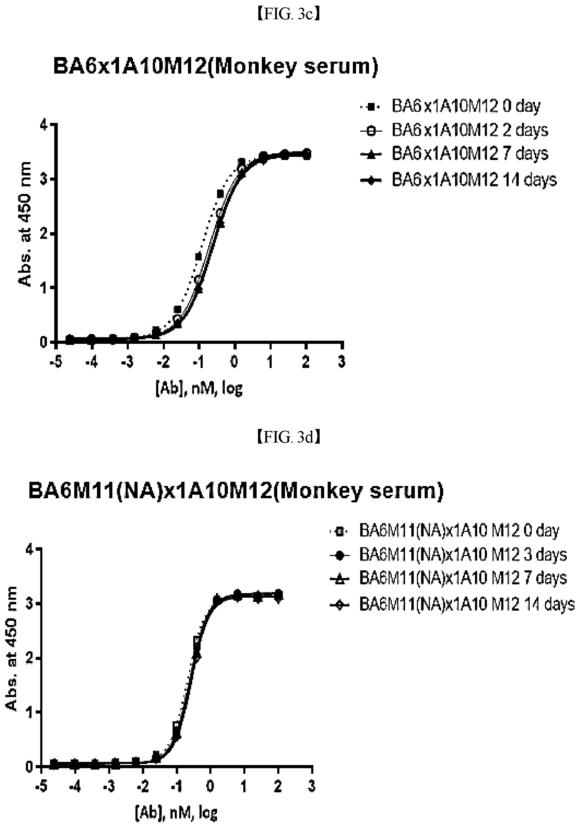

The results obtained are presented in Table 15 and FIGS. 3a to 3d.

**TABLE 15**

| BA6x1A10M12 | | | | BA6M11(NA)x1A10M12 | | | |
|---|---|---|---|---|---|---|---|
| Serum | Incubat ion days | EC50 (nM) | EC50 Ratio (%) | Serum | Incubati on days | EC50 (nM) | EC50 Ratio (%) |
| Human serum | 0 day | 0.228 | 100 | Human serum | 0 day | 0.211 | 100 |
| | 2 days | 0.365 | 62 | | 3 days | 0.220 | 96 |
| | 7 days | 0.420 | 54 | | 7 days | 0.236 | 89 |
| | 14 days | 0.457 | 50 | | 14 days | 0.233 | 91 |
| Monke y serum | 0 day | 0.240 | 100 | Monke y serum | 0 day | 0.208 | 100 |
| | 2 days | 0.360 | 67 | | 3 days | 0.238 | 87 |
| | 7 days | 0.427 | 56 | | 7 days | 0.257 | 81 |
| | 14 days | 0.419 | 57 | | 14 days | 0.273 | 76 |

As indicated in Table 15 and FIGS. 3a to 3d, the anti-ROR1/anti-4-1BB bispecific antibodies comprising BA6M11 maintained stability over a longer period in both human and monkey serum compared to the bispecific antibodies comprising the parental BA6 clone, demonstrating improved serum stability. Serum stability is a crucial property that significantly affects the efficacy of antibody drugs when administered in vivo, particularly in terms of binding affinity and specificity, and should be considered in antibody development and production. Thus, these results confirm that BA6M11 significantly contributes to enhancing the serum stability of the antibodies compared to BA6.

### 2.2.6. 4-1BB signal activation of anti-ROR1/Anti-4-1BB bispecific antibody (4-1BB reporter bioassay)

### 2.2.6.1. 4-1BB signal activation depending on ROR1 expression

To measure, compare, and analyze the 4-1BB signal activation of anti-ROR1/4-1BB bispecific antibodies, comprising the BA6 clone or BA6M11 clone, a 4-1BB NFκB luciferase reporter bioassay was conducted. The 4-1BB reporter bioassay used a human ROR1-positive cell line, the gastric cancer cell line AGS, and a hamster ovarian epithelial cell CHOK1 stably overexpressing human ROR1 (CHOK1-hROR1).

In this analysis, the GloResponse^{™} NFκB-luc2/4-1BB Jurkat cell line (Promega, cat# CS 196004), genetically engineered to stably express human 4-1BB and luciferase downstream of the response element, was used as the effector cell.

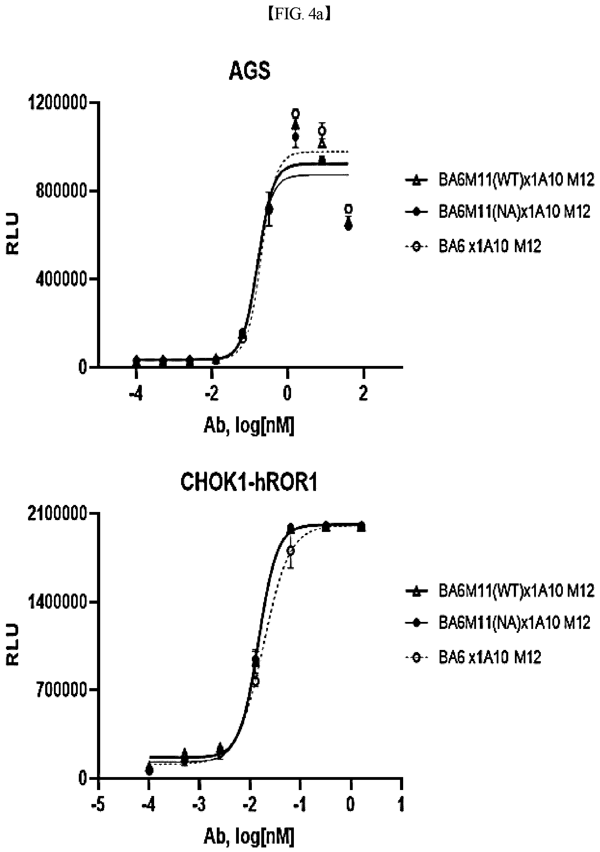

In brief, AGS (ROR1 positive gastric cancer cell line, ATCC, CRL-1739, 2.5 x 10⁴) or CHOK1-hROR1 (ROR1 overexpressing hamster ovarian epithelial cells, BPS Bioscience, 79609-H, 2.5 x 10⁴) were each dispensed into white 96-well assay plates containing 100 µL of culture medium (10% (v/v) FBS, RPMI 1640) and incubated overnight at 37°C in a 5% CO₂ humidified incubator. After overnight incubation, 100 µL of culture medium was removed, and 25µL of assay medium (1% (v/v) FBS, RPMI 1640) was dispensed onto the pre-attached target cells (AGS or CHOK1-hROR1) as previously described. 25µL of bispecific antibody (starting from 40 nM and serially diluted 5-fold) was added to the plate. 25µL of GLORESPONSE^{™} NFκB-luc2/4-1BB Jurkat cell suspension (Promega, CS196004) was dispensed into the plate to achieve 2.5 x 10⁴ cells per well and incubated for 6 hours at 37°C in a 5% CO₂ humidified incubator. During the incubation period, the BIO-GLO^{™} Luciferase Assay System (Promega, G7940) was reconstituted according to the manufacturer's instructions. After 6 hours of incubation, 75 µL of BIO-GLO^{™} Reagent was added to each well of the assay plate. Five 5 minutes later, luminescence was measured using a microplate reader. The four-parameter logistic curve was evaluated using GraphPad software. The results of the experiments using AGS and CHOK1-hROR1 cells are presented in Table 16 and FIG. 4a, respectively.

**TABLE 16**

| Clone Name | EC50 (nM) | |
|---|---|---|
| | AGS | CHOK1-ROR1 |
| BA6M11(WT)x1A10M12 | 0.162 | 0.015 |
| BA6M11(NA)x1A10M12 | 0.149 | 0.014 |
| BA6x1A10M12 | 0.200 | 0.018 |

As shown in Table 16 and FIG. 4a, the anti-ROR1/4-1BB bispecific antibody comprising BA6M11 exhibited lower EC50 (nM) values in ROR1-expressing cells compared to the bispecific antibody comprising the parental clone, BA6, indicating that the anti-ROR1/4-1BB bispecific antibody comprising BA6M11 induces a stronger 4-1BB signal activation in the presence of the ROR1 antigen. Specifically, the anti-ROR1/4-1BB bispecific antibody comprising BA6M11 showed superior 4-1BB signal activation effects in both ROR1 positive tumor cells (AGS) and human ROR1 overexpressing cells (CHOK1-hROR1), and these results were observed regardless of the presence or absence of the NA mutation.

The data demonstrates that the anti-ROR1/4-1BB bispecific antibody comprising BA6M11 specifically targets ROR1-expressing cancer cells.

To further validate that the activation of 4-1BB by BA6M11(NA)x1A10M12 and BA6M11(WT)x1A10M12 is dependent on the expression of ROR1, a mixture of parental CHOK1 cells and CHOK1 cells overexpressing human ROR1 (CHOK1-hROR1) was used to measure 4-1BB signal activation as described previously. For comparison, Urelumab (BMS-663513), an anti-4-1BB antibody, was used.

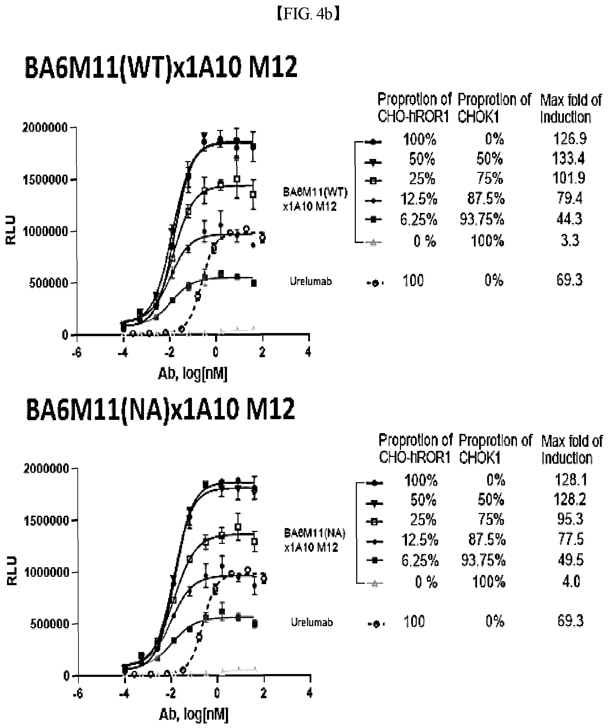

The results are presented in Table 17 and FIG. 4b

**TABLE 17**

| | |
|---|---|
| **Clone** | **EC50 (nM)** |
| **Name** | CHO-hROR1 100% |
| **BA6M11(WT)x1A10M12** | **0.016** |
| **BA6M11(NA)x1A10M12** | **0.015** |
| **Urelumab** | 0.235 |

As illustrated in FIG. 4b, both BA6M11(WT)x1A10M12 and BA6M11(NA)x1A10M12 demonstrated a higher Max fold induction as the proportion of CHOK1-ROR1 cells increased. This indicates that the more cells express ROR1, the more 4-1BB activation is induced. As shown in FIG. 4b and Table 17, BA6M11(WT)x1A10M12 and BA6M11(NA)x1A10M12 induced 4-1BB activation at similar levels.

### 2.2.6.2. 4-1BB signal activation of anti-ROR1/anti-4-1BB bispecific antibody depending on ROR1 expression level

The cell surface expression levels of ROR1 in various cancer cell lines were quantified using the QIFIKIT quantification kit (Dako, K0078) according to the manufacturer's recommendations. Briefly, the cancer cells used in the test were stained at saturation with an unlabeled anti-ROR1 mouse monoclonal antibody (BD, 564464) or a purified mouse IgG2b isotype control antibody (BD, 555740). After washing, the stained cells and calibration beads were simultaneously labeled with the same FITC-conjugated goat anti-mouse IgG secondary antibody. Both the calibration beads and the secondary antibody are included in the kit. The labeled cells and calibration beads were analyzed by flow cytometry. Linear regression analysis was performed using the MFI values of the calibration beads. Using the regression line derived from the linear regression analysis, the Antibody Binding Capacity (ABC) for both the anti-ROR1 antibody and the isotype control antibody was calculated, and the specific ABC (sABC) value was obtained by subtracting the value of the isotype control antibody from the ABC value of the anti-ROR1 antibody.

The results obtained are presented in Table 18.

**TABLE 18**

| Cell lines | | ROR1 sABC | MFI Fold |
|---|---|---|---|
| CHO-k1/ROR1 | BPS Bioscience, 79609-H | 765,378 | 836 |
| HCC1187 | KCLB, 9S1187 | 22,824 | 10.3 |
| NCI-N87 | ATCC, CRL-5822 | 21,512 | 31 |
| Kasumi-2 | DSMZ, ACC 526 | 10,156 | 26.8 |
| MDA-MB-231 | ATCC, HTB-26 | 8,902 | 10 |
| AGS | ATCC, CRL-1739 | 7,209 | 11.5 |
| 697 | DSMZ, ACC 42 | 6,081 | 22 |
| DLD1 | ATCC, CCL-221 | 5,327 | 9.6 |
| HCC1954 | ATCC, CRL-2338 | 1,874 | 1.9 |
| MCF-7 | ATCC, HTB-22 | 57 | 1 |
| CHO-k1 | ATCC, CRL-1469 | 4 | 1 |
| Jurkat | ATCC, TIB-152 | 1 | 1 |
| BT474 | ATCC, HTB-20 | 1 | 1 |
| MC38 | Kerafast, ENH204 | 0 | 1 |

The correlation between the obtained ROR1 expression levels (ROR1 sABC) and the 4-1BB-induced NF-kB signal by the antibody was tested.

The ROR1 levels measured in Table 18 were standardized to the ROR1 expression levels in NCI-N87, and the levels of 4-1BB activation induced by the bispecific antibodies BA6M11(WT)x1A10M12, BA6M11(NA)x1A10M12, and the control antibody Urelumab were determined as the maximum fold change compared to the control group in the 4-1BB NF-kB luciferase reporter assay. The method used was the same as described in Example 2.2.6.1. The shaded area represents the confidence interval for the linear fit.

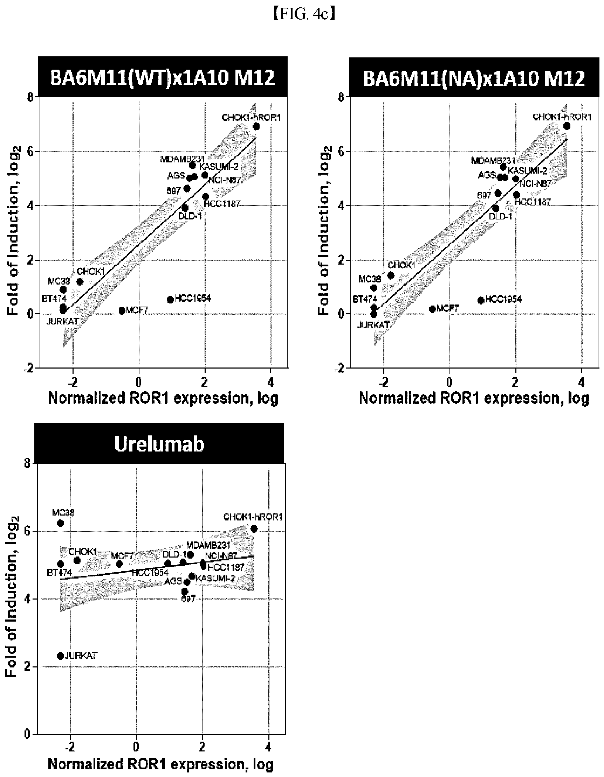

The results obtained are presented in FIG. 4c. As shown in FIG. 4c, compared to the control antibody, the 4-1BB activation induced by the anti-ROR1/4-1BB bispecific antibodies BA6M11(WT)x1A10M12 and BA6M11(NA)x1A10M12 showed a high correlation with ROR1 cell surface expression.

### 2.6.3. 4-1BB Signal activation according to FcγRI Engagement

4-1BB activation signals were evaluated using CHO-K1 cells (Promega) expressing FcγRI, FcγRIIb, or FcγRIIIa. The method used was the same as described in Example 2.2.6.1. Four-parameter logistic curve analysis was performed using GraphPad Prism^{®} software.

As shown in FIG. 4d, only CHO-K1 cells overexpressing FcγRI induced the 4-1BB signal of BA6M11(WT)x1A10M12. From this data, it was determined that for BA6M11(NA)x1A10M12, which has an NA mutation in the Fc, the binding to FcγRI is weakened and thus could not induce 4-1BB signal activation, however, BA6M11(WT)x1A10M12, without the mutation introduced in the Fc, was found to induce strong 4-1BB signal activation in the presence of FcγRI. CHO-K1 cells overexpressing FcγRIIb or FcγRIIIa, which have weaker binding to the Fc region compared to FcγRI, did not induce 4-1BB signal activation.

### 2.2.7. Induction of T cell immune response and cytokine secretion activity by anti-ROR1/anti-4-1BB bispecific antibody in ROR1-expressing cells (in vitro efficacy test)

### 2.2.7.1. PBMC activation test using CHOK1 cells artificially overexpressing ROR1

The 4-1BB signal activation ability of anti-ROR1/4-1BB bispecific antibodies comprising either the BA6 clone or BA6M11 clone to activate human peripheral blood mononuclear cells (PBMCs) was evaluated by co-culturing these bispecific antibodies with hamster ovarian epithelial cells (CHOK1-hROR1) stably expressing human ROR1. Interferon gamma (IFN-gamma), known as a cytokine, is primarily produced by natural killer (NK) cells, CD4+ helper T cells (Th1), and CD8+ cytotoxic T cells. Interferon gamma was measured to assess PBMC activation in this Example.

Briefly, a 96-well plate was coated with 5µg/mL of anti-human CD3 (Biolegend, 300438) (in PBS) at 37°C for 2 hours. After removing the anti-human CD3 solution, human PBMCs (Peripheral Blood Mononuclear Cell; CTL-UP-1) were added at a density of 3x10⁴ cells per well, and CHOK1-hROR1 cells were added at a density of 3x10⁴ cells per well. The test antibody (starting at 50nM, diluted 5-fold) was added to the wells of the plate. The plate was then incubated for 72 hours at 37°C in a 5% CO₂ humidification incubator. After incubation, the concentration of interferon gamma (IFN-gamma) released into the supernatant was measured using the Human IFN-gamma Duoset ELISA Kit (R&D Systems, DY285) according to the manufacturer's protocol. Four-parameter logistic curve was evaluated using GraphPad software.

As shown in FIG. 5a, the anti-ROR1/4-1BB bispecific antibody BA6M11(NA)x1A10M12 induced the release of IFN-gamma at lower concentrations compared to BA6x1A10M12, and at the same concentration, it induced a higher release of IFN-gamma, thus, the bispecific antibody comprising BA6M11 demonstrated superior PBMC activation efficacy compared to the parental clone, BA6. Furthermore, bispecific antibodies comprising BA6M11, such as BA6M11(NA)x1A10M12 and BA6M11(NA)x1A10M12, induced the release of IFN-gamma at similar levels.

### 2.2.7.2. PBMC activation test using gastric cancer cell line expressing ROR1

NCI-N87, a gastric cancer cell line expressing human ROR1, was used to evaluate whether the 4-1BB signal activation activity of the bispecific antibodies of the present disclosure can activate human peripheral blood mononuclear cells (PBMCs) when co-cultured with these human ROR1 expressing gastric cancer cells. In brief, a 96-well plate was coated with 5µg/mL of anti-human CD3 (Biolegend, 300438) dissolved in PBS at 37°C for 2 hours. Subsequently, human PBMCs (CTL) and NCI-N87 (ATCC) were added to the plate. Four-fold diluted solutions of each test antibody (starting at 20nM) were added to each well and incubated for 72 hours. After 72 hours of incubation, the concentration of IFN-gamma in the supernatant was measured using the Human IFN-gamma Duoset ELISA Kit (R&D System, DY285B). The viability of NCI-N87 was measured with the cell counting kit-8 (Dojindo, CK04-20).

The results obtained are shown in FIGS. 5b and 5c. As indicated in FIGS. 5b and 5c, the anti-ROR1/4-1BB bispecific antibody comprising BA6M11, compared to the control antibody (urelumab), had a lower EC50 (nM) value, induced IFN-gamma secretion at lower concentrations, and induced cell death in NCI-N87 at lower concentrations. This data indicates a superior PBMC activation capability in the presence of the human ROR1 antigen compared to the control antibody. Furthermore, the anti-ROR1/4-1BB bispecific antibody comprising BA6M11, in both WT and NA forms, demonstrated superior PBMC activation capability compared to the combination of BA6M11 alone and 1A10M12 alone (BA6M11 + 1A10M12). From this observation, it was confirmed that the anti-ROR1/4-1BB bispecific antibody of the present disclosure exhibits a superior synergistic effect compared to the simple combination of monospecific antibodies.

### 2.3. In vivo efficacy of anti-ROR1/anti-4-1BB bispecific antibody

### 2.3.1. Anti-tumor efficacy

In vivo anti-tumor efficacy of the anti-ROR1/anti-4-1BB bispecific antibodies BA6M11(WT)x1A10M12 and BA6M11(NA)x1A10M12 was evaluated using human 4-1BB knock-in mice (Biocytogen). Briefly, MC38 colon cancer cells, stably expressing human ROR1 (hROR1-expressing MC38 cells, 5x10⁵, Biocytogen), were subcutaneously injected into the right flank of the mice. The tumor-bearing humanized mice were divided into three groups based on tumor size (average tumor size: about 89 mm³, *n* = 5 mice/group). After grouping, on day 0, a single intravenous dose of human IgG1 antibody (hIgG1 antibody, 3 mg/kg), BA6M11(WT)x1A10M12 (4 mg/kg), or 1(NA)x1A10M12 (4 mg/kg) was administered. Tumor size was measured twice a week using a digital caliper, and tumor volume was calculated using the formula: V(mm³) = a*b²/2 (where a and b represent the long and short dimensions of the tumor, respectively). The tumor growth inhibition (TGI) percentage was calculated based on tumor volume using the formula: TGI% (Day i) =[1-(T_{Day(i)}-T_{Day0})/(V_{Day(i)}-V _{Day0})] × 100% (T: tumor volume of the treated group; V: tumor volume of the control group).

The results of the anti-tumor efficacy are presented in FIG. 6a and Table 19.

**TABLE 19**

| Test articles | Dosage | Dosing Time | TGI (%) (Day 23) | Survival rate (%) (Day 44) |
|---|---|---|---|---|
| hIgG1 | 3 mg/kg | 1 | 0 | 0 |
| BA6M11(WT)x1A10M12 | 4 mg/kg | 1 | 98.6 | 100.0 |
| BA6M11(NA)x1A10M12 | 4 mg/kg | 1 | 69.9 | 80.0 |

As shown in FIG. 6a and Table 19, BA6M11(WT)x1A10M12 and BA6M11(NA)x1A10M12 demonstrated significant tumor suppression effects. On the 23^{rd} day after grouping, the TGI for the groups treated with BA6M11(WT)x1A10M12 and BA6M11(NA)x1A10M12 were 98.6% and 69.9%, respectively, and on the 44^{th} day, the survival rates for the BA6M11(WT)x1A10M12 and BA6M11(NA)x1A10M12 treated groups were 100% and 80%, respectively, while the hIgG1 control group had no survivors. This confirms that both bispecific antibodies, BA6M11(WT)x1A10M12 and BA6M11(NA)x1A10M12, exhibited superior anti-tumor efficacy and high survival rates even with a single administration.

### 2.3.2. Dose-dependent anti-tumor efficacy

To confirm the dose-dependent effects of the bispecific antibody BA6M11(WT)x1A10M12, in vivo anti-tumor efficacy was evaluated in human ROR1-expressing MC38-bearing mice. Specifically, tumor cells (hROR1-expressing MC38 cells, 5×10⁵ cells) were subcutaneously injected into the right flank of human 4-1BB knock-in mice to prepare the human ROR1-expressing MC38-bearing mice. These prepared mice were randomly grouped based on a tumor volume (about 80mm³). On day 0 of grouping, a single intravenous injection of hIgG1 antibody (10 mg/kg) or BAOM11(WT)x1A10M12 (0.4 mg/kg, 1 mg/kg, or 4 mg/kg) was administered. The anti-tumor efficacy was measured referring to Example 2.3.1.

As shown in FIG. 6b, BA6M11(WT)x1A10M12 exhibited dose-dependent anti-tumor efficacy. Additionally, treatment with BA6M11(WT)x1A10M12 increased the number of mice with complete tumor regression (0% in the hIgG1 treated group vs. 50% in the BA6M11(WT)x1A10M12 4 mg/kg treated group), and the mice with complete regression survived for more than 100 days without signs of tumor recurrence after the initial tumor implantation.

### 2.3.3. Memory response upon serial tumor re-challenge

To evaluate the long-lasting memory response, tumor re-challenge was performed in mice that had been cured of their tumors by BA6M11(WT)x1A10M12. The tumor re-challenge was performed 100 days after the last dose of the antibody in cured mice. Specifically, hROR1-expressing MC38 cells (2.5x10⁶ cells, 5 times the initial inoculation) were subcutaneously injected into both the cured mice and control naive mice (mice that had not undergone tumor inoculation and antibody treatment) for a secondary inoculation. 59 days after the secondary inoculation, surviving mice were re-challenged with the tumor (2.5×10⁶ cells, tertiary inoculation). The anti-tumor efficacy was measured referring to Example 2.3.1.

As shown in FIG. 6c, mice previously treated with the bispecific antibody demonstrated resistance to repeated secondary and tertiary tumor re-challenges, while control naive mice did not exhibit tumor reduction. This confirmed that treatment with BA6M11(WT)x1A10M12 induces a sustained anticancer memory response.

### 2.3.4. In vivo immune modulation

To understand the effects of the bispecific antibody on immune cells, tumor and peripheral tissues (blood and liver) were analyzed using flow cytometry.

In particular, human ROR1-expressing MC38 tumor-bearing humanized mice used in the previous example were randomly grouped based on a tumor volume of about 100mm³. On days 0 and 4 after grouping, mice were intraperitoneally administered with hIgG1 antibody (7.5 mg/kg), urelumab (7.5 mg/kg), or the bispecific antibody BA6M11(WT)x1A10M12 (10 mg/kg). On day 7 after grouping, immune cells were analyzed using flow cytometry. In the tumor and peripheral blood, the proportions of leukocytes (CD45⁺) and Treg cells (CD45⁺CD3⁺CD4⁺FOXP3⁺) and the expression of 4-1BB on T cells (CD45⁺CD3⁺) were analyzed. In the liver, the proportions of T cells, CD8⁺ T cells (CD45⁺CD3⁺CD8⁺), and macrophages (CD45⁺CD11b⁺F4/80⁺) were analyzed. The results are shown in FIG. 6d (A: Tumor; B: Peripheral Blood; C: Liver).

As illustrated in FIG. 6d, a significant increase in tumor-infiltrating leukocytes (CD45⁺/Live) was observed in the BA6M11(WT)x1A10M12 treated group. Additionally, unlike urelumab, BA6M11(WT)x1A10M12 specifically induced the depletion of immunosuppressive Treg cells (Treg/CD45+) in the tumor, not in the systemic circulation. The expression of 4-1BB on T cells (4-1BB+/CD3+) was measured at higher levels in the tumor compared to the blood, and its levels were increased by treatment with BA6M11(WT)x1A10M12. These results confirm the tumor-specific T cell activation effects of the bispecific antibody of this application. Furthermore, while urelumab's systemic activation was accompanied by a massive infiltration of immune cells into the liver, the bispecific antibody of this application did not show an increase in T cells and macrophages in the liver. These results confirmed that the bispecific antibody of the present application exerts a strong anti-tumor effect through tumor-specific immune modulation while minimizing the risk of peripheral toxicity.

### 2.3.5. Additional demonstration of tumor-specific T cell activation by anti-ROR1/anti-4-1BB bispecific antibody

To prove that the bispecific antibody of the present application specifically activates T cells only in cells expressing ROR1 (e.g., tumor cells) and does not act on normal cells and tissues that do not express ROR1, the cytokine secretion activity induced by the anti-ROR1/anti-4-1BB bispecific antibody was verified using human PBMCs in the absence of ROR1-expressing cells.

In particular, five types of human PBMCs (CTL, CTL-UP1) were thawed and then seeded in a 96-well plate at a concentration of 2x10⁵/well in 10% RPMI medium. As a positive control, anti-human CD3 antibody (Biolegend, 317326) was diluted in RPMI with 10% FBS to concentrations of 0.01µg, 0.1µg, 1µg, 25µg, and 50µg and added to each well. For the test group, 1µg, 25µg, and 50µg of the bispecific antibody (BA6M11(WT)x1A10M12) were added to each well.

The plate was incubated in a 37°C, 5% CO2 incubator for 48 hours. After 48 hours, the plate was centrifuged to separate the supernatant. ELISA analysis was performed according to the manufacturer's protocol to measure the concentrations of IFN-gamma (R&D System, DY285B), IL-2 (R&D System, DY202), IL-6 (R&D System, DY206), and TNF-alpha (R&D System, DY210) in the culture supernatant. The results are presented in FIG. 6e.

As shown in FIG. 6e, compared to the positive control, BA6M11(WT)x1A10M12 did not induce cytokine release in the absence of ROR1. This means that the bispecific antibody of the present application activates T cells only in tumor tissues expressing ROR1 and not in normal cells and tissues, suggesting a reduced risk of unexpected toxicity upon administration in the body.

### EXAMPLE 3. ROR1xB7-H3 Bispecific Antibody and Antibody-Drug Conjugate (ADC) Comprising Same

### 3.1. Design and construction of improved anti-ROR1/anti-B7-H3 bispecific antibody

Based on the anti-ROR1 antibody clone BA6M11 selected in Example 1 and the anti-B7-H3 antibody clone B5 disclosed in the international publication WO2019-226017 A1, an anti-ROR1/anti-B7-H3 bispecific antibody was manufactured that comprises both an anti-ROR1 antibody and an anti-B7-H3 antibody.

This bispecific antibody was designed in an IgG-scFv fusion format, where the scFv antibody fragment against one of the antigens (either ROR1 or B7-H3) is fused to the C-terminus (heavy chain C-terminus) of the full-length IgG antibody against the other antigen. In this Example, a bispecific antibody comprising the anti-ROR1 IgG antibody and the B5 scFv linked to the C terminus of each of the heavy chains of the anti-ROR1 IgG antibody was constructed.

To construct the anti-ROR1/anti-B7-H3 bispecific antibody, a DNA segment 1 comprising the nucleotide sequence coding for the heavy chain of the anti-ROR1 IgG antibody (BA6M11) was inserted into pcDNA 3.4 (Invitrogen, A14697; Plasmid 1), and a DNA segment 2 comprising the nucleotide sequence coding for the light chain of the IgG antibody in the anti-ROR1/anti-B7-H3 bispecific antibody was inserted into pcDNA 3.4 (Invitrogen, A14697; Plasmid 2). Subsequently, a DNA segment 3 coding for the scFv was fused to a part of DNA segment 1 corresponding to the C-terminus of the Fc region of the IgG antibody in Plasmid 1 using DNA segment 4 coding for a peptide linker composed of 20 amino acids length (GGGGS)3 (SEQ ID NO: 120), to construct a vector for the expression of the bispecific antibody.

Additionally, the anti-B7-H3 scFv (named B5) is in a form where the C-terminus of the light chain variable region (VL) and the C-terminus of the heavy chain variable region (VH) are each fused through a linker ((GGGGS)4) (SEQ ID NO: 114) with VL103-VH44 (VL103: VL with a G(Gly)→C(Cys) mutation introduced at position 103; VH44: VH with a G→C mutation introduced at position 44). These mutations stabilize the scFv and enable the formation of disulfide bridges.

The amino acid sequences of the thus prepared bispecific antibody (BA6M11xB5) are listed in Table 20, below.

**TABLE 20**

| | Amino acid sequence (N→C) | SEQ ID NO: # |
|---|---|---|
| Light chain | QSVLTQPPSASGTPGQRVTISCSGSSSNIGSNDVSWYQ | 111 |
| BA6M11 | QLPGTAPKLLIYYENNRPSGVPDRFSGSKSGTSASLAIS GLRSEDEADYYCGAWDDSLSGYVFGGGTKLTVLGQP KAAPSVTLFPPSSEELQANKATLVCLISDFYPGAVTVA WKADSSPVKAGVETTTPSKQSNNKYAASSYLSLTPEQ WKSHRSYSCQVTHEGSTVEKTVAPAECS | |

| Heavy chain BA6M11(WT)xB5 | | |
|---|---|---|
| Heavy chain BA6M11( WT) | EVQLLESGGGLVQPGGSLRLSCAASGFTFSNYDMSWV RQAPGKGLEWVSAIYHSGSSKYYADSVKGRFTISRDN SKNTLYLQMNSLRAEDTAVYYCARGGSGAWDTGFD YWGQGTLVTVSSASTKGPSVFPLAPSSKSTSGGTAAL GCLVKDYFPEPVTVSWNSGALTSGVHTFPAVLQSSGL YSLSSVVTVPSSSLGTQTYICNVNHKPSNTKVDKKVEP KSCDKTHTCPPCPAPELLGGPSVFLFPPKPKDTLMISRT PEVTCVVVDVSHEDPEVKFNWYVDGVEVHNAKTKPR EEQYNSTYRVVSVLTVLHQDWLNGKEYKCKVSNKAL PAPIEKTISKAKGQPREPQVYTLPPSREEMTKNQVSLTC LVKGFYPSDIAVEWESNGQPENNYKTTPPVLDSDGSFF LYSKLTVDKSRWQQGNVFSCSVMHEALHNHYTQKSL SLSPGK | 112 |
| Linker1 | GGGGSGGGGSGGGGS | 120 |
| B5 (VL) | QSVLTQPPSASGTPGQRVTISCSGSSSNIGSNAVSWYQ QLPGTAPKLLIYYNSHRPSGVPDRFSGSKSGTSASLAIS GLRSEDEADYYCGSWDASLNAYVFGCGTKLTVL | 98 |
| Linker2 | GGGGSGGGGSGGGGSGGGGS | 114 |
| B5 (VH) | EVQLLESGGGLVQPGGSLRLSCAASGFTFSDYAMSWV RQAPGKCLEWVSSISSGSGSIYYADSVKGRFTISRDNS KNTLYLQMNSLRAEDTAVYYCAKNLIPLDYWGQGTL VTVSS | 86 |
| Full-length | EVQLLESGGGLVQPGGSLRLSCAASGFTFSNYDMSWV RQAPGKGLEWVSAIYHSGSSKYYADSVKGRFTISRDN SKNTLYLQMNSLRAEDTAVYYCARGGSGAWDTGFD YWGQGTLVTVSSASTKGPSVFPLAPSSKSTSGGTAAL GCLVKDYFPEPVTVSWNSGALTSGVHTFPAVLQSSGL YSLSSVVTVPSSSLGTQTYICNVNHKPSNTKVDKKVEP | 121 |
| | KSCDKTHTCPPCPAPELLGGPSVFLFPPKPKDTLMISRT PEVTCVVVDVSHEDPEVKFNWYVDGVEVHNAKTKPR EEQYNSTYRVVSVLTVLHQDWLNGKEYKCKVSNKAL PAPIEKTISKAKGQPREPQVYTLPPSREEMTKNQVSLTC LVKGFYPSDIAVEWESNGQPENNYKTTPPVLDSDGSFF LYSKLTVDKSRWQQGNVFSCSVMHEALHNHYTQKSL SLSPGKGGGGSGGGGSGGGGSQSVLTQPPSASGTPGQ RVTISCSGSSSNIGSNAVSWYQQLPGTAPKLLIYYNSH RPSGVPDRFSGSKSGTSASLAISGLRSEDEADYYCGSW DASLNAYVFGCGTKLTVLGGGGSGGGGSGGGGSGGG GSEVQLLESGGGLVQPGGSLRLSCAASGFTFSDYAMS WVRQAPGKCLEWVSSISSGSGSIYYADSVKGRFTISRD NSKNTLYLQMNSLRAEDTAVYYCAKNLIPLDYWGQG TLVTVSS | |

| | | |
|---|---|---|
| (Linker1: linkage between BA6M11(IgG) and B5(scFv) (C-terminus of heavy chain in BA6M11 linked to N-terminus of VL in B5); Linker2: linkage between VL and VH in B5 (scFv) (C-terminus of VL linked to N-terminus of VH) | | |

Coding nucleic acid sequences of the bispecific antibody (BA6M11xB5) are as follows:
[Heavy chain_BA6M11xB5] (SEQ ID NO: 122) 
gaagttcagctgctggaatctggcggcggattggttcaacctggcggctctctgagactgtcttgtgccgcttct
ggcttcaccttctccaactacgacatgtcctgggtccgacaggctcccggcaaaggattggaatgggtgtccgcc
atctaccactccggctcctctaagtactacgccgactccgtgaagggcagattcaccatctctcgggacaactcc
aagaacaccctgtacctgcagatgaactccctgagagccgaggacaccgccgtgtactattgtgccagaggcgga
tctggcgcctgggacaccggctttgattattggggacagggcaccctggtgaccgtgtcctccgcctccaccaag
ggcccctccgtgttccccctggccccctcctccaagtccacctccggcggcaccgccgccctgggctgcctggtg
aaggactacttccccgagcccgtgaccgtgtcctggaactccggcgccctgacctccggcgtgcacaccttcccc
gccgtgctgcagtcctccggcctgtactccctgtcctccgtcgtgaccgtgccctcctcctccctgggcacccag
acctacatctgcaacgtgaaccacaagccctccaacaccaaggtggacaagaaggtggagcccaagtcctgcgac
aagacccacacctgccctccctgccccgcccccgagctgctgggcggcccctccgtgttcctgttccctcctaag
cccaaggacaccctgatgatctcccggacccccgaggtgacttgcgtggtggtggacgtgtcccacgaggacccc
gaggtgaagttcaactggtacgtggacggcgtggaggtgcacaacgccaagaccaagccccgggaggagcagtac
aactccacctaccgggtggtgtccgtgctgaccgtgctgcaccaggactggctgaacggcaaggagtacaagtgc
aaggtgtccaacaaggccctgcccgcccccatcgagaagaccatctccaaggccaagggccagccccgggagccc
caggtgtacaccctgcctcctagcagagaagagatgaccaagaaccaggtgtccctgacctgcctggtcaagggc
ttctacccttccgatatcgccgtggaatgggagtccaatggccagcctgagaacaactacaagaccacacctcct
gtgctggactccgacggctcattcttcctgtactccaagctgaccgtggacaagtccagatggcagcagggcaac
gtgttctcctgctccgtgatgcacgaggccctgcacaatcactacacccagaagtccctgtctctgtcccctgga
aaaggcggcggaggatctggcggaggtggaagcggaggcggtggatctcagtctgttctgacccagcctccttcc
gcttctggcacacctggacagagagtgaccatctcttgctccggctcctcctccaacatcggctctaatgccgtg
tcctggtatcagcagctgcctggcacagctcccaaactgctgatctactacaactcccaccggccttccggcgtg
cccgatagattttccggctctaagtccggcacctctgccagcctggctatctctggcctgagatctgaggacgag
gccgactactactgcggctcttgggatgcctctctgaacgcctacgtgttcggctgtggcacaaagctgacagtg
cttggaggtggtggtagtggtggtggcggttcaggtggcggaggaagcggcggaggcggatctgaagttcagctg
ttggaatctggcggcggactggttcaacctggcggatctctgagactgtcttgtgccgcctctggcttcaccttc
tccgactacgctatgtcttgggtccgacaggcccctggcaagtgtctggaatgggtttcctccatctcctccggc
agcggctctatctactacgccgactctgtgaagggcagattcaccatcagccgggacaactccaagaacaccctg
tacctgcagatgaactccctgagagccgaggacaccgccgtgtactactgtgccaagaatctgatccctctggac
tactggggccagggcacactggttacagtgtcctct
[Light chain_BA6M11]( SEQ ID NO: 123)

cagtccgtgctgacccagcccccctccgcctccggcacccccggccagcgggtgaccatctcctgctccggctcc
tcctccaacatcggctccaacgacgtgtcctggtaccagcagctgcccggcaccgcccccaagctgctgatctac
tacgaaaacaaccggccctccggcgtgcccgaccggttctccggctccaagtccggcacctccgcctccctggcc
atctccggcctgcggtccgaggacgaggccgactactactgcggcgcctgggacgactccctgtccggctacgtg
ttcggcggcggcaccaagctgaccgtgctgggccagcccaaggccgccccctccgtgaccctgttccccccctcc
tccgaggagctgcaggccaacaaggccaccctggtgtgcctgatctccgacttctaccccggcgccgtgaccgtg
gcctggaaggccgactcctcccccgtgaaggccggcgtggagaccaccaccccctccaagcagtccaacaacaag
tacgccgcctcctcctacctgtccctgacccccgagcagtggaagtcccaccggtcctactcctgccaggtgacc
cacgagggctccaccgtggagaagaccgtggcccccgccgagtgctcc

The coding DNAs of the cloned bispecific antibody were transiently expressed using the ExpiCHO system (Thermo Fisher) according to the manufacturer's protocol. The culture medium comprising expressed antibody was clarified using centrifugation and filtration to remove suspensions, and the antibody was purified using affinity chromatography with MabSelect Sure resin and size exclusion chromatography with Superdex200 resin. The purity of the purified antibody was analyzed using HPLC with a TSKgel SuperSW3000 column, and the purity was found to be over 95%.

### 3.2. Stability evaluation of anti-ROR1B7-H3 bispecific antibody comprising improved anti-ROR1 clone

### (1) Binding specificity analysis of anti-ROR1B7-H3 bispecific antibody under stressed conditions (ELISA)

As a control group, the BA6xB5 bispecific antibody which comprised the parental clone anti-ROR1 antibody BA6 without removed PTM hotspot, and for the experimental group, the BA6M11xB5 bispecific antibody was utilized, and after storing both samples at 4°C (normal conditions) and 40°C (stress conditions) for two weeks, the antibody-antigen binding affinity to human ROR1 and human B7H3 recombinant proteins was evaluated using an ELISA-based solution binding assay. Specifically, 96-well microtiter plates (Nunc-Immuno Plates, NUNC) were coated for 16 hours at 4°C with 1 µg/ml concentration of human ROR1 protein (ROR1-His; Sino Biological, Catalog # 13968-H08H) and human B7-H3 protein (B7H3-His; Sino Biological, Catalog # 11188-H08H) in PBS solution, and then the plates were then blocked with 1% (v/v) BSA (bovine serum albumin) at 37°C for 2 hours to prevent non-specific binding.

Afterwards, the BA6xB5 and BA6M11xB5 bispecific antibodies stored for two weeks were added to the microtiter plates at the concentrations listed in FIGS. 7a-7d, and their binding ability was analyzed by ELISA as follows. Specifically, after incubating at 37°C for 2 hours, the plates were washed five times with PBS containing 0.05% (v/v) Tween 20, and then HRP-conjugated Fc polyclonal antibody reagent (Pierce, Catalog # 31413) was diluted at a 1:30,000 ratio in 1% (v/v) BSA and added to the washed microtiter plates, incubated for 1 hour at 37°C to allow the reaction, and then used to detect the bound bispecific antibodies. The reaction was developed using TMB (tetramethylbenzidine, Sigma, T0440). The enzymatic reaction was stopped with 0.5mol/L sulfuric acid, and the absorbance was measured at 450nm and 650nm using a microplate reader (Molecular Devices).

The results obtained are presented in FIGS. 7a-7d. As can be seen from FIGS. 7a-7d, upon evaluating the antigen-antibody binding capacity (ELISA; Protein Binding) using human ROR1 protein and human B7-H3 protein, it was confirmed that there was no significant difference in binding ability between the samples stored at 4°C and 40°C for two weeks. These results indicate that BA6M11xB5 maintains its potency compared to BA6xB5 while showing improved physicochemical property in terms of its developability. Ultimately, it was confirmed that the BA6M11 clone not only shows binding comparable to WT but also has clearly improved developability through PTM engineering.

### (2) Developability analysis of anti-ROR1B7-H3 bispecific antibody

Selection was made of the BA6M11 clone, which were cleared of PTM liability through PTM engineering from BA6, and both BA6M11xB5 bispecific antibody comprising BA6M11 and BA6xB5 bispecific antibody were manufactured to check for increased stability under stressed conditions.

More specifically, after storing the BA6xB5 bispecific antibody and BA6M11xB5 bispecific antibody at 4°C (normal conditions) and 40°C (stressed conditions) for two weeks, analyses such as SE-HPLC, icIEF, and LC/MS were performed on each sample to check for purity and impurity content. Additionally, to determine whether changes in the substance content also affected efficacy, antigen-antibody binding ability analysis (ELISA; refer to Example 3.2(1)) was conducted.

The results obtained are presented in Table 21, below.

**TABLE 21**

| **Content** | **Method** | | **Acceptance Criteria** | **BA6M11xB 5** | | **BA6xB5** | |
|---|---|---|---|---|---|---|---|
| | | | | **4 °C** | **40 °C** | **4 °C** | **40 °C** |
| **Purity (%)** | **SEC** | | **ΔPurity% ≤3.0%** | 99.8 | 99.2 | 99.8 | 99.2 |
| | | | | 0.6 | | 0.6 | |
| **Charge Heterogeneit y** | **icIEF** | **Acidic** | **Information only** | 49.1 | 64.9 | 51.5 | 45.6 |
| | | **Main** | | 43.9 | 29.0 | 31.8 | 23.1 |
| | | **Basic** | | 7.0 | 6.1 | 16.6 | 31.2 |
| | | **ΔMain %** | | 14.9 | | 8.7 | |
| **Protein Binding** | **ELIS A** | **SACE (EC50 to Human ROR1), nM)** | **70%≤Ratio(EC50)≤130 %, similar curve fit** | 0.152 | 0.134 | 0.14 1 | 0.180 |
| | | | | 113 | | 78 | |
| | | **SACE (EC50 to Human B7-H3), nM)** | **70%≤Ratio(EC50)≤130 %, similar curve fit** | 0.232 | 0.190 | 0.17 3 | 0.186 |
| | | | | 122 | | 93 | |
| **PTM** | **LC/MS** | | **Report result ΔSuccinimide% in Fd** | *N/A | *N/A | 9.8 | 12.6 |
| | | | | N/A | | 2.8 | |
| | | | **Report result (ΔSuccinimide% in LC)** | *N/A | *N/A | 16.8 | 45.6 |
| | | | | *N/A | | 28.8 | |

| | | | | | | | |
|---|---|---|---|---|---|---|---|
| (* N/A : Not applicable) | | | | | | | |

As indicated in Table 21, the BA6M11xB5 clone maintained a high level of antibody purity when stored at both 4°C and 40°C, with no significant difference compared to BA6xB5. In terms of charge variants, the BA6M11xB5 clone showed an improvement over the BA6xB5 clone, with a reduction in the basic variant peak related to the formation of succinimide, and analysis using LC-MS also revealed that, compared to BA6xB5, BA6M11xB5 did not show an increase in succinimide in the Fd and LC under 40°C stressed conditions, and fragments that appeared to be cleaved within the Fd region were not formed. This reduction in charge variants implies an increase in the stability of the antibody quality and the reliability of the purification process, as discussed in Example 1.3. It also suggests that when administered to humans, the bispecific antibody is expected to maintain low immunogenicity and valency.

### 3.3. Binding specificity analysis of anti-ROR1B7-H3 bispecific antibody to cell surface expressed ROR1 and B7-H3 (FACS)

To evaluate the cell-binding ability of the anti-ROR1/B7-H3 bispecific antibody, cells expressing both ROR1 and B7-H3 were used to compare the cell-binding ability of the bispecific antibody with that of the monospecific antibodies.

As a cell line for the comparison of cell-binding ability, CHO-huROR1-huB7-H3 cell line (obtained by transfecting the ROR1-CHO recombinant cell line (BPS Bioscience) with the human B7-H3 gene (Origene) and selecting for the desired gene-inserted single cells using G418), which stably transfected the human ROR1 and human B7-H3 gene to artificially overexpress both human ROR1 and human B7-H3 proteins were used, and analysis was performed using a flow cytometer (LSRFortessa X-20, BD Biosciences). Specifically, after dissociating and washing the overexpressing cell line with PBS, the cell number was counted, and the cells were seeded at a density of 2x10⁵ cells per well into a V-bottom 96-well plate (96 Well Plate-RV, Bioneer, 910D09). Antibodies diluted 4-fold starting from 100 nM in 1% BSA solution were added at 100 µL per well to the centrifuged cells and incubated at 4°C for 1 hour. After 1 hour, the cells were washed twice with the same buffer and then incubated with FITC-labeled Fc-specific antibody (Goat anti-human IgG-FITC antibody produced in goat, Sigma, F9512) diluted 500 times in 1% BSA at 100 µL per well at 4°C for 1 hour. After the reaction, the cells were washed twice with the same buffer, resuspended in 100 µL of PBS, and analyzed using a FACS instrument. A total of 10,000 cells were detected per measurement, and the data was analyzed using FlowJo software. The Fold change values on the graphs were represented by dividing the MFI (Mean fluorescence intensity) of the experimental group treated with antibody by the MFI of the control group treated with the secondary antibody only.

As shown in FIG. 8, the anti-ROR1/B7-H3 bispecific antibody, which has more antigen-binding sites, showed higher binding affinity to the cell line expressing both ROR1 and B7-H3 compared to monospecific antibodies.

These results confirm that the anti-ROR1/B7-H3 bispecific antibodies provided herein exhibit superior binding affinity by targeting two antigens simultaneously. Such enhanced binding ability demonstrates excellent selectivity for cells expressing two targets (antigens) simultaneously compared to cells expressing a single target (antigen). This feature aligns with the goals of reducing toxicity and increasing efficacy through selectivity, which are important elements in the design of cancer treatment methods.

### 3.4. Comparison of cellular internalization between monospecific antibody and bispecific antibody

To compare the cellular internalization rates of the anti-ROR1/B7-H3 bispecific antibody, cells expressing both ROR1 and B7-H3 were used to evaluate the internalization of the bispecific antibody. The experiment was conducted using the Incucyte^{®} FabFluor-pH Red antibody labeling reagent (Sartorius, 4722), which fluoresces at low pH values. CHO-huROR1-huB7-H3 cells, the same cell line used in Example 3.3, were plated at a density of 1 x 10⁴ cells per well in a 96-well plate (96-well Clear Flat Bottom TC-treated Culture Microplate, Falcon, 353072) and cultured for 24 hours. The antibody and Incucyte^{®} FabFluor-pH Red antibody labeling reagent were mixed at a 1:1 molar ratio and incubated at 37°C for 15 minutes before being added to the plate with cells. The plate was then placed in the IncuCyte^{®} real-time cell analysis system (IncuCyte S3, Sartorius), and phase-contrast and fluorescence were analyzed at 1-hour intervals through the software. As shown in FIG. 9, an increase in fluorescence signal over time was observed, confirming the internalization process of the antibody entering the cell and reaching the lysosome.

As shown in FIG. 9, the anti-ROR1/B7-H3 bispecific antibody, which has more antigen-binding sites, increased the fluorescence signal more rapidly in the cell line expressing both ROR1 and B7-H3 compared to monospecific antibodies, conforming that the bispecific antibody is internalized into cells more efficiently than monospecific antibodies due to its ability to target two antigens simultaneously.

These results indicate that the anti-ROR1/B7-H3 bispecific antibodies provided herein promote efficient cellular internalization by simultaneously targeting two antigens.

From the described outcomes, it is anticipated that developing the bispecific antibody into an ADC (Antibody-Drug Conjugate) could enhance the efficacy of the ADC selectively in cells that express both targets (antigens). This characteristic was further explored and confirmed in subsequent Examples.

### 3.5. Manufacturing of anti-ROR1/anti-B7-H3 bispecific antibody ADC

### (1) Manufacturing of bispecific antibody ADC

For the conjugation of the drug to the amino acid position 205 of the antibody light chain, the valine (Valine) at Kabat position 205 in the constant region of the antibody light chain was mutated to cysteine (Cysteine) (V205C), and dithiothreitol (DTT) or a similar reducing agent was reacted to generate a thiol group at antibody light chain V205C, and the antibody was conjugated to the drug through thiosuccinimide formation via Michael addition. Specifically, the antibody was prepared to a concentration of over 5mg/ml through ultrafiltration/diafiltration (UF/DF), adding 1M tris(hydroxymethyl)aminomethane (Tris-HCl), pH8.8, and 500mM ethylenediaminetetraacetic acid (EDTA), to adjust the final concentration of the antibody to 5mg/ml, 75mM Tris-HCl, and 2mM EDTA. 100mM dithiothreitol (DTT) was added to the prepared antibody at an antibody: DTT molar ratio of 1:20 and reacted for 16.5 hours at 25°C to detach the free cysteine linked through disulfide bonds at cysteine 205 of the antibody light chain. This process, referred to as "decapping," was followed by separation of the decapped antibody using cation exchange chromatography (CEX). The reaction product was eluted with SPHP-B buffer [50mM Tris(hydroxymethyl)aminomethane (Tris-HCl), pH7.5, 0.5M Sodium chloride] after equilibrating on a Hitrap SPHP column (GE Healthcare) with SPHP-A buffer [10mM succinate, pH5.0]. For the recombination of the decapped antibody, the antibody to be oxidized was prepared in 75mM Tris(hydroxymethyl)aminomethane (pH7.5) using 1M Tris-HCl, and dehydroascorbic acid (DHAA, oxidized vitamin C) was added at an antibody: DHAA ratio of 1:20, and reoxidized for 2 hours at 25°C in the dark. Then, the reoxidized antibody was isolated using cation exchange chromatography (CEX), eluting with SPHP-C buffer [10mM succinate pH 5.0, 0.5 M Sodium chloride]. The purified antibody was concentrated to over 5mg/ml through ultrafiltration/diafiltration (UF/DF). To create the antibody-drug conjugate, the antibody was prepared in 1M Tris(hydroxymethyl)aminomethane (Tris-HCl, pH7.0) to a final concentration of 5mg/ml, and the drug was added at an antibody: drug molar ratio of 1:10, reacting for 16.5 hours at 25°C. The antibody-drug conjugate was isolated using cation exchange chromatography (CEX), eluting with SPHP-C buffer. To isolate only DAR2 antibody-drug conjugates, hydrophobic interaction chromatography (HIC) was utilized. For this, the Hitrap Butyl HP column (GE Healthcare) was equilibrated with HIC-A buffer [50mM Potassium phosphate, pH7.0, 1.0 M Ammonium sulfate] and eluted with HIC-B buffer [50mM Potassium phosphate, pH7.0, 30% 2-Propanol]. The antibody was exchanged into HS buffer [20mM Histidine, pH6.0, 240mM sucrose] through ultrafiltration/diafiltration (UF/DF) to remove 2-Propanol and finalize the antibody-drug conjugate, andthe drug used was pyrrolobenzodiazepine (PBD), and the resulting antibody was named 'antibody(V205C)-T-PBD'.

### (2) Purity and DAR measurement of manufactured bispecific antibody ADC

The purity of the manufactured ADC was measured using size-exclusion high-performance liquid chromatography (SE-HPLC). An Agilent 1200 series HPLC instrument equipped with a Tosoh TSKgel G3000SWxl column (Tosoh Bioscience) was used for the measurement, and the elution positions and the area under the curve (AUC) of each sample were compared to determine the purity of each sample. The main peak's purity of the ADC was over 99.0% (see Table 23 and FIGS. 10a to 10c).

The drug-to-antibody ratio (DAR) of the ADC was determined using liquid chromatography-mass spectrometry (LC/MS) method. To remove N-glycans, 1 unit of PNGaseF (NEB) per 100 µg of ADC (in PBS) at a concentration of 1 mg/ml was added and incubated at 37°C for 15 hours. A Waters UPLC I-class instrument coupled with a Waters Synapt G2-S LC/MS system was equipped with an Acquity UPLC BEH200 SEC 1.7 µm (4.6*150 mm) column and equilibrated with a mobile phase consisting of 30% (v/v) acetonitrile, 0.1% (v/v) formic acid, and 0.05% trifluoroacetic acid (TFA). 5 µg of the N-glycan-removed sample was loaded and analyzed by LC/MS. The measurement of the ADC's DAR (drug-to-antibody ratio) by LC/MS confirmed that there were 2 drugs conjugated per antibody molecule (see Tables 22, 23 and FIGS. 11a to 11c).

**TABLE 22**

| ADC | Purity (%) | DAR |
|---|---|---|
| BA6(M11/V205C)xB5-T-PBD | 99.63 | 2.0 |
| BA6(M11/V205C)-T-PBD | 99.54 | 2.0 |
| B5(V205C)-T-PBD | 99.57 | 2.0 |

**TABLE 23**

| ADC (DAR) | Theo. Mass (Da) | Exper. Mass (Da) | Delta Mass (Da) | Accuracy (ppm) |
|---|---|---|---|---|
| BA6(M11N205C)xB5-PBD (DAR2) | 199162.32 | 199187.00 | 24.68 | 123.92 |
| BA6(M11/V205C)-T-PBD (DAR2) | 146561.30 | 146585.00 | 23.70 | 161.71 |
| B5(V205C)-T-PBD (DAR2) | 145238.34 | 145267.00 | 28.66 | 197.33 |

### (3) Ligand binding activity test using bispecific antibody ADC

The ligand binding affinity of the BA6M11(V205C)-T-PBD monospecific antibody and BA6xB5(M11) (V205C)-T-PBD bispecific antibody ADCs to human ROR1 recombinant protein was evaluated using an ELISA-based solution binding assay. Specifically, 96-well microtiter plates (Nunc-Immuno Plates, NUNC) were coated for 16 hours at 4°C with ROR1 protein (ROR1-His; Sino Biological, Catalog # 13968-H08H) at a concentration of 1 µg/ml in PBS solution and blocked with 1% (v/v) BSA (bovine serum albumin) at 37°C for 2 hours to prevent non-specific binding.

Afterwards, the BA6M11(V205C)-T-PBD monospecific antibody and BA6xB5(M1 1)(V205C)-T-PBD bispecific antibody were added to the microtiter plates at the concentrations listed in FIG. 12, and their binding ability was analyzed by ELISA as follows: After incubating at 37°C for 2 hours, the plates were washed five times with PBS containing 0.05% (v/v) Tween 20, then HRP-conjugated Fc polyclonal antibody reagent (Pierce, Catalog # 31413) diluted at a 1:30,000 ratio in 1% (v/v) BSA was added to the washed microtiter plates, incubated for 1 hour at 37°C to allow the reaction, and used to detect the bound bispecific antibodies. The reaction was developed using TMB (tetramethylbenzidine, Sigma, T0440). The enzymatic reaction was stopped with 0.5mol/L sulfuric acid, and the absorbance was measured at 450nm and 650nm using a microplate reader (Molecular Devices).

The results displayed in FIG. 12 show that the ligand binding capability of the manufactured BA6xB5(M1 1) (V205C)-T-PBD bispecific antibody to human ROR1 protein was on par with that of the manufactured BA6M11(V205C)-T-PBD monospecific antibody. This confirms that the excellent ROR1 antigen binding ability of BA6M11 is retained even after being manufactured into a bispecific antibody ADC form.

### 3.6. Comparative evaluation of cytotoxicity of bispecific antibody ADC

The anticancer cell death efficacy of ADCs manufactured using the bispecific antibody of the present disclosure was compared with that of monospecific antibody ADCs. The cancer cell lines Calu-3 (KCLB, Catalog No. 30055) and Calu-6 (ATCC, Catalog No. HTB-56), which express both ROR1 and B7-H3, were employed, and the cell death capability was compared by administering ADCs to the cells and measuring the cells' metabolic activity. To determine if the effect of the bispecific antibody was merely a combination of the monospecific antibody targets, a control group treated with both the anti-ROR1 monospecific antibody and the anti-B7-H3 monospecific antibody (combination treatment, indicated with a "+") was also evaluated for comparison. The cancer cell lines were cultured using the recommended culture media. 5 x 10³ cells per well were inoculated into a 96-well culture plate with a volume of 50µl and cultured for 4-6 hours, and after this, 50µl of ADCs diluted at various concentrations were dispensed into the 96-well plate with cells. Then, the cells were cultured at 37°C, 5% CO₂ for about 6 days. The number of living cells was quantified using WST-8 (Dojindo, CK04).

FIG. 13 depicts the cytotoxicity results of the monospecific antibodies or bispecific antibody against cancer cell lines, using B5 as the anti-B7-H3 antibody and BA6M11 as the anti-ROR1 antibody. It was observed that in cancer cell lines (Calu-3, Calu-6) expressing both ROR1 and B7-H3, the cytotoxicity of the bispecific antibody (BA6M11xB5) ADC was greater than the combined cytotoxicity of monospecific antibody (BA6M11, B5) ADCs. Conversely, in the KATOIII cell line (ATCC, Catalog No. HTB-103), which barely expresses ROR1 and B7-H3, ADCs showed minimal cytotoxicity, and there was no significant difference in cytotoxicity between monospecific antibody ADCs and the bispecific antibody ADC. This indicates that the enhanced cytotoxicity of the bispecific antibody ADC compared to monospecific antibody ADCs is antigen-dependent. The results demonstrating higher cytotoxicity with the treatment of the bispecific antibody compared to the combination of monospecific antibodies confirm that the superior cytotoxicity of the bispecific antibody ADC of the present disclosure is more than just the effect of combining monospecific antibodies.

Although some embodiments have been described herein, it should be understood by those skilled in the art that these embodiments are given by way of illustration only, and that various modifications, variations, and alterations can be made without departing from the spirit and scope of the invention. Therefore, the scope of the invention should be limited only by the accompanying claims and equivalents thereof.

## Claims

1. An anti-ROR1 antibody or an antigen-binding fragment thereof, comprising:
HCDR1 comprising the amino acid sequence of SEQ ID NO: 1;
HCDR2 comprising the amino acid sequence of SEQ ID NO: 2;
HCDR3 comprising the amino acid sequence of SEQ ID NO: 3;
LCDR1 comprising the amino acid sequence of SEQ ID NO: 4;
LCDR2 comprising the amino acid sequence of SEQ ID NO: 5; and
LCDR3 comprising the amino acid sequence of SEQ ID NO: 6.

2. The anti-ROR1 antibody or antigen-binding fragment thereof according to claim 1, comprising a heavy chain variable region of SEQ ID NO: 7 or SEQ ID NO: 127, and a light chain variable region of SEQ ID NO: 8 or SEQ ID NO: 126.

3. A nucleic acid molecule encoding the anti-ROR1 antibody or antigen-binding fragment thereof according to claim 1 or 2.

4. A pharmaceutical composition for prevention or treatment of cancer, comprising the anti-ROR1 antibody or antigen-binding fragment thereof according to claim 1 or 2 and a pharmaceutically acceptable excipient.

5. The pharmaceutical composition according to claim 4, wherein the cancer expresses ROR1.

6. A composition for detecting ROR1, comprising the anti-ROR1 antibody or antigen-binding fragment thereof according to claim 1 or 2.

7. A composition for diagnosing cancer, comprising the anti-ROR1 antibody or antigen-binding fragment thereof according to claim 1 or 2.

8. The composition according to claim 7, wherein the cancer expresses ROR1.

9. An anti-ROR1/anti-4-1BB bispecific antibody, comprising:
an anti-ROR1 antibody or an antigen-binding fragment thereof; and
an anti-4-1BB antibody or an antigen-binding fragment thereof,
wherein the anti-ROR1 antibody or antigen-binding fragment thereof comprises HCDR1 comprising the amino sequence of SEQ ID NO: 1, HCDR2 comprising the amino sequence of SEQ ID NO: 2, HCDR3 comprising the amino sequence of SEQ ID NO: 3, LCDR1 comprising the amino sequence of SEQ ID NO: 4, LCDR2 comprising the amino sequence of SEQ ID NO: 5, and LCDR3 comprising the amino sequence of SEQ ID NO: 6.

10. The anti-ROR1/anti-4-1BB bispecific antibody according to claim 9, wherein the anti-ROR1 antibody or antigen-binding fragment thereof comprises a heavy chain variable region of SEQ ID NO: 7 or 127, and a light chain variable region of SEQ ID NO: 8 or 126.

11. The anti-ROR1/anti-4-1BB bispecific antibody according to claim 9 or 10, wherein the anti-4-1BB antibody or antigen-binding fragment thereof comprises:
HCDR1 comprising an amino acid sequence of SEQ ID NO: 13, 14, or 15;
HCDR2 comprising an amino acid sequence of SEQ ID NO: 16, 17, or 18;
HCDR3 comprising an amino acid sequence of SEQ ID NO: 19, 20, 21, 22, or 23;
LCDR1 comprising an amino acid sequence of SEQ ID NO: 24 or 25;
LCDR2 comprising an amino acid sequence of SEQ ID NO: 26 or 27; and
LCDR3 comprising an amino acid sequence of SEQ ID NO: 28 or 29.

12. The anti-ROR1/anti-4-1BB bispecific antibody according to any one of claims 9 to 11, wherein the anti-4-1BB antibody or antigen-binding fragment thereof,
(1) a heavy chain complementarity-determining region selected from the group consisting of:
(a) HCDR1 comprising the amino acid of SEQ ID NO: 13, HCDR2 comprising the amino acid of SEQ ID NO: 16, and HCDR3 comprising the amino acid of SEQ ID NO: 19,
(b) HCDR1 comprising the amino acid of SEQ ID NO: 13, HCDR2 comprising the amino acid of SEQ ID NO: 16, and HCDR3 comprising the amino acid of SEQ ID NO: 20,
(c) HCDR1 comprising the amino acid of SEQ ID NO: 13, HCDR2 comprising the amino acid of SEQ ID NO: 16, and HCDR3 comprising the amino acid of SEQ ID NO: 21,
(d) HCDR1 comprising the amino acid of SEQ ID NO: 14, HCDR2 comprising the amino acid of SEQ ID NO: 17, and HCDR3 comprising the amino acid of SEQ ID NO: 22, and
(e) HCDR1 comprising the amino acid of SEQ ID NO: 15, HCDR2 comprising the amino acid of SEQ ID NO: 18, and HCDR3 comprising the amino acid of SEQ ID NO: 23; and
(2) a light chain complementarity-determining region selected from the group consisting of:
(a) LCDR1 comprising the amino acid of SEQ ID NO: 24, LCDR2 comprising the amino acid of SEQ ID NO: 26, and LCDR3 comprising the amino acid of SEQ ID NO: 28, and
(b) LCDR1 comprising the amino acid of SEQ ID NO: 24, LCDR2 comprising the amino acid of SEQ ID NO: 26, and LCDR3 comprising the amino acid of SEQ ID NO: 28.

13. The anti-ROR1/anti-4-1BB bispecific antibody according to any one of claims 9 to 12, wherein the anti-4-1BB antibody or antigen-binding fragment thereof comprises:
a heavy chain variable region comprising an amino acid sequence of SEQ ID NO: 30, 31, 32, 33, 34, 35, 36, 37, 38, 39, 40, or 41; and
a light chain variable region comprising an amino acid sequence of SEQ ID NO: 42, 43, 44, 45, 46, or 47.

14. A nucleic acid molecule, encoding the anti-ROR1/anti-4-1BB bispecific antibody or antigen-binding fragment thereof according to any one of claims 9 to 13.

15. A pharmaceutical composition for prevention or treatment of cancer, comprising the anti-ROR1/anti-4-1BB bispecific antibody or antigen-binding fragment thereof according to any one of claims 9 to 13 and a pharmaceutically acceptable excipient.

16. The pharmaceutical composition according to claim 15, wherein the cancer expresses ROR1.

17. An anti-ROR1/anti-B7-H3 bispecific antibody, comprising:
an anti-ROR1 antibody or an antigen-binding fragment thereof; and
an anti-B7-H3 antibody or an antigen-binding fragment thereof,
wherein the anti-ROR1 antibody or antigen-binding fragment thereof comprises HCDR1 comprising the amino sequence of SEQ ID NO: 1, HCDR2 comprising the amino sequence of SEQ ID NO: 2, HCDR3 comprising the amino sequence of SEQ ID NO: 3, LCDR1 comprising the amino sequence of SEQ ID NO: 4, LCDR2 comprising the amino sequence of SEQ ID NO: 5, and LCDR3 comprising the amino sequence of SEQ ID NO: 6.

18. The anti-ROR1/anti-B7-H3 bispecific antibody according to claim 17, wherein the anti-ROR1 antibody or antigen-binding fragment thereof comprises a heavy chain variable region of SEQ ID NO: 7 or 127, and a light chain variable region of SEQ ID NO: 8 or 126.

19. The anti-ROR1/anti-B7-H3 bispecific antibody according to claim 17 or 18, wherein the anti-B7-H3 antibody or antigen-binding fragment thereof comprises:
HCDR1 comprising an amino acid sequence selected from SEQ ID NOS: 48 to 51;
HCDR2 comprising an amino acid sequence selected from SEQ ID NOS: 52 to 57;
HCDR3 comprising an amino acid sequence selected from SEQ ID NOS: 58 to 62;
LCDR1 comprising an amino acid sequence selected from SEQ ID NOS: 63 to 67;
LCDR2 comprising an amino acid sequence selected from SEQ ID NOS: 68 to 73; and
LCDR3 comprising an amino acid sequence selected from SEQ ID NOS: 74 to 79.

20. The anti-ROR1/anti-B7-H3 bispecific antibody according to any one of claims 16 to 19, wherein the anti-B7-H3 antibody or antigen-binding fragment thereof comprises:
(i) a heavy chain complementarity-determining region selected from the group consisting of:
(a) HCDR1 comprising the amino acid of SEQ ID NO: 48, HCDR2 comprising the amino acid of SEQ ID NO: 52, and HCDR3 comprising the amino acid of SEQ ID NO: 58,
(b) HCDR1 comprising the amino acid of SEQ ID NO: 49, HCDR2 comprising the amino acid of SEQ ID NO: 53, and HCDR3 comprising the amino acid of SEQ ID NO: 59,
(c) HCDR1 comprising the amino acid of SEQ ID NO: 50, HCDR2 comprising the amino acid of SEQ ID NO: 54, and HCDR3 comprising the amino acid of SEQ ID NO: 60,
(d) HCDR1 comprising the amino acid of SEQ ID NO: 48, HCDR2 comprising the amino acid of SEQ ID NO: 55, and HCDR3 comprising the amino acid of SEQ ID NO: 61, and
(e) HCDR1 comprising the amino acid of SEQ ID NO: 51, HCDR2 comprising the amino acid of SEQ ID NO: 56, and HCDR3 comprising the amino acid of SEQ ID NO: 62, and
(f) HCDR1 comprising the amino acid of SEQ ID NO: 50, HCDR2 comprising the amino acid of SEQ ID NO: 57, and HCDR3 comprising the amino acid of SEQ ID NO: 60; and
(ii) a light chain complementarity-determining region selected from the group consisting of:
(a) LCDR1 comprising the amino acid of SEQ ID NO: 63, LCDR2 comprising the amino acid of SEQ ID NO: 68, and LCDR3 comprising the amino acid of SEQ ID NO: 74,
(b) LCDR1 comprising the amino acid of SEQ ID NO: 64, LCDR2 comprising the amino acid of SEQ ID NO: 69, and LCDR3 comprising the amino acid of SEQ ID NO: 75;
(c) LCDR1 comprising the amino acid of SEQ ID NO: 65, LCDR2 comprising the amino acid of SEQ ID NO: 70, and LCDR3 comprising the amino acid of SEQ ID NO: 76;
(d) LCDR1 comprising the amino acid of SEQ ID NO: 66, LCDR2 comprising the amino acid of SEQ ID NO: 71, and LCDR3 comprising the amino acid of SEQ ID NO: 77;
(e) LCDR1 comprising the amino acid of SEQ ID NO: 67, LCDR2 comprising the amino acid of SEQ ID NO: 72, and LCDR3 comprising the amino acid of SEQ ID NO: 78; and
(f) LCDR1 comprising the amino acid of SEQ ID NO: 65, LCDR2 comprising the amino acid of SEQ ID NO: 73, and LCDR3 comprising the amino acid of SEQ ID NO: 76.

21. The anti-ROR1/anti-B7-H3 bispecific antibody according to any one of claims 16 to 20, wherein the anti-B7-H3 antibody or antigen-binding fragment thereof comprises:
a heavy chain variable region comprising an amino acid sequence selected from SEQ ID NO: 80 to 91; and
a light chain variable region comprising an amino acid sequence selected from SEQ ID NO: 92 to 103.

22. A nucleic acid molecule, encoding the anti-ROR1/anti-B7-H3 bispecific antibody or antigen-binding fragment thereof according to any one of claims 16 to 21.

23. A pharmaceutical composition for prevention or treatment of cancer, comprising the anti-ROR1/anti-B7-H3 bispecific antibody or antigen-binding fragment thereof according to any one of claims 16 to 21, and a pharmaceutically acceptable excipient.

24. The pharmaceutical composition according to claim 23, wherein the cancer expresses either or both of ROR1 and B7-H3.

25. An antibody-drug conjugate, comprising the anti-ROR1/anti-B7-H3 bispecific antibody or antigen-binding fragment thereof according to any one of claims 16 to 21 and a cytotoxic drug linked thereto.

26. The antibody-drug conjugate according to claim 25, wherein the antibody-drug conjugate has a s structure of antibody-linker-drug.

27. The antibody-drug conjugate according to claim 25 or 26, wherein the cytotoxic drug is an anticancer agent.

28. A pharmaceutical composition for prevention or treatment of cancer, comprising the antibody-drug conjugate of any one of claims 25 to 27 and a pharmaceutically acceptable excipient.

29. The pharmaceutical composition according to claim 28, wherein the cancer expresses either or both of ROR1 and B7-H3.
